# EUROPÄISCHE PATENTANMELDUNG

(11) **EP 4 238 450 A1**
(43) Veröffentlichungstag der Anmeldung: **06.09.2023**
(21) Anmeldenummer: 22159453.4
(22) Anmeldetag: 01.03.2022
(51) Int. Cl.: A46B 7/04

(54) **KÖRPERPFLEGEPRODUKT**

(71) Anmelder: Trisa Holding AG, 6234 Triengen (CH)
(72) Erfinder: BÄRTSCHI, Armin, 4652 Winznau (CH); FISCHER, Herbert, 5057 Reitnau (CH); ZWIMPFER, Martin, 6004 Luzern (CH)
(74) Vertreter: Daub, Thomas

(57) **Zusammenfassung**

Die Erfindung geht aus von einem Körperpflegeprodukt, insbesondere Zahnbürste, Rasierer oder Applikationspinsel, mit zumindest einer Anwendungseinheit (12a; 12b; 12c; 12d; 12e; 12f), mit einer Griffeinheit (14a; 14b; 14c; 14d; 14e; 14f), welche zumindest einen materiellen Griffkörper (16a; 16b; 16c; 16d; 16e; 16f) aufweist, der zumindest zu einem Großteil aus einem Papierwerkstoff besteht, und mit zumindest einer Verbindungseinheit (18a; 18b; 18c; 18d; 18e; 18f), welche die Anwendungseinheit (12a; 12b; 12c; 12d; 12e; 12f) mit der Griffeinheit (14a; 14b; 14c; 14d; 14e; 14f) verbindet.

Es wird vorgeschlagen, dass die Verbindungseinheit (18a; 18b; 18c; 18d; 18e; 18f) zumindest teilweise zu einer formschlüssigen Verbindung mit dem materiellen Griffkörper (16a; 16b; 16c; 16d; 16e; 16f) der Griffeinheit (14a; 14b; 14c; 14d; 14e; 14f) vorgesehen ist.

## Beschreibung

### Stand der Technik

Die Erfindung betrifft ein Körperpflegeprodukt.

Es ist ein Körperpflegeprodukt, insbesondere Zahnbürste, Rasierer oder Applikationspinsel, mit zumindest einer Anwendungseinheit, mit einer Griffeinheit, welche zumindest einen materiellen Griffkörper aufweist, der zumindest zu einem Großteil aus einem Papierwerkstoff besteht, und mit zumindest einer Verbindungseinheit, welche die Anwendungseinheit mit der Griffeinheit verbindet.

Aus der DE 20 2020 000 613 U1, der WO 2014/169398 A1, der WO 2021/081438 A1 sowie der DE 10 2020 100 106 A1 sind bereits Mundhygienemittel bekannt.

Die Aufgabe der Erfindung besteht insbesondere darin, eine gattungsgemäße Vorrichtung mit verbesserten Eigenschaften hinsichtlich einer Herstellbarkeit, eines Komforts sowie hinsichtlich der Ökologie bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einem Körperpflegeprodukt, insbesondere einer Zahnbürste, einem Rasierer oder einem Applikationspinsel, mit zumindest einer Anwendungseinheit, mit einer Griffeinheit, welche zumindest einen materiellen Griffkörper aufweist, der zumindest zu einem Großteil aus einem Papierwerkstoff besteht, und mit zumindest einer Verbindungseinheit, welche die Anwendungseinheit mit der Griffeinheit verbindet.

Es wird vorgeschlagen, dass die Verbindungseinheit zumindest teilweise zu einer formschlüssigen Verbindung mit dem materiellen Griffkörper der Griffeinheit vorgesehen ist. Zusätzlich zu der formschlüssigen Verbindung kann zudem eine kraft- und/oder stoffschlüssige Verbindung vorgesehen sein. Es ist insbesondere denkbar, dass die Verbindungseinheit zusätzlich zumindest teilweise mit dem materiellen Griffkörper der Griffeinheit verklebt, verschweißt, vergossen oder stoffschlüssig verbunden ist. Ferner wäre denkbar, dass eine Verbindung mittels Anspritzen und/oder Umspritzen oder mittels Klemmen hergestellt ist.

Unter "zumindest zu einem Großteil" soll insbesondere verstanden werden, dass zumindest ein Hauptbestandteil eines Materials des materiellen Griffkörpers der Griffeinheit und/oder der Anwendungseinheit ein Papierwerkstoff ist. Vorzugsweise sind zumindest 50 Gew.% (Gewichtsprozent), vorzugsweise zumindest 70 Gew.% und besonders bevorzugt zumindest 90 Gew.% des materiellen Griffkörpers der Griffeinheit aus einem Papierwerkstoff. Besonders bevorzugt besteht der materielle Griffkörper der Griffeinheit vollständig aus einem Papierwerkstoff. Ferner soll in diesem Zusammenhang unter einem "Papierwerkstoff" insbesondere ein, vorzugsweise flächiger, Werkstoff verstanden werden, der im Wesentlichen aus Fasern, vorzugsweise pflanzlicher Herkunft, besteht. Der Werkstoff wird vorzugsweise durch Entwässerung einer Fasersuspension auf einem Sieb gebildet. Das entstehende Faservlies wird anschließend insbesondere verdichtet und getrocknet. Vorzugsweise ist der Werkstoff aus Zellstoff, Holzschliff und/oder Altpapier hergestellt.

Vorzugsweise besteht der Papierwerkstoff insbesondere aus Fasern, wobei insbesondere verschiedene Verarbeitungsmöglichkeiten bestehen. Der Papierwerkstoff kann zudem behandelt sein. Der Papierwerkstoff kann beschichtet sein, wie insbesondere lackiert, beispielsweise mit einem Lack zu einer Verbesserung der Verbindung beim Verbinden und/oder zur Verbesserung der Resistenz gegen Wasser, und/oder getränkt. Der Papierwerkstoff kann insbesondere sowohl in flacher Form, in Block-Form oder in flüssiger bis pappeförmiger Form und/oder Konsistenz vorliegen, wobei ausgehend von der Form verschiedene Verarbeitungen möglich sind. In flacher Form kann der Papierwerkstoff gerollt, geschichtet, gepresst, laminiert, gerillt, geprägt, gefaltet, gestanzt und/oder grainiert, also insbesondere aufgeraut oder gekörnt, werden. Zu einem Verbinden kann der Papierwerkstoff geklebt werden, wobei allenfalls Vorbereitungsschritte wie Grainieren oder dergleichen nötig sind, verschweißt werden, wie insbesondere mittels Ultraschall-Schweißen, Siegeln (heiß oder kalt) oder Laminieren, etc. Zu einem Verbinden können auf dem Papierwerkstoff Spezialschichten nötig sein, um die Verbindung zu schaffen. Verbindungen können mit einem Papierwerkstoff oder anderen in dieser Schrift genannten Materialien bzw. Werkstoffen hergestellt werden. In Block-Form kann der Papierwerkstoff mechanisch bearbeitet, beispielsweise gefräst, gebohrt oder gestanzt werden. In flüssiger Form kann der Papierwerkstoff gegossen oder spritzgegossen werden, wie insbesondere mittels Papierspritzguss oder Papierguss bzw. Faserguss. Hierbei wird der Papierwerkstoff insbesondere analog zu einem Spritzgussprozess in einer Kavität geformt. Anschließend wird das gegossene Teil ausgehärtet bzw. getrocknet. Beim Papierspritzguss ist insbesondere auch ein Anspritzen an andere Teile bzw. Materialien, beispielsweise Kunststoff mit Papier umspritzt, denkbar. Alternativ kann ein Kunststoffteil auch an einem Papierwerkstoff angespritzt werden. Insgesamt kann der Produktaufbau des Körperpflegeprodukts verschiedener Art sein, wie insbesondere vollständig aus einem Papierwerkstoff oder in einer Hybridtechnik, beispielsweise aus Papierwerkstoff mit Kunststoff, insbesondere beispielsweise mit einem Wechselkopf und/oder mit einer Griffeinheit aus Papier und einer Anwendungseinheit aus Kunststoff. Weitere Materialkombinationen mit Papier können wie erwähnt Kunststoff und/oder Metall und/oder organische Materialien wie Holz, Bambus und/oder anorganische Materialien wie Stein, Glas etc. sein.

Durch die erfindungsgemäße Ausgestaltung des Körperpflegeprodukts können vorteilhafte Eigenschaften hinsichtlich einer Ergonomie sowie einer Herstellbarkeit des Körperpflegeprodukts bereitgestellt werden. Es kann insbesondere ein vorteilhaft ökologisches Körperpflegeprodukt bereitgestellt werden. Es kann insbesondere eine vorteilhaft einfache Trennung bzw. Entsorgung des Körperpflegeprodukts erreicht werden.

Das Körperpflegeprodukt ist insbesondere von einem Rasierer, insbesondere einem manuellen Rasierer, einem Pinsel, insbesondere einem Applikationspinsel für kosmetische Anwendungen wie z.B. zur Haarfärbung, und/oder einem Mundhygienemittel gebildet. Der Rasierer kann beispielsweise von einem Einwegrasierer oder einem Mehrwegrasierer mit einer wechselbaren Klinge gebildet sein. Unter einem "Mundhygienemittel" soll insbesondere eine Zahnbürste und/oder ein Zungenreiniger verstanden werden. Vorteilhaft ist das Mundhygienemittel als eine Zahnbürste, insbesondere eine Handzahnbürste, ausgebildet. Das Mundhygienemittel kann hierbei bei einer Ausbildung als Zahnbürste insbesondere eine Einwegzahnbürste (für Einmalgebrauch), eine Mehrwegzahnbürste (für Mehrfachgebrauch) oder auch eine Wechselkopfzahnbürste sein. Das Mundhygienemittel könnte jedoch auch allgemein von einem Bürstenprodukt gebildet sein. Unter einem "Körperpflegeprodukt" soll insbesondere eine Mundhygienebürste und/oder eine Kosmetikbürste und/oder eine Haarbürste und/oder eine Haushaltsbürste verstanden werden. Als Mundhygienebürsten sind beispielsweise manuelle Zahnbürsten wie Mehrwegzahnbürsten, Wechselkopfzahnbürsten, Einwegzahnbürsten oder Single-Tuft-Bürsten oder Zungenreiniger denkbar. Als Körperpflegeprodukte, insbesondere Kosmetikbürsten, sind ferner beispielsweise Mascarabürsten, Nagellackpinsel, Gesichtsbürsten, Applikatoren, Massagegeräte, Make-up-Pinsel, Rasierpinsel und/oder Nassrasierer oder andere Körperpflegeprodukte denkbar.

Bei einer Ausbildung als Zahnbürste ist insbesondere denkbar, dass die Anwendungseinheit aus einem Grundkörper und einem Borstenplättchen, welches mit Borsten und/oder alternativen Reinigungselementen besetzt ist, zusammengesetzt ist. Weiter kann die Anwendungseinheit aus einem Grundkörper bestehen, welcher mit Borsten umspritzt wird, oder die Anwendungseinheit kann ohne Grundkörper direkt gespritzt sein. Vorzugsweise besteht das Körperpflegeprodukt aus einer Anwendungseinheit mit Borsten, der Verbindungseinheit und der Griffeinheit, wobei die Griffeinheit insbesondere einen Halsteil aufweist. Alle Teilelemente können aus mindestens einer Hart- und/oder einer oder mehreren Weichkomponente/n bestehen.

Das Körperpflegeprodukt weist insbesondere eine Längsachse auf, die vorteilhaft zumindest im Wesentlichen parallel zu einer Haupterstreckungsrichtung des Körperpflegeprodukts angeordnet ist. Bevorzugt verläuft die Längsachse zumindest abschnittsweise innerhalb des Körperpflegeprodukts und insbesondere durch dessen Schwerpunkt. Insbesondere ist die Längsachse des Körperpflegeprodukts eine Zentralachse des Körperpflegeprodukts und/oder eine Zentralachse der Griffeinheit. Unter einer "Zentralachse" eines Objekts soll dabei insbesondere eine gedachte Achse verstanden werden, die innerhalb des Objekts parallel zu einer Haupterstreckungsrichtung des Objekts verläuft und das Objekt an höchstens zwei Punkten schneidet. Unter "zumindest im Wesentlichen parallel" soll hier insbesondere eine Ausrichtung einer Richtung relativ zu einer Bezugsrichtung, insbesondere in einer Ebene, verstanden werden, wobei die Richtung gegenüber der Bezugsrichtung eine Abweichung insbesondere kleiner als 8°, vorteilhaft kleiner als 5° und besonders vorteilhaft kleiner als 2° aufweist. Unter einer "Haupterstreckungsrichtung" eines Objekts soll dabei insbesondere eine Richtung verstanden werden, welche parallel zu einer längsten Kante eines kleinsten gedachten Quaders verläuft, welcher das Objekt gerade noch vollständig umschließt. Unter einer "Haupterstreckung" eines Objekts soll in diesem Zusammenhang insbesondere eine Erstreckung einer längsten Kante eines kleinsten gedachten Quaders, welcher das Objekt gerade noch vollständig umschließt, verstanden werden.

Vorteilhaft weist die Anwendungseinheit zumindest einen Reinigungsbereich auf, der zu einer Zahnreinigungsanwendung, insbesondere in einem Mundraum des Benutzers, vorgesehen ist. Vorzugsweise umfasst der Reinigungsbereich zumindest eine Reinigungseinheit. Die Reinigungseinheit kann einen Bürstenkopf, vorteilhaft einen Zahnbürstenkopf, bevorzugt mit mehreren Borsten und/oder Borstenbündeln und/oder gespritzten Reinigungselementen bzw. gespritzten Borsten und/oder weichelastischen Reinigungselementen, umfassen. Alternativ oder zusätzlich kann die Reinigungseinheit auch als ein Single Tuft (d.h. als einzelnes großes Borstenbündel) oder dergleichen ausgebildet sein. Ferner weist die Anwendungseinheit vorteilhaft zumindest einen Grundkörper auf, welcher bevorzugt mit dem Reinigungsbereich, insbesondere unmittelbar und/oder einstückig, verbunden ist. Alternativ oder zusätzlich weist die Anwendungseinheit zumindest einen Applikationsbereich auf, der beispielsweise zu einer Speicherung und Abgabe einer Farbe, insbesondere einer Haarfarbe, vorgesehen ist. Alternativ oder zusätzlich weist die Anwendungseinheit zumindest einen Rasierbereich mit zumindest einer Klinge auf.

Unter "einstückig" soll insbesondere zumindest stoffschlüssig verbunden, beispielsweise durch einen Verbindungsprozess wie einen Schweißprozess, einen Siegelprozess, einen Klebeprozess, einen Anspritzprozess und/oder einen anderen, dem Fachmann als sinnvoll erscheinenden Prozess, und/oder vorteilhaft in einem Stück geformt verstanden werden, wie beispielsweise durch eine Herstellung aus einem Guss und/oder durch eine Herstellung in einem Ein- oder Mehrkomponentenspritzverfahren und vorteilhaft aus einem einzelnen Rohling. Unter "vorgesehen" soll insbesondere speziell ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Vorzugsweise weist die Griffeinheit zumindest ein Griffelement auf, das vorteilhaft zu einem Halten mit einer Hand vorgesehen ist. Es ist denkbar, dass das Griffelement zumindest bereichsweise tailliert und/oder mit einem konkaven Oberflächenbereich und/oder mit einem konvexen Oberflächenbereich ausgebildet ist. Dies erlaubt vorteilhaft einen sicheren Halt und optimiert die Ergonomie. Besonders bevorzugt ist das Griffelement länglich ausgebildet, wobei vorteilhaft eine Längsachse des Griffelements der Längsachse des Körperpflegeprodukts entspricht. Das Griffelement ist zumindest im Wesentlichen, insbesondere vollständig, aus mindestens einem Papierwerkstoff ausgebildet. Alternativ oder zusätzlich kann das Griffelement und/oder die Griffeinheit zumindest teilweise aus einer Hartkomponente und/oder aus einer oder mehreren Weichkomponenten ausgebildet sein. Vorzugsweise ist der materielle Griffkörper zumindest zu einem Großteil, insbesondere vollständig, aus mindestens einem Papierwerkstoff ausgebildet. Insbesondere umfasst das Griffelement vorteilhaft zumindest einen Daumengriffbereich und/oder zumindest einen Handgriffbereich. Vorteilhaft ist der Daumengriffbereich auf der Vorderseite des Körperpflegeprodukts und insbesondere auf einer Vorderseite des Griffelements angeordnet. Es ist denkbar, dass der Daumengriffbereich und/oder der Handgriffbereich zumindest ein Element und/oder eine Oberflächenstrukturierung aus mindestens einem Papierwerkstoff, aus mindestens einer Weichkomponente und/oder mindestens einer Hartkomponente aufweisen/aufweist.

Die für die Herstellung eingesetzten Kunststoffe aus Hart- und/oder Weichkomponenten, welche mittels Spritzguss verarbeitet werden, können insbesondere wie folgt eingeteilt werden:
- Konventionelle Materialien: Materialien, im Wesentlichen Neumaterialien, die größtenteils erdölbasiert sind.
- Nachhaltige Materialien: wie nachfolgend aufgezählt und später beschrieben vorzugsweise biobasiert, abbaubar und/oder recycliert.
   ∘ Biobasierte Materialien: Material, welches insbesondere zu mehr als 60 %, vorzugsweise zu mehr als 80 % und besonders bevorzugt zu 100 % aus nachwachsenden Rohstoffen hergestellt ist. Eine weitere mögliche zusätzliche Eigenschaft biobasierter Materialien ist insbesondere, dass die biobasierten Materialien biologisch abbaubar sind. Vorzugsweise basieren die Materialien nicht auf Nahrungsmitteln, wie insbesondere Mais etc..
   ∘ Biologisch abbaubare Materialien: Material, das gemäß den gängigen Normen biologisch abbaubar ist. Hierzu zählt insbesondere die Kompostierbarkeit (industriell oder nicht-industriell). Dabei können Materialien aus nachwachsenden Rohstoffen diese Eigenschaft aufweisen.
   ∘ Recyclierte Materialien: Materialien, die aus einem Recycling-Prozess stammen, wie beispielsweise Post Consumer Recycled Materialien, Post Industrial Recycled Materialien, Ocean Waste Plastic oder Social Plastic.

Die eingesetzten Materialien können recyclebare Materialien sein. Für recyclierbare Materialien besteht nach dem Gebrauch vorteilhaft eine Recyclingmöglichkeit.

Im Rahmen dieser Offenbarung kommen nahezu beliebige Hartkomponenten und Weichkomponenten infrage, die der Fachmann zweckgemäß geeignet kombinieren und/oder auswählen wird. Als Hartkomponente kommen beispielsweise Styrolpolymerisate wie Styrolacrylnitril (SAN), Polystyrol (PS), Acrylnitrylbutadienstyrol (ABS), Styrolmethylmethacrylate (SMMA), Styrolbutadien (SB) oder dergleichen infrage. Ferner kann eine Hartkomponente Polyolefine wie Polypropylen (PP), Polyethylen (PE) oder dergleichen umfassen, insbesondere auch in Form von High-Density-Polyethylen (HDPE) oder Low-Density-Polyethylen (LDPE). Zudem kommen Polyester wie beispielsweise Polyethylenterephthalat (PET), insbesondere in Form von säuremodifiziertem Polyethylenterephthalat (PETA) oder glykolmodifiziertem Polyethylenterephthalat (PETG), Polybutylenterephthalat (PBT), säuremodifiziertes Polycyclohexylendimethylenterephthalat (PCT-A), glykolmodifiziertes Polycyclohexylendimethylenterephthalat (PCT-G) oder dergleichen infrage. Weiterhin ist eine Verwendung von Cellulosederivaten wie beispielsweise Celluloseacetat (CA), Celluloseacetobutyrat (CAB), Cellulosepropionat (CP), Celluloseacetatphthalat (CAP), Cellulosebutyrat (CB) oder dergleichen denkbar. Ferner kann eine Hartkomponente beispielsweise Polyamide (PA) wie PA 6.6, PA 6.10, PA 6.12 oder dergleichen, Polymethylmethacrylat (PMMA), Polycarbonat (PC), Polyoxymethylen (POM), Polyvinylchlorid (PVC), Polyurethan (PUR), Polyamid (PA) oder dergleichen mehr umfassen. Insbesondere Polyethylen (PE) und Polyurethan (PU) können als Hartkomponente und/oder als Weichkomponente eingesetzt werden. Insbesondere weist eine Hartkomponente ein Elastizitätsmodul von wenigstens 1000 N/mm² und vorteilhaft von wenigstens 1300 N/mm² und/oder von höchstens 2400 N/mm² und vorteilhaft von höchstens 1800 N/mm² auf. Als Hartkomponente wird bevorzugt Polypropylen (PP) eingesetzt. Mindestens gewisse als Hartkomponente genannte Materialien können nachhaltige Materialien sein. Insbesondere Materialien mit Celluloseanteil sind zumindest teilweise biobasiert.

Vorteilhaft werden Hartkomponenten für stabile und/oder strukturtragende Elemente, insbesondere in dem Griffelement und/oder in einem Verbindungselement der Anwendungseinheit oder dergleichen eingesetzt. Vorteilhaft ist denkbar, dass eine verwendete Hartkomponente und eine verwendete Weichkomponente unterschiedliche Farben aufweisen, sodass Oberflächenstrukturen, Beschriftungen, Motive und dergleichen mittels geeigneter Gestaltung von Grundkörper und Weichelement realisierbar sind.

Als Weichkomponenten kommen beispielsweise thermoplastische Styrol-Elastomere (TPE-S) wie etwa ein Styrol-Ethylen-Butylen-Styrol-Copolymer (SEBS), ein StyrolButadien-Styrol-Copolymer (SBS) oder dergleichen infrage. Zudem ist eine Verwendung thermoplastischer Polyurethan-Elastomere (TPE-U), thermoplastischer Polyamid-Elastomere (TPE-A), thermoplastischer Polyolefin-Elastomere (TPE-O), thermoplastischer Polyester-Elastomere (TPE-E) oder dergleichen denkbar. Weiterhin kann eine Weichkomponente beispielsweise zumindest ein Silikon umfassen. Vorteilhaft weist eine Weichkomponente eine Shore-A-Härte von höchstens 90, vorteilhaft von höchstens 50 und besonders vorteilhaft von höchstens 30 auf. Vorzugsweise bildet zumindest eine Weichkomponente mit zumindest einer Hartkomponente, insbesondere in zumindest einem Zwei- und/oder Mehrkomponentenspritzguss, vorteilhaft mittels zumindest eines Überspritzens und/oder Umspritzens, zumindest einen Materialschluss. Die unter der Weichkomponente genannten Materialien können nachhaltige Materialien sein. Hartkomponente, Weichkomponente oder Material für gespritzte Borsten bilden mit dem Papierwerkstoff keinen Materialschluss.

Das Körperpflegeprodukt weist bei einer Ausbildung als Zahnbürste insbesondere eine Vorderseite und eine Rückseite auf, die insbesondere einander abgewandt angeordnet sind. Vorzugsweise ist der Reinigungsbereich der Anwendungseinheit auf der Vorderseite des Körperpflegeprodukts angeordnet. Die Vorderseite ist insbesondere eine in einer Betrachtungsrichtung senkrecht zu der Längsachse des Körperpflegeprodukts und senkrecht zu der Breitenachse des Körperpflegeprodukts sichtbare Seite des Körperpflegeprodukts. Als Vorderseite des Körperpflegeprodukts ist insbesondere jene Seite der Bürste bezeichnet, auf welcher der Daumen aufgelegt wird. Die Vorderseite ist normalerweise auch jene Seite, auf welche das Borstenfeld gerichtet ist. Die Rückseite entspricht vorteilhaft einer in einer entgegengesetzten Betrachtungsrichtung sichtbaren Seite des Körperpflegeprodukts. Als Rückseite des Körperpflegeprodukts wird die dem Borstenfeld entgegengesetzte Seite der Zahnbürste bezeichnet. Als linke Seite des Körperpflegeprodukts wird insbesondere eine Seite bezeichnet, welche links liegt, wenn man senkrecht auf die Vorderseite des Körperpflegeprodukts blickt und der Reinigungsbereich oben liegt. Als rechte Seite des Körperpflegeprodukts wird insbesondere eine Seite bezeichnet, welche rechts liegt, wenn man senkrecht auf die Vorderseite des Körperpflegeprodukts blickt. Als Oberseite wird insbesondere ein Ende des Körperpflegeprodukts bezeichnet, an welchem der Reinigungsbereich angeordnet ist. Als Unterseite wird insbesondere ein der Oberseite entgegengesetztes Ende des Körperpflegeprodukts bezeichnet, welches dem Griffbereich am nächsten liegt.

Unter einer "Verbindungseinheit" soll in diesem Zusammenhang insbesondere eine Einheit verstanden werden, welche zu einer Bereitstellung einer festen und/oder lösbaren Verbindung zwischen zwei Einheiten, insbesondere Bauteilen, vorgesehen ist. Vorzugsweise verbindet die Verbindungseinheit die Anwendungseinheit mit der Griffeinheit. Bevorzugt sind die Anwendungseinheit und die Griffeinheit zumindest für eine Benutzung miteinander verbunden. Vorzugsweise kann die Verbindungseinheit sowohl von einer separaten Einheit gebildet sein, als auch zumindest teilweise fest mit der Griffeinheit und/oder der Anwendungseinheit verbunden sein. Darunter, dass zumindest ein erstes Element mit zumindest einem weiteren Element "verbunden" ist, soll insbesondere verstanden werden, dass das erste Element vorteilhaft über zumindest einen Kraftschluss und/oder zumindest einen Formschluss mit dem weiteren Element verbunden ist, beispielsweise über eine Vernietung und/oder eine Rastverbindung und/oder eine Nut-Feder-Verbindung und/oder eine Klemmverbindung und/oder eine weitere, dem Fachmann als sinnvoll erscheinende Verbindung, und/oder stoffschlüssig mit dem weiteren Element verbunden ist, beispielsweise durch einen Schweißprozess, einen Siegelprozess, einen Klebeprozess, einen Anspritzprozess und/oder einen anderen, dem Fachmann als sinnvoll erscheinenden Prozess. Unter einer "formschlüssigen Verbindung" soll in diesem Zusammenhang insbesondere eine Verbindung verstanden werden, bei welcher aneinanderliegende Flächen von miteinander formschlüssig verbundenen Bauteilen eine in Normalenrichtung der Flächen wirkende Haltekraft aufeinander ausüben. Insbesondere befinden sich die Bauteile in einem geometrischen Eingriff miteinander. Vorzugsweise ist insbesondere zwischen der Anwendungseinheit und der Griffeinheit ein Formschluss vorgesehen, wie beispielsweise durch aneinander angepasste Geometrien. So können insbesondere an der Anwendungseinheit, der Griffeinheit und/oder der Verbindungseinheit Vertiefungen, Kämme und/oder Formschlusselemente vorgesehen sein. Insbesondere kann die formschlüssige Verbindung auch lediglich einer Orientierung der Verbindungsmöglichkeiten dienen, um bei einer Montage eine einfache Verbindung zu ermöglichen oder um beispielsweise bei einer Klebeverbindung schädliche Querkräfte abzufangen. Die Orientierung der Verbindungsmöglichkeiten kann insbesondere rund um die Griffeinheit erfolgen, wie beispielsweise bei einem Pinsel auf der Vorderseite, der Rückseite oder den kurzen Seiten. Wenn bei dem Körperpflegeprodukt lange und kurze Seiten vorhanden sind, erfolgt eine Orientierung der Verbindungsmöglichkeiten insbesondere an einer langen Seite.

Die Verbindungseinheit weist insbesondere die Funktion auf, die Anwendungseinheit oder eine Schnittstelle der Anwendungseinheit mit der Griffeinheit zu verbinden. Die Verbindungseinheit weist insbesondere mehrere Funktionen auf. Insbesondere ist die Verbindungseinheit dazu vorgesehen, die Griffeinheit zu verbinden und die Anwendungseinheit zu halten. Die Verbindungseinheit bildet insbesondere eine Schnittstelle zu der Griffeinheit aus. Ferner kann die Verbindungseinheit insbesondere ein Wechselsystem enthalten, mit welchem die Anwendungseinheit ausgetauscht werden kann. Zudem kann die Verbindungseinheit beispielsweise eine Haltegeometrie zu einem Greifen des Körperpflegeproduktes beispielsweise während der Anwendung aufweisen. So ist insbesondere eine Kombination aus verschiedenen genannten Funktionen denkbar, wie beispielsweise Innen einer Einführungsgeometrie oder einer Stützgeometrie und Außen eines Halteelements.

Ferner wird vorgeschlagen, dass die Verbindungseinheit zumindest ein Formschlusselement aufweist, welches dazu vorgesehen ist, in eine Ausnehmung der Anwendungseinheit und/oder der Griffeinheit einzugreifen. Vorzugsweise ist das Formschlusselement insbesondere dazu vorgesehen, einen geometrischen Eingriff bereitzustellen, wobei das Formschlusselement geometrisch in die Ausnehmung eingreift, sodass in einer Ebene senkrecht zu einer Mittelachse der Ausnehmung das Formschlusselement vollständig von einem die Ausnehmung begrenzenden Bauteil umgeben ist. Bevorzugt ist das Formschlusselement direkt von einer die Ausnehmung begrenzenden Begrenzungsfläche der Anwendungseinheit und/oder der Griffeinheit begrenzt. Vorzugsweise kann das Formschlusselement beispielweise von einem Rastzapfen, einem Niet, einer Feder oder dergleichen gebildet sein. Vorzugsweise ist das Formschlusselement dazu vorgesehen, in eine Ausnehmung der Anwendungseinheit und/oder der Griffeinheit einzugreifen und in dieser Ausnehmung, beispielsweise durch einen Hinterschnitt und/oder durch ein Hintergreifen, einen Formschluss zu erzeugen, welcher über einen Formschluss quer zu einer Einschubrichtung in die Ausnehmung hinausgeht. Dadurch kann insbesondere eine vorteilhafte Verbindungseinheit bereitgestellt werden. Es kann insbesondere eine vorteilhaft leicht herstellbare und stabile Verbindung bereitgestellt werden.

Des Weiteren wird vorgeschlagen, dass der materielle Griffkörper zumindest eine Durchgangsausnehmung begrenzt, welche dazu vorgesehen ist, dass zumindest eine Formschlusselement der Verbindungseinheit aufzunehmen. Dadurch kann insbesondere vorteilhaft eine Formschlussausnehmung für das Formschlusselement bereitgestellt werden. Ferner kann vorteilhaft einfach eine Formschlussausnehmung in den materiellen Grundkörper eingebracht werden. Vorzugsweise ist die Durchgangsausnehmung korrespondierend zu dem Formschlusselement der Verbindungseinheit ausgebildet. Vorzugsweise ist die Verbindungseinheit zu einer formschlüssigen Verbindung mit der Griffeinheit vorgesehen. Die Durchgangsausnehmung ist beispielsweise von einer zylindrischen, insbesondere von einer kreiszylindrischen, Ausnehmung gebildet. Die zumindest eine Durchgangsausnehmung erstreckt sich vollständig durch den materiellen Griffkörper hindurch. Vorzugsweise erstreckt sich die zumindest eine Durchgangsausnehmung senkrecht zu einer Haupterstreckungsebene des materiellen Griffkörpers durch den materiellen Griffkörper. Unter einer "Haupterstreckungsebene" eines Objekts soll insbesondere eine Ebene verstanden werden, welche parallel zu einer größten Seitenfläche eines kleinsten gedachten Quaders ist, welcher das Objekt gerade noch vollständig umschließt, und durch den Mittelpunkt des Quaders verläuft. Die zumindest eine Durchgangsausnehmung bildet insbesondere eine Gegenkontur für das Formschlusselement der Verbindungseinheit aus.

Es wird ferner vorgeschlagen, dass das Formschlusselement von einem Rastelement gebildet ist. Unter einem "Rastelement" soll in diesem Zusammenhang insbesondere ein federelastisches Element zur Herstellung einer Rastverbindung verstanden werden, das dazu vorgesehen ist, bei einer Montage elastisch ausgelenkt zu werden. Vorzugsweise ist das Rastelement dazu vorgesehen, bei einem Befestigungsvorgang elastisch ausgelenkt zu werden, um anschließend durch eine innere Spannkraft hinter einem korrespondierenden Rastelement, insbesondere in einer Rastausnehmung, besonders bevorzugt hinter einem Rand der Durchgangsausnehmung, einzurasten. Das Rastelement bildet insbesondere ein Element mit einem Hinterschnitt aus, welches dazu vorgesehen ist, hinter einem korrespondierenden Element zu verrasten. Vorzugsweise ist das Rastelement dazu vorgesehen, sich durch die Durchgangsausnehmung zu erstrecken und hinter der Durchgangsausnehmung an dem materiellen Griffkörper zu verrasten. Das Rastelement ist beispielsweise von einer Schnappzunge gebildet. Alternativ wäre auch denkbar, dass das Rastelement an dem materiellen Griffkörper angeordnet ist, während ein korrespondierendes Element und/oder eine Ausnehmung an der Verbindungseinheit angeordnet ist. Beide Elemente können insbesondere an beiden zu verbindenden Einheiten angebracht sein. Das Rastelement wird insbesondere an das korrespondierende Element angerastet oder in die Ausnehmung eingerastet. Eine Positionierung des Rastelements bei einer Montage erfolgt insbesondere durch eine Kombination von Aufsetzen und Einführen. Es erfolgt insbesondere eine Positionierung und Fixierung in einem. Bei einer Herstellung werden insbesondere die Verbindungseinheit, vorzugsweise zusammen mit der Anwendungseinheit, und die Griffeinheit jeweils separat hergestellt und nachträglich bei einer Montage durch Verrasten zusammengefügt.

Es sind verschiedene, einem Fachmann als sinnvoll erscheinende Anordnungen und/oder Gestaltungsvarianten des zumindest einen Rastelements denkbar. Bei mehreren Rastelementen kann eine Anordnung auf einer Linie oder, insbesondere bei drei Rastelementen, im Dreieck erfolgen. Eine Anordnung im Dreieck ist insbesondere stabiler und im montierten Zustand weniger wackelig als eine Anordnung auf einer Linie. Ferner ist eine vorteilhafte Aufnahme von Torsions- und Scherkräften möglich. Es ist jedoch insbesondere jede beliebige Anordnung der Rastelemente denkbar. Bei mehreren Rastelementen können diese insbesondere einzeln gefedert sein oder auf einem gemeinsamen Federarm angeordnet sein. Die Rastelemente können insbesondere verteilt an einem gemeinsamen freistehenden Federarm angeordnet sein. Es ist jedoch auch denkbar, dass mehrere Rastelemente vorgesehen sind, die jeweils an einem freistehenden Federarm angeordnet sind. Die Rastelemente können sowohl in Ausnehmungen einer Hauptfläche des materiellen Griffkörpers einrasten als auch in Ausnehmungen in einer Seitenfläche des materiellen Griffkörpers einrasten. Das Rastelement kann insbesondere über die gesamte Dicke oder Breite des materiellen Griffkörpers reichen. Ferner wäre denkbar, dass das Rastelement am Ende einer längeren Zunge der Verbindungseinheit angeordnet ist, insbesondere um Hebelkräfte für eine stabile Anordnung zu erzeugen. Alternativ können die Rastelemente auch auf einem starren Untergrund der Verbindungseinheit angeordnet sein. Alternativ oder zusätzlich kann ein, insbesondere großes, Rastelement vorgesehen sein.

In einer bevorzugten Ausführungsform ist das Rastelement insbesondere pilzförmig mit einem Stielabschnitt und einem Kopfabschnitt ausgebildet. Vorzugsweise kann das pilzförmige Rastelement geteilt sein und so zwei oder mehrere federnde Teilelemente enthalten. Das Rastelement oder die Teilelemente enthalten insbesondere Hinterschnitte, die die Verbindungseinheit an der Griffeinheit halten. Der materielle Griffkörper der Griffeinheit wird insbesondere zwischen einer Fläche der Verbindungseinheit und einem Hinterschnitt am Rastelement gehalten. Hierdurch kann insbesondere eine vorteilhaft mechanische Verbindung bereitgestellt werden. Vorzugsweise sind/ist insbesondere ein bis sechs Rastelemente, vorzugsweise ein bis drei Rastelemente und besonders bevorzugt genau ein Rastelement an der Verbindungseinheit vorgesehen.

Es wird weiter vorgeschlagen, dass das Formschlusselement von einem Niet, insbesondere einem Kunststoffniet, gebildet ist. Vorzugsweise ist das Formschlusselement dazu vorgesehen, sich durch die Durchgangsausnehmung hindurch zu erstrecken und hinter der Durchgangsausnehmung gestaucht, insbesondere aufgeschmolzen, zu werden. Es kann dadurch eine pilzförmige Nietstruktur geschaffen werden. Die zumindest eine Durchgangsausnehmung ist insbesondere korrespondierend zu dem zumindest einen Formschlusselement ausgebildet. Die Durchgangsausnehmung bildet insbesondere eine Gegenkontur zu dem Formschlusselement aus. Das Formschlusselement ist insbesondere von einem zylindrischen vorstehenden Schaft gebildet, wobei das Formschlusselement insbesondere aus einer Formschlusskontur der Verbindungseinheit absteht oder in dieser angeordnet ist. Ferner dient das zumindest eine Formschlusselement insbesondere auch der Positionierung der Verbindungseinheit zu der Griffeinheit und/oder des zumindest einen Formschlusselements zu der Durchgangsausnehmung. Durch die Ausgestaltung des Formschlusselements als Niet kann insbesondere eine vorteilhaft mechanische Verbindung bereitgestellt werden, welche nur durch Bruch lösbar ist.

Bei einer Herstellung werden insbesondere die Griffeinheit sowie die Verbindungseinheit bereitgestellt. Anschließend werden die Griffeinheit und die Verbindungseinheit zusammengeführt, wobei sich insbesondere das Formschlusselement durch die Durchgangsausnehmung erstreckt. Darauffolgend wird der zumindest eine hindurchstehende Schaft des Formschlusselements verformt. Dabei sind verschiedene, einem Fachmann als sinnvoll erscheinende Verformungsmöglichkeiten denkbar, wie beispielsweise durch Druck, wobei das Formschlusselement verdrückt bzw. angestaucht wird, oder durch Druck und Wärme, wobei das Formschlusselement mit einem geheizten Stempel verformt bzw. angestaucht wird. Der Druck ist dabei insbesondere so gewählt, dass ein kontrolliertes Stauchen erfolgt. Bei einem Aufbringen des Drucks wird insbesondere eine Ausbreitung des gestauchten Formschlusselements gesteuert. Vorzugsweise ist ein Stempel dafür insbesondere nicht flach, sondern konkav gewölbt. Eine runde Form der Berührungsfläche steuert insbesondere das Fließen des Materials des Formschlusselements. Vorzugsweise wird der Druck explizit nur im Bereich des zumindest einen Formschlusselements aufgebracht. Optional ist denkbar, dass der Stempel größer ist als das Formschlusselement, dass der Stempel nur zum Teil von dem zumindest einen Formschlusselement berührt wird und/oder dass in einer Endposition der Stempel die ganze Fläche und nicht nur das Formschlusselement berührt. Zur Herstellung der Nietverbindung ist insbesondere ein Gegenhalter nötig. Damit der Druck aufgebaut werden kann, muss insbesondere das zu drückende Teil abgestützt sein. Vorzugsweise ist der Gegenhalter dazu vorgesehen, an einer Abstützungsfläche, insbesondere der Verbindungseinheit, anzuliegen und einen Gegendruck bereitzustellen. Eine Geometrie der Abstützungsfläche am Körper kann insbesondere in ein Gesamtprodukt integriert sein, damit sie nicht auffällt. So ist beispielsweise denkbar, dass anstatt einer großen Abstützungsfläche mehrere kleinere Abstützungsflächen vorgesehen sind, die nicht auffallen. Die Abstützungsfläche kann vorzugsweise verschieden liegen. Ein Aufbau und/oder eine Lage der Abstützungsfläche kann deren Größe beeinflussen. Die Abstützungsflächen liegen in einem entsprechenden Bereich in einer Beziehung zum Aufbau des Produkts und zu dem nötigen Druck, dem standgehalten werden muss. Insbesondere muss bei der Ausgestaltung der Verbindungseinheit berücksichtigt werden, dass bei Anwendung von Druck ein Gegenhalter bzw. ein Anschlag vorhanden sein muss, damit das Formschlusselement nicht ausweichen kann. Die Größe der Abstützungsflächen (kumuliert) ist mindestens gleich groß, vorzugsweise größer wie der Querschnitt des zu verformenden Teils.

Alternativ oder zusätzlich zu dem zumindest einen Formschlusselement der Verbindungseinheit wäre auch denkbar, dass die Verbindungseinheit mittels einer Klebeverbindung mit der Anwendungseinheit und/oder der Griffeinheit verbunden ist. Vorzugsweise kann die Verbindungseinheit mittels einer Klebeverbindung mit der Griffeinheit, insbesondere mit dem materiellen Griffkörper, verbunden werden. Die Klebeverbindung ist vorzugsweise mit einer Formschlussverbindung kombinierbar. Vorzugsweise sind zwischen der Verbindungseinheit und der Anwendungseinheit und/oder der Griffeinheit insbesondere Vertiefungen und/oder Kämme vorgesehen, welche eine Formschlusskontur ausbilden. Es ist insbesondere an dem materiellen Griffkörper eine Formschlusskontur vorgesehen. Der Formschluss soll insbesondere einen Teil der Kräfte aufnehmen, damit nicht alle Haltekräfte über den Klebstoff abgestützt werden müssen. Insbesondere können schädliche Scherkräfte abgefangen werden. Die Formschlusskontur bringt daher insbesondere eine bessere, stabilere Verbindung. Zusätzlich wird durch die Formschlusskontur verhindert, dass sich Verunreinigungen sammeln können. Vorzugsweise ist an der Verbindungseinheit eine zu der Formschlusskontur der Griffeinheit korrespondierende Formschlusskontur angeordnet. Eine Positionierung der Verbindungseinheit relativ zu der Griffeinheit erfolgt insbesondere über die Formschlusskonturen. Alternativ wäre jedoch auch denkbar, dass eine Positionierung der Einheiten über Werkzeuge für die Verbindungserstellung erfolgt.

Es sind verschiedene, einem Fachmann als sinnvoll erscheinende Klebstoffe für die Klebeverbindung denkbar. Der Klebstoff muss insbesondere für die Verbindung von Papierwerkstoff und Kunststoff geeignet sein. Der Klebstoff kann insbesondere von einem Schmelzklebstoff gebildet sein. Bei dem Klebstoff sind als Kenngrößen insbesondere die offene Zeit sowie die Abbindezeit relevant. Als offene Zeit bezeichnet man bei Klebeverfahren und Klebetechniken die zulässige Zeitspanne vom Beginn des Auftragens des Klebstoffs bis zum Zusammenfügen der Fügeteile und bis die Abbindezeit beginnt. Bei Schmelzklebstoffen ist die offene Zeit die Zeit, in der sich ein flüssiger Schmelzklebstoff auf den zu verbindenden Oberflächen verteilt oder verteilt wird. Da sich die Viskosität eines Schmelzklebstoffes nach dem Auftrag erhöht, ist die Offenzeit bei Schmelzklebstoffen zeitlich begrenzt. Die Abbindezeit ist ein Begriff aus der Klebstofftechnik und ist die Zeitspanne, die ein Klebstoff zum Erreichen einer für eine bestimmungsgemäße Beanspruchung erforderlichen Festigkeit benötigt, beziehungsweise die Zeit, die der Klebstoff benötigt, um von einem flüssigen in einen festen Zustand überzugehen. Falls im Klebeprozess Pressdruck aufgebaut wird, kann dieser nach Ende der Abbindezeit aufgehoben werden. Bei manchen Klebstoffen ist bis zur Weiterverarbeitung noch eine Nachbindezeit nötig, bis die Endfestigkeit erreicht ist. Nach der Abbindezeit hat der Klebstoff seine innere Festigkeit, die Kohäsion, erreicht. Bei einer Verarbeitung ist insbesondere eine lange offene Zeit gut, während dieser Zeit kann der Klebstoff aufgebracht und können die verschiedenen Teile zusammengebracht werden. Bei großen Werten kann die Verarbeitung insbesondere langsamer erfolgen. Die offene Zeit kann in der Regel nicht von außen beschleunigt werden und ist vom Klebstoff her gegeben. Zu lange offene Zeit oder zu viel Zeitreserve kann daher den gesamten Prozess verlangsamen. Die offene Zeit ist vorzugsweise länger als die Abbindezeit. Die offene Zeit beträgt insbesondere von 2 Sekunden bis 40 Sekunden, vorzugsweise von 5 Sekunden bis 30 Sekunden. Ab der Abbindezeit gilt insbesondere je kürzer desto besser. Die Abbindezeit definiert dabei die Zeit nach der offenen Zeit, bis der Klebstoff die Teile fest verbunden hat. Während der Abbindezeit wird insbesondere beispielsweise Druck aufgebracht, damit die Teile so zusammengefügt werden, wie sie schließlich angeordnet sein sollen oder es wird ohne Druckanwendung eine Positionierung/Lage der Teile einander gegenüber sichergestellt. Nach der Abbindezeit sind die Teile fest verbunden. Die Abbindezeit beträgt insbesondere von 0,5 Sekunden bis 10 Sekunden, vorzugsweise von 1 Sekunde bis 5 Sekunden. Die Abbindezeit ist vorzugsweise kürzer als die offene Zeit. Bei der Herstellung muss insbesondere ein Timing beim Auftragen des Klebstoffs eingehalten werden.

Bei der Klebeverbindung ist insbesondere keine vollflächige Applikation vorgesehen, eine Verteilung der Klebemasse erfolgt beispielsweise über das Verdrücken während der offenen Zeit. Es sind verschiedene, einem Fachmann als sinnvoll erscheinende Applikationsformen denkbar, beispielsweise als Klebepunkt, als mehrere Klebepunkte oder als Raupe. Die Applikation in Punkten oder als Raupe mit anschließendem Verdrücken ergibt in der Regel insbesondere keine komplett vollflächige Verteilung, es ist daher insbesondere eine Kombination mit Strukturen zur Aufnahme von Kräften sinnvoll.

Der Klebstoff für die Klebeverbindung sollte vorzugsweise mundhygienetauglich und damit lebensmitteltauglich sein. Ferner sollte vorzugsweise eine industrielle Applikation möglich sein, sodass eine Applikation in einem automatischen System möglich ist. Des Weiteren sollten Klebstoff und Materialien der Griffeinheit und/oder der Verbindungseinheit kompatibel sein. Mittels einer Klebstoffverbindung kann insbesondere ein spielfreies Montieren ermöglicht werden. Ferner werden Spalten insbesondere zuverlässig mit Klebstoff aufgefüllt, da sich der Klebstoff unter Druck verteilt. Es kann insbesondere auch ein Stehen von Flüssigkeit in Spalten vermieden werden. Für eine bessere Haftung wären zudem Oberflächenstrukturen an der Griffeinheit und/oder der Verbindungseinheit denkbar, damit der Klebstoff besser hält. Alternativ oder zusätzlich wäre auch eine Kombination mit einer Formschlussverbindung denkbar, damit gewisse Kräfte mechanisch aufgenommen werden können.

Alternativ oder zusätzlich zu dem Formschlusselement und/oder der Klebeverbindung wäre auch eine Steckverbindung denkbar. Die Anwendungseinheit kann dabei insbesondere durch Einstecken und/oder Aufstecken auf den materiellen Griffkörper mit dem materiellen Griffkörper verbunden werden. Dabei kann das Körperpflegeprodukt insbesondere eine als Steckverbindungseinheit ausgebildete Verbindungseinheit aufweisen, welche zu einer lösbaren Steckverbindung der Anwendungseinheit mit der Griffeinheit vorgesehen ist. Dabei wäre insbesondere auch denkbar, dass die Anwendungseinheit als ein Wechselkopf ausgebildet ist, wobei die Steckverbindungseinheit direkt zwischen der Anwendungseinheit und der Griffeinheit angeordnet ist. Die Steckverbindungseinheit ist insbesondere zumindest teilweise einstückig mit der Anwendungseinheit und/oder zumindest teilweise einstückig mit der Griffeinheit ausgebildet. Vorzugsweise weist die Steckverbindungseinheit zumindest ein erstes Steckverbindungselement auf, welches fest mit der Griffeinheit verbunden ist, und weist zumindest ein zu dem ersten Steckverbindungselement korrespondierendes zweites Steckverbindungselement auf, welches fest mit der Anwendungseinheit verbunden ist. Über die Steckverbindungseinheit kann insbesondere die Anwendungseinheit von dem materiellen Griffkörper getrennt werden. Die Steckverbindungseinheit ist insbesondere zu einer Bereitstellung einer lösbaren oder unlösbaren Rastverbindung vorgesehen. Das erste Steckverbindungselement bildet insbesondere einen Rastfortsatz aus, während das zweite Steckverbindungselement eine zu dem ersten Steckverbindungselement korrespondierende Rastausnehmung ausbildet. Es wäre jedoch auch eine getauschte und/oder eine andere, einem Fachmann als sinnvoll erscheinende Ausgestaltung der Steckverbindungseinheit denkbar.

Alternativ oder zusätzlich zu dem Formschlusselement wäre auch eine Schweiß- oder Siegelverbindung denkbar. Hierfür kommen beispielsweise Ultraschall-Schweißverfahren ,Reibschweißverfahren oder Siegeln in Frage. Für die Schweißverbindung können insbesondere unterstützende Mittel, wie beispielsweise Lacke und/oder Beschichtungen am Papierwerkstoff des materiellen Griffkörpers, welche ermöglichen, dass die Verbindung durch die Schweißung entsteht, eingesetzt werden. Für Schweißverfahren ist insbesondere eine Zugänglichkeit der Schweißstelle, beispielsweise für Sonotroden bzw. Siegelstempel notwendig.

Grundsätzlich ist auch eine Kombination von Verbindungsmöglichkeiten, insbesondere der oben beschriebenen Verbindungsmöglichkeiten, denkbar. Generell sind Kombinationen der verschiedenen Verbindungsmöglichkeiten möglich. So ist beispielsweise eine Kombination einer Klemmverbindung und einer Schnappverbindung denkbar, wobei der materielle Griffkörper beispielsweise in eine Ausnehmung der Verbindungseinheit eingefahren wird und zumindest mittels eines Formschlusselements verrastet wird. Zusätzlich können Führungen bei der Verbindungseinheit vorgesehen sein, welche den materiellen Griffkörper seitlich führen. Die Führung kann auch mit einem zusätzlichen Formschlusselement umgesetzt werden oder das Formschlusselement ist nur seitlich umgesetzt. Ferner wäre auch eine Kombination einer Nietverbindung und einer Klebeverbindung denkbar. Bei einem Körperpflegeprodukt mit einer Griffeinheit aus mehreren Schichten ist beispielsweise denkbar, dass die Verbindungseinheit zwischen die Schichten der Griffeinheit eingebracht bzw. geklemmt wird, wobei die Schichten und die Verbindungseinheit verbunden werden. Die Schichten können dabei beispielweise mittels einer Nietverbindung mit der Verbindungseinheit verbunden werden, wobei insbesondere auf einander gegenüberliegenden Seiten der Verbindungseinheit Formschlusselemente zu einer Verbindung mit den Schichten vorgesehen sind. Alternativ können die Schichten und die Verbindungseinheit auch lediglich durch Kleben, Schweißen oder Siegeln verbunden werden. Bevorzugt ist jedoch eine Kombination einzelner genannter Verfahren, da die Griffeinheit Teile enthält, die nicht an der Verbindungseinheit anliegen. In diesem Zusammenhang wäre auch ein Körperpflegeprodukt mit Doppelschale denkbar, wobei zwei geformte Schalen der Griffeinheit einander gegenüber angeordnet sind, sodass sich dazwischen mindestens teilweise ein Hohlraum durch die Schalen ausbildet, und wobei die Verbindungseinheit zwischen den Schalen geklemmt wird.

Ferner wird vorgeschlagen, dass die Anwendungseinheit zumindest einen Grundkörper aufweist, welcher fest mit der Verbindungseinheit verbunden ist. Der Grundkörper der Anwendungseinheit kann bei einer Zahnbürste beispielsweise von einem Borstenplättchen oder einem Bürstenkopf gebildet sein. Die Verbindungseinheit kann beispielsweise mittels einer Klebeverbindung mit der Anwendungseinheit verbunden sein. Andere in dieser Schrift genannte Verbindungsmöglichkeiten sind auch möglich. Vorzugsweise kann die Verbindungseinheit die Anwendungseinheit enthalten oder es kann eine Schnittstelle zwischen der Anwendungseinheit und der Verbindungseinheit vorgesehen sein. Die Schnittstelle zu der Griffeinheit und die Schnittstelle zu der Anwendungseinheit können separat hergestellt sein und anschließend verbunden werden. So können beispielsweise die Anwendungseinheit und die Verbindungseinheit hergestellt und anschließend verbunden werden und darauffolgend kann die mit der Verbindungseinheit verbundene Anwendungseinheit mit der Griffeinheit verbunden werden. Dadurch kann insbesondere eine vorteilhaft einfache Montage erreicht werden. Es kann insbesondere eine Anzahl an Bauteilen gering gehalten werden.

Des Weiteren wird vorgeschlagen, dass die Anwendungseinheit zumindest einen Grundkörper aufweist, welcher wechselbar mit der Verbindungseinheit verbunden ist. Die Anwendungseinheit kann beispielsweise über eine lösbare Steckverbindung und/oder Rastverbindung mit der Verbindungseinheit verbunden sein. Vorzugsweise weist die Verbindungseinheit ein erstes Kopplungselement auf und die Anwendungseinheit weist ein zu dem ersten Kopplungselement korrespondierendes Kopplungselement auf. Das erste Kopplungselement und das zweite Kopplungselement bilden insbesondere eine Rasteinheit aus. Unter einer "Rasteinheit" soll dabei insbesondere eine Einheit mit wenigstens einem Rastelement verstanden werden, das bei einem Befestigungsvorgang elastisch ausgelenkt wird, um anschließend durch eine innere Spannkraft hinter einem korrespondierenden Rastelement einzurasten. Unter "lösbar" soll in diesem Zusammenhang insbesondere "zerstörungsfrei trennbar" verstanden werden. Vorzugsweise ist die Anwendungseinheit in diesem Zusammenhang insbesondere als Zahnbürstenwechselkopf, als Rasierwechselkopf oder dergleichen ausgebildet.

Es wird ferner vorgeschlagen, dass die Anwendungseinheit zumindest einen Grundkörper aufweist, wobei das Formschlusselement einstückig mit dem Grundkörper der Anwendungseinheit ausgebildet ist. Vorzugsweise ist die Verbindungseinheit einstückig mit der Anwendungseinheit ausgebildet. Bevorzugt sind die Verbindungseinheit und die Anwendungseinheit beispielsweise gemeinsamen in einem Spritzgussverfahren hergestellt. Dadurch kann insbesondere eine vorteilhaft einfache Montage erreicht werden. Es kann insbesondere eine Anzahl an Bauteilen gering gehalten werden. Ferner kann insbesondere ein vorteilhaft stabiles Körperpflegeprodukt bereitgestellt werden.

Bei einer Ausgestaltung des Körperpflegeprodukts als Zahnbürste weist die Anwendungseinheit insbesondere ein Borstenfeld auf. Das Borstenfeld kann dabei insbesondere aufgesetzt sein. Alternativ wäre auch denkbar, dass die Anwendungseinheit genietet ist, wobei das Borstenfeld so gestaltet ist, dass die Anordnung eines Gegenhalters im Bereich von Formschlusselementen möglich ist. Die Anwendungseinheit weist insbesondere ein Borstenplättchen auf. Das Borstenfeld kann dabei in einem AFT-Verfahren hergestellt sein. Dabei ist insbesondere auch denkbar, dass das Borstenplättchen aus einem Papierwerkstoff besteht, wobei eine Verbindung zu der Verbindungseinheit beispielsweise mittels Kleben herstellbar ist. Alternativ wäre denkbar, dass das Borstenplättchen aus Kunststoff insbesondere zumindest aus einer Hartkomponente besteht, wobei eine Verbindung zu der Verbindungseinheit beispielsweise mittels Schweißen, Siegeln, Kleben, Nieten, Rasten, Schnappen oder einer Kombination davon erfolgen kann. Das Borstenplättchen kann dabei insbesondere direkt eine Verbindungsstruktur, wie beispielsweise Niete, zu einer Verbindung mit der Verbindungseinheit und/oder direkt mit der Griffeinheit aufweisen. Alternativ wäre jedoch auch eine Ausgestaltung der Anwendungseinheit ohne Borstenplättchen denkbar, wobei das Borstenfeld weiterhin in einem AFT-Verfahren hergestellt werden kann. Hierbei kann insbesondere das Schmelzbad zur Verankerung genutzt werden. Die Anwendungseinheit kann hier mittels Kleben, Einklemmen oder Siegeln bzw. Schweißen, Nieten, Rasten, Schnappen oder einer Kombination davon mit der Verbindungseinheit und/oder direkt mit der Griffeinheit verbunden werden. Alternativ können die Borsten in das Borstenplättchen mittels Anker gestanzt werden. Dabei sind konventionelle Verfahren, bei welchen das Borstenplättchen mit Borstenlöchern und Borsten versehen wird, bekannt (Ankerstanzverfahren). Die Anwendungseinheit kann hier mittels Kleben, Nieten oder Siegeln bzw. Schweißen oder einer Kombination davon mit der Verbindungseinheit und/oder direkt mit der Griffeinheit verbunden werden. Das Borstenplättchen kann dabei insbesondere direkt eine Verbindungsstruktur, wie beispielsweise Niete, zu einer Verbindung mit der Verbindungseinheit und/oder direkt mit der Griffeinheit aufweisen. Alternativ können die Borsten direkt an einen Grundkörper der Anwendungseinheit angespritzt werden. Die Borsten und die Anwendungseinheit können dabei einstückig ausgebildet sein. Es können dabei eine oder mehrere Materialkomponenten verwendet werden. Beispielsweise kann eine Hartkomponente und/oder eine Weichkomponente und/oder ein Material für gespritzte Borsten verwendet werden. Die Anwendungseinheit kann hier beispielsweise mittels Siegeln bzw. Schweißen, Nieten, Schnappen, Rasten und/oder Umspritzen oder einer Kombination davon mit der Verbindungseinheit und/oder direkt mit der Griffeinheit verbunden werden. Der Grundkörper der Anwendungseinheit kann dabei insbesondere direkt eine Verbindungsstruktur, wie beispielsweise Nieten, zu einer Verbindung mit der Verbindungseinheit und/oder direkt mit der Griffeinheit aufweisen, welche direkt auf einer Unterseite des Grundkörpers der Anwendungseinheit angespritzt ist.

Die Anwendungseinheit weist vorteilhaft zumindest eine Reinigungseinheit, insbesondere einen Zahnbürstenkopf, mit Borsten auf. Die Reinigungseinheit weist zudem vorteilhaft zumindest einen Borstenträger, beispielsweise einen Bürstenkopfgrundkörper, insbesondere den Grundkörper, auf. Ferner wird vorgeschlagen, dass ein Grundkörper der Anwendungseinheit vollständig aus einer Hartkomponente ausgebildet ist und einen Borstenträger ausbildet. Alternativ oder zusätzlich wird vorgeschlagen, dass das Körperpflegeprodukt ein von dem Grundkörper aufgenommenes Borstenplättchen aufweist, welches eine Mehrzahl von Borstenbündeln umfasst. Hierzu werden zunächst mittels Spritzgießen Borstenplättchen mit Durchgangslöchern gefertigt, durch welche anschließend Borsten geführt werden. Vorzugsweise werden die Borsten anschließend auf einer Rückseite mindestens bündelweise verbunden, insbesondere verschmolzen, vorzugsweise miteinander und/oder mit dem entsprechenden Borstenplättchen. Auf diese Weise beborstete Borstenplättchen können sodann mit einem Grundkörper, insbesondere der Verbindungseinheit, verbunden, z.B. verschweißt, gesiegelt und/oder verklebt werden, oder mechanisch mittels Nieten, Verstemmen, vorzugsweise mittels eines Ultraschallschweißens, verbunden werden. Hierzu weist der Grundkörper vorzugsweise eine entsprechende Struktur zur Aufnahme / Verbindung auf, welche zur Verbindung des Borstenplättchens genutzt werden kann. Alternativ kann das Borstenplättchen mit in dieser Schrift genannten Verfahren direkt d.h. ohne zusätzlichen Grundkörper mit dem Griffkörper verbunden werden.

Ferner wäre denkbar, dass das Borstenplättchen aus einem Papierwerkstoff hergestellt ist. Das Borstenplättchen kann aus einem Papierwerkstoff gestanzt sein. Dazu können eine oder mehrere Schichten aus Papierwerkstoff bereitgestellt werden, die den Körper des Borstenplättchens bilden. Die Ausnehmungen werden anschließend gebildet, dafür kann ein Stanzprozess, Schneiden z.B. Laserschneiden, Fräsen/Bohren oder ein anderes geeignetes Verfahren zur Bearbeitung von Papierwerkstoff eingesetzt werden. Die Borsten werden wiederum im AFT-Verfahren mit dem Borstenplättchen verbunden, wobei dieses dann im Anschluss in der entsprechenden Ausnehmung in der Griffeinheit oder auf der Griffeinheit fixiert werden kann.

Die Anwendungseinheit weist vorteilhaft zumindest eine Reinigungseinheit, insbesondere einen Zahnbürstenkopf, mit Borsten auf. Die Reinigungseinheit weist zudem vorteilhaft zumindest einen Borstenträger, beispielsweise einen Bürstenkopfgrundkörper, auf. Zumindest einige oder alle der Borsten sind vorteilhaft konventionell extrudierte Borsten. Borsten können hierbei insbesondere zumindest eine Hartkomponente und/oder zumindest eine Weichkomponente umfassen. Vorzugsweise sind die Borsten zumindest teilweise oder vollständig aus Polyamid (PA) und/oder aus Polyester (PBT, PET, PTT) gefertigt, wobei beliebige andere Materialien (wie z.B. die aufgeführten Hartkomponenten) denkbar sind und auch in dieser Schrift aufgeführte nachhaltige Materialien insbesondere abbaubare (wie z.B. PLA) möglich sind. Ferner ist denkbar, dass zumindest einige der Borsten am nutzungsseitigen Ende eine Zuspitzung und/oder einen veränderlichen Querschnitt aufweisen. Vorzugsweise sind die Borsten aus einem einzelnen, insbesondere auch gemischten, Material ausgebildet. Es sind aber auch Borsten mit mehreren Komponenten denkbar, die insbesondere mittels zumindest einer Koextrusion herstellbar und/oder hergestellt sein können. Die Borsten können beispielsweise mittels Extrusion, Ablängen und/oder Nachbearbeitung herstellbar und/oder hergestellt sein. Im Gegensatz zu gespritzten Borsten oder gummielastischen Massage- und Reinigungselementen, welche mittels Spritzguss hergestellt sind, werden konventionelle Borsten extrudiert, geschnitten, bearbeitet und am Zahnbürstengriff mittels eines angepassten Verfahrens eingesetzt, wie beispielsweise mittels des Ankerstanz-Verfahrens oder eines ankerlosen Verfahrens.

Insbesondere kommen zylindrische oder zugespitzte Borsten mit rundem Querschnitt infrage, wobei beliebige andere Querschnitte wie beispielsweise polygonale, dreieckige, rechteckige, quadratische, elliptische, sternförmige, trapezförmige, kreuzförmige, parallelogrammförmige, rhombusförmige oder beliebige andere Querschnitte denkbar sind. Insbesondere können unterschiedliche Borsten in einem Borstenbündel, aber auch unterschiedliche Borstenbündel, insbesondere jeweils mit einer bestimmten Art von Borsten, verwendet werden. Borsten und/oder Borstenbündel können hierbei regelmäßig, aber auch unregelmäßig angeordnet sein. Insbesondere können sich in Gruppen und/oder benachbart angeordnete Borsten und/oder Borstenbündel hinsichtlich zumindest eines Merkmals wie beispielsweise einer Länge, eines Durchmessers, eines Materials, einer Farbe, einer Materialhärte, einer Geometrie, einer Anspitzung und dergleichen, insbesondere abwechselnd, unterscheiden. Vorzugsweise weisen die Borsten einen Durchmesser, insbesondere senkrecht zu ihrer Längsachse, von wenigstens 0,075 mm und/oder von höchstens 0,25 mm auf. Vorteilhaft weisen die Borsten eine Querschnittsfläche, insbesondere senkrecht zu ihrer Längsachse, von wenigstens 0,002 mm² und/oder von höchstens 0,2 mm² auf. Im Fall von Borsten, die im Kosmetikbereich eingesetzt werden, beispielsweise Borsten eines zusätzlichen Anwendungselements, können auch dünnere Borsten und/oder Borsten mit einem kleineren Querschnitt verwendet werden, insbesondere Borsten mit einem Durchmesser, insbesondere senkrecht zu ihrer Längsachse, von wenigstens 0,025 mm und/oder von höchstens 0,2 mm und/oder mit einer Querschnittsfläche, insbesondere senkrecht zu ihrer Längsachse, von wenigstens 0,001 mm² und/oder von höchstens 0,15 mm². Im Fall von zugespitzten Borsten ist insbesondere Polyester (z.B. PBT, PET, PTT) als Material geeignet, wobei auch nachhaltige Materialien möglich sind, wobei eine Zuspitzung mechanisch und/oder chemisch erzeugt sein kann. Andere Materialien sind jedoch ebenso denkbar. Vorzugsweise sind die Borsten in Längsrichtung gerade, es sind jedoch in Längsrichtung auch gewellte und/oder gedrillte und/oder wendelförmige und/oder gedrehte Borsten denkbar sowie insbesondere auch Kombinationen unterschiedlicher Borsten. Ferner sind Borsten mit einer glatten Oberfläche denkbar, ebenso wie Borsten mit strukturierter und/oder texturierter Oberfläche.

Ferner sind die Borsten, insbesondere als Borstenbündel, vorzugsweise mittels zumindest eines Ankerstanz-Verfahrens oder eines ankerlosen Verfahrens oder dergleichen verarbeitet, insbesondere an dem Borstenträger befestigt. Vorzugsweise weist der Borstenträger eine Mehrzahl von, insbesondere gebohrten und/oder im Spritzgussverfahren geformten, Borstenaufnahmen, insbesondere Löchern für Borstenbündel, auf. Im Fall eines Ankerstanzens ist beispielsweise denkbar, dass zunächst ein Grundkörper, insbesondere aus einer Hartkomponente, vorzugsweise des Bürstenkopfs, mittels eines Spritzgießens gefertigt wird, wobei vorteilhaft Sacklöcher für Borstenbündel bei dem Spritzgießen geformt werden. Selbstverständlich ist jedoch auch ein anschließendes Bohren von Sacklöchern denkbar. Vorzugsweise werden anschließend Borsten beziehungsweise Borstenbündel gefaltet und mittels zumindest eines Ankers in jeweils einem Sackloch befestigt, insbesondere mittels eines Einstanzens. Ebenso ist ein Schlingenstanzen denkbar.

Alternativ sind, wie erwähnt, auch ankerlose Verfahren denkbar, wobei vorteilhaft Borsten beziehungsweise Borstenbündel nicht gefaltet werden. Borsten beziehungsweise Borstenbündel weisen in diesem Fall im Vergleich zu einem Ankerstanzen in etwa die halbe Länge auf. Beispielsweise ist hierbei denkbar, dass die Borstenbündel zunächst vereinzelt verschmolzen und/oder ihre Borstenenden, insbesondere anschließend, zu deren Befestigung z.B. umspritzt oder mittels Materialverdichtung des Bürstenkopfes fixiert werden können. Hierbei können vorteilhaft Borstenbündel zusammengeführt werden.

Ferner ist denkbar, dass die Anwendungseinheit aus einem Grundkörper und einem Borstenplättchen, welches mit Borsten und/oder alternativen Reinigungselementen besetzt ist, zusammengesetzt ist. Hierzu werden zunächst mittels Spritzgießens Borstenplättchen mit Durchgangslöchern gefertigt, durch welche anschließend Borsten geführt werden. Vorzugsweise werden die Borsten anschließend auf einer Rückseite mindestens bündelweise verbunden, insbesondere verschmolzen, vorzugsweise miteinander und/oder mit dem entsprechenden Borstenplättchen. Auf diese Weise beborstete Borstenplättchen können sodann mit einem Grundkörper, insbesondere einem Bürstenkopf, verbunden, z.B. verschweißt, gesiegelt und/oder verklebt werden, und/oder mechanisch, mittels Nieten, Verstemmen, vorzugsweise mittels eines Ultraschallschweißens oder einer Kombination davon, verbunden werden. Hierzu weist der Grundkörper, insbesondere der Bürstenkopf, vorzugsweise eine Ausnehmung auf, in welche das Borstenplättchen eingesetzt werden kann. Alternativ kann das Borstenplättchen auch auf der Oberfläche des Grundkörpers aufgesetzt werden. Als bekanntes Herstellungsverfahren ist in diesem Zusammenhang das Anchor-Free-Tufting-Verfahren zu nennen, das insbesondere ein Zusammenführen von Borstenbündeln ermöglicht. Als Unterseite des Borstenplättchens wird insbesondere eine Seite bezeichnet, welche in eine Ausnehmung des Grundkörpers bzw. auf den Grundkörper gelegt wird und in Richtung Rückseite des Körperpflegeprodukts zeigt. Entsprechend zeigt die Oberseite des Borstenplättchens in Richtung der Vorderseite des Körperpflegeprodukts. Der Grundkörper kann aus einer Hartkomponente aus einem nachhaltigen Material oder einem Papierwerkstoff bestehen. Alternativ kann der Grundkörper auch weggelassen werden und das Borstenplättchen kann direkt mit dem Griffkörper mit erwähnten Verfahren verbunden werden.

Alternativ kann das Borstenplättchen aus einem Papierwerkstoff oder einem Werkstoff aus Fasern, bzw. Zellstoff. oder aus Holz hergestellt werden. Das Borstenplättchen aus Papierwerkstoff ist außerhalb des Borstenfeldes mit einem Randbereich versehen, welcher mit einem Verbindungsmittel wie Siegellack, Leim oder Ähnlichem versehen ist und welcher eine Siegelung des Borstenplättchens mit einer Griffeinheit oder einem Grundkörper z.B. aus einem Papierwerkstoff oder einer Hartkomponente oder einem nachhaltigen Material ermöglicht. Dabei wird insbesondere der genannte Randbereich mit der Griffeinheit bzw. mit dem Grundkörper verbunden. Weiter kann der Randbereich des Borstenplättchens auch mit einem effektiven Absatz versehen sein, sodass sich auf der Rückseite des Borstenplättchens eine Ausnehmung ausbildet, die sicherstellt, dass die Schmelze der Borsten nicht über den Rand des Borstenplättchens reicht bzw. auch den Randbereich nicht bedeckt.

Ein Borstenplättchen aus Papierwerkstoff oder Holz wird mittels eines geeigneten Verfahrens aus flächigem Papier oder Holzblättern (z.B. Furnier) gebildet. Mögliche Verfahren sind z.B. Stanzen, Laserschneiden oder Schneiden. Der flächige Bereich des Borstenplättchens hat eine Dicke von 0,1 mm - 3,0 mm, vorzugsweise 0,3 mm - 2,0 mm, besonders bevorzugt 0,5 mm - 1,5 mm.

Alternativ kann ein mit den Borsten / Reinigungselementen zusammen hergestelltes, vorzugsweise einstückiges Borstenplättchen realisiert werden. Dabei kann alles in einem Spritzvorgang hergestellt werden. Bevorzugt wird dafür Material für gespritzte Borsten verwendet. Zusätzlich kann auch eine Hartkomponente und/oder eine Weichkomponente eingesetzt werden. Auch dieses Borstenplättchen kann wiederum mit den verschiedensten erwähnten Verbindungstechniken mit dem Grundkörper verbunden werden, beispielsweise mittels Kleben, Siegeln, Schweißen, Verstemmen, Vernieten oder eine Kombination davon.

Alternativ kann die Herstellung eines beborsteten Elements ohne Borstenplättchen vorgesehen sein. Die Herstellung bzw. Beborstung dieses Elements geschieht ähnlich zur Herstellung eines bestückten Borstenplättchens. Borsten werden in der nötigen Konfiguration bereitgestellt und an ihren Enden verschmolzen. Die Verschmelzung muss in diesem Fall so sein, dass sich über alle Borstenbündel und allfälligen weiteren Elementen ein Schmelzeteppich aus der Borstenkomponente ausbildet. Das beborstete Element wird im Anschluss auf einen Bürstenkörper/Grundkörper montiert bzw. verbunden. Hierbei ist das Element nicht mit einem Borstenplättchen ausgestattet, sondern ist in sich selbst über den eigenen Schmelzeteppich stabil. Der Schmelzeteppich bildet im Wesentlichen eine Fläche und dient so als Basis für die Verbindung mit dem Grundkörper oder direkt mit der Griffeinheit. Der Schmelzeteppich kann auch so geformt werden, dass er Verbindungselemente in Form von Vorsprüngen, Nieten, Kämmen oder anderen formschlüssigen Elementen aufweist. Auch dieses Element kann wiederum mit den verschiedensten erwähnten Verbindungstechniken mit dem Grundkörper bzw. der Griffeinheit verbunden werden, beispielsweise mittels Kleben, Siegeln, Schweissen, Verstemmen, Vernieten oder Kombinationen davon. Der Grundkörper kann hierbei mit einer Ausnehmung versehen sein, damit der Schmelzeteppich in dieser versenkt befestigt werden kann. Grundsätzlich ist aber auch eine Befestigung direkt auf der Oberfläche der Griffeinheit denkbar.

Anstelle eines Borstenplättchens können die Borsten / Reinigungselemente auch direkt am Grundkörper bzw. an der Griffeinheit angespritzt werden. Damit würde auch ein Grundkörper aus einem Papierwerkstoff direkt umspritzt.

Als weiteres Verfahren zur ankerlosen Beborstung kommt eine Fertigung, insbesondere ein Spritzgießen, eines Bürstenkopfs mit Durchgangslöchern für Borsten infrage. Borsten können anschließend durch die Durchgangslöcher geführt und auf einer Rückseite verschmolzen werden, insbesondere miteinander und/oder mit dem Bürstenkopf. Vorzugsweise erfolgt anschließend ein Überspritzen, insbesondere mit zumindest einer Weichkomponente, der verschmolzenen Bereiche und/oder des Bürstenkopfs.

Alternativ zum separaten Borstenplättchen ist das Borstenplättchen als ein Teil des Grundkörpers aus einem Papierwerkstoff oder Holz ausgebildet, bzw. damit einstückig verbunden. Analog wird der Bürstenkopf auch in dieser Variante mit Durchgangslöchern versehen. Die Borsten können auch in diesem Fall durch die Durchgangslöcher geführt werden und auf der Rückseite mindestens bündelweise verschmolzen werden, dies insbesondere miteinander und/oder mit dem Bürstenkopf. Die verschmolzenen Bereiche bzw. die Rückseite des Bürstenkopfs können/kann anschließend mit einem Deckelelement aus Papierwerkstoff verschlossen oder abgedeckt werden, indem das Deckelelement mit dem Bürstenkopf verbunden wird. Diesbezüglich kommen wieder die oben erwähnten Verbindungstechniken in Frage.

Zudem ist denkbar, zunächst einen Bürstenkopf mit Sacklöchern, beispielsweise mittels Spritzgießens und/oder mittels eines Bohrens der Sacklöcher und/oder mittels Schneiden/Stanzen, zu fertigen. Borsten werden in diesem Fall insbesondere zu Bündeln zusammengelegt und an einem Ende verschmolzen und/oder anderweitig verbunden. Der Bürstenkopf wird anschließend erwärmt und/oder mit Wasser oder Wasserdampf befeuchtet. Sodann können vorteilhaft Borstenbündel auf der verbundenen Seite in die Sacklöcher eingeführt und mittels eines Andrückens bzw. Komprimierens des Bürstenkopfs verankert werden. Insbesondere verformen bzw. komprimieren sich hierbei die erwärmten und/oder befeuchteten Sacklöcher bzw. der Bürstenkopf, sodass die Borstenbündel in denselben verankert werden. Gleichzeitig kann auch der Bürstenkopf verformt werden. Dieses Verfahren kann für Hartkomponenten, vorzugsweise mittels zusätzlicher Befeuchtung, aber auch für Papierwerkstoff bzw. Holz eingesetzt werden.

Vorzugsweise gehen Materialien gespritzter Borsten bei einem Spritzgussprozess, insbesondere einem Zwei- und/oder Mehrkomponentenspritzgießen, keinen Materialschluss mit anderen Weichkomponenten und/oder Hartkomponenten und/oder Papierwerkstoff und/oder Holz und/oder anderen Materialien des Körperpflegeprodukts ein. Bevorzugt werden gespritzte Borsten vielmehr mittels eines Formschlusses, beispielsweise mittels zumindest eines Hinterschnitts und/oder zumindest eines Durchbruchs am Grundkörper der Anwendungseinheit und/oder mittels zumindest einer zumindest teilweisen Umspritzung mit Weichkomponenten und/oder Hartkomponenten verbunden, wobei insbesondere eine Schwundverbindung und/oder eine Schrumpfverbindung denkbar sind. Es ist jedoch auch eine Verbindung mittels zumindest eines Materialschlusses insbesondere mit affinen bzw. kompatiblen Kunststoff-Materialien bzw. -Komponenten denkbar.

Für sämtliche erwähnten möglichen Spritzgussprozesse ist grundsätzlich ein Ein-, Zwei- und/oder Mehrkomponentenspritzguss denkbar. Verwendete Materialien, insbesondere unterschiedliche Weichkomponenten und/oder Hartkomponenten und/oder Material für gespritzte Borsten, können hierbei, wie erwähnt, stoffschlüssig und/oder formschlüssig verbunden werden und/oder sein. Auch eine Ausbildung von gelenkigen bzw. beweglichen oder flexiblen Verbindungen mittels geeigneter Spritzgussschritte ist denkbar. Es kommen grundsätzlich beispielsweise Heißkanalverfahren, Kaltkanalverfahren und/oder Co-Injektionsverfahren in Frage.

Alternativ oder zusätzlich zu einem mit Borsten besetzten Bürstenkopf kann die Anwendungseinheit auch zumindest einen Zungenreiniger und/oder zumindest ein alternatives Reinigungs- und/oder Massageelement aufweisen. Diese können jeweils aus einer Weichkomponente, aus einer Hartkomponente oder aus einer Kombination von Weich- und Hartkomponente ausgebildet und/oder vorteilhaft mittels Spritzgießens herstellbar und/oder hergestellt sein.

Vorzugsweise sind gespritzte Borsten zumindest teilweise und vorteilhaft vollständig aus einem thermoplastischen Polyurethan-Elastomer (TPE-U) ausgebildet. Hierbei ist eine Verwendung eines modifizierten Polyurethan-Elastomers (TPE-U) denkbar, welches insbesondere bezüglich verbesserter Fließeigenschaften und/oder einer schnellen Erstarrung, insbesondere einer schnellen Kristallisation, vorteilhaft bereits bei höheren Temperaturen, modifiziert sein kann. Selbstverständlich sind aber auch andere Materialien denkbar, beispielsweise thermoplastische Polyester-Elastomere (TPE-E), thermoplastische Polyamid-Elastomere (TPE-A), Polyethylen (PE), beispielsweise in den Formen low density Polyethylen (LDPE) oder linear low density Polyethylen (LLDPE), oder dergleichen. Materialien für gespritzte Borsten weisen vorteilhaft eine Shore-D-Härte von wenigstens 0 und besonders vorteilhaft von wenigstens 30 und/oder von höchstens 100 und vorteilhaft von höchstens 80 auf. Insbesondere ist eine Shore-D-Härte eines Materials gespritzter Borsten vorteilhaft höher als eine Shore-D-Härte übriger verwendeter Weichkomponenten, beispielsweise für Griffelemente, Massageelemente, weitere Reinigungselemente oder dergleichen. Die für die Herstellung von gespritzten Borsten eingesetzten Materialien können nachhaltige Materialien sein.

Grundsätzlich ist ferner eine Verwendung wasserlöslicher Polymere denkbar, beispielsweise für Hartkomponenten, Weichkomponenten, gespritzte Borsten, Zungenreiniger oder andere Elemente des Körperpflegeprodukts.

Ebenso können für die Hartkomponente, die Weichkomponente und/oder das Material für gespritzte Borsten Biokunststoffe herangezogen werden, welche insbesondere aus nachwachsenden Rohstoffen gewonnen sein können, biologisch abbaubar, insbesondere kompostierbar, sein können und/oder aus einem recycelten und/oder recycelbaren Material bestehen können. Vorzugsweise ist das Material insbesondere aus einem Kunststoff gebildet. Vorzugsweise ist das biologisch abbaubare, insbesondere kompostierbare, und/oder recycelte und/oder recycelbare Material von einem Biokunststoff, insbesondere aus einem Kunststoff auf Basis nachwachsender Rohstoffe und/oder aus einem biologisch abbaubaren Kunststoff, gebildet. Das Material kann daher insbesondere fossil-basiert und bioabbaubar sein, wie beispielsweise PVOH, PCL, PBAT, PET oder PBS, auf nachwachsenden Rohstoffen basieren und bioabbaubar sein, wie beispielsweise PLA, PHA, Cellophan oder Stärke-Blends, oder auf nachwachsenden Rohstoffen basieren und nicht bioabbaubar sein, wie beispielsweise Ca, Bio-PE, Bio-PP, Bio-PA, Bio-PET. Es sind verschiedene, einem Fachmann als sinnvoll erscheinende Biokunststoffe denkbar, wie beispielsweise stärkebasierte Biokunststoffe, cellulosebasierte Biokunststoffe, Polyhydroxy-Alkanoate, wie insbesondere Polyhydroxybuttersäure (PHB), Polymilchsäure (PLA), aliphatische und/oder aromatische Copolyester, oder weitere Biokunststoffe wie beispielsweise Lignin-basierte Biokunststoffe. Bevorzugt kann die Anwendungseinheit bzw. die Griffeinheit zu einem Großteil aus einem biologisch abbaubaren, insbesondere kompostierbaren, und/oder aus einem recycelten Material bestehen. Vorzugsweise besteht das Körperpflegeprodukt nur aus einer Komponente. Insbesondere weist das Körperpflegeprodukt eine Hartkomponente auf. Der materielle Griffkörper der Griffeinheit kann mindestens teilweise aus der Hartkomponente bestehen. Die Hartkomponente und/oder die Weichkomponente und/oder das Material für gespritzte Borsten besteht vorteilhaft aus einem Biokunststoff, welcher insbesondere aus nachwachsenden Rohstoffen gewonnen sein kann. Als Rohstoffe kommen hierbei insbesondere Mais, Hanf, Zucker, Rizinusöl, Palmöl, Kartoffeln, Weizen, Zuckerrohr, Kautschuk, Holz, die Castor-Pflanze / der Wunderbaum und dergleichen infrage. Entsprechende mögliche Grundstoffe könnten beispielsweise Cellulose, Stärke, Milchsäure (PLA), Glucose, Chitin, Chitosan oder dergleichen sein, aus denen insbesondere entsprechende Biokunststoffe synthetisiert sein können.

Bei einer Ausgestaltung des Körperpflegeprodukts als Rasierer weist die Anwendungseinheit insbesondere eine Rasierklinge auf, wobei die Rasierklinge sowohl direkt mit der Verbindungseinheit verbunden hergestellt sein kann, als auch wechselbar ausgeführt sein kann, wobei die wechselbare Montage der Rasierklingen an der Verbindungseinheit erfolgt. Alternativ kann die Rasierklinge auch direkt an der Griffeinheit angebracht werden. Die Verbindungspunkte müssten in diesem Fall speziell behandelt werden, z.B. mittels Verdichten und/oder Oberflächenbehandlung des Papierwerkstoffes.

Vorzugsweise ist die Anwendungseinheit zumindest teilweise einstückig mit der Verbindungseinheit ausgebildet. Vorzugsweise sind bei einer Ausgestaltung des Körperpflegeprodukts als Rasierer mehrere Verankerungspunkte zwischen der Verbindungseinheit und der Griffeinheit vorgesehen. Dabei sind insbesondere drei Verbindungsmöglichkeiten kombiniert denkbar und zwar Nieten, Einklemmen und/oder seitliches Halten, zusätzlich oder alternativ sind auch Kleben, Schweißen oder Siegeln denkbar. Eine universelle Schnittstellengestaltung lässt dabei insbesondere einen einfachen Modellwechsel zu, sodass eine Verbindungseinheit mit Anwendungseinheit für verschiedene Griffe genutzt werden kann. Die verschiedenen Griffe können wiederum mit derselben Verbindungseinheit kombiniert werden.

Bei einer Ausgestaltung des Körperpflegeprodukts als Pinsel können die Borsten der Anwendungseinheit insbesondere direkt an die Verbindungseinheit angespritzt werden.

Des Weiteren wird vorgeschlagen, dass der materielle Griffkörper der Griffeinheit zumindest einen Kopfbereich aufweist, welcher zu einer Anbindung an der Anwendungseinheit vorgesehen ist, wobei der materielle Griffkörper der Griffeinheit in dem Kopfbereich zumindest Mittel beispielsweise in Form einer Vertiefung aufweist, welche zu einer Positionierung der Verbindungseinheit relativ zu der Griffeinheit vorgesehen ist. Die Vertiefung bildet insbesondere eine Formschlusskontur aus. Die Vertiefung ist insbesondere von einer Nut in dem materiellen Griffkörper gebildet, die sich parallel zu einer Längsachse der Griffeinheit erstreckt. Der materielle Griffkörper weist die Formschlusskontur auf, welche zu einer Formschlusskontur der Verbindungseinheit korrespondierend ausgebildet ist. Die Formschlusskontur der Verbindungseinheit zur anliegenden Griffeinheit schafft einen gewissen Formschluss durch die Geometrie neben dem Formschlusselement. Vorzugsweise sind zwischen der Verbindungseinheit und der Griffeinheit insbesondere Vertiefungen und/oder Kämme vorgesehen, welche jeweils die Formschlusskontur ausbilden. Die Formschlusskontur soll insbesondere einen Teil der Kräfte aufnehmen, damit nicht alle Kräfte über eine Nietverbindung und/oder eine Klebeverbindung abgestützt werden müssen. Insbesondere können schädliche Scherkräfte abgefangen werden. Die Formschlusskontur bringt daher insbesondere eine bessere, stabilere Verbindung. Vorzugsweise ist an der Verbindungseinheit eine zu der Formschlusskontur der Griffeinheit korrespondierende Formschlusskontur angeordnet. Eine Positionierung der Verbindungseinheit relativ zu der Griffeinheit erfolgt insbesondere über die Formschlusskonturen.

Es wird ferner vorgeschlagen, dass der materielle Griffkörper der Griffeinheit aus mindestens einer, bevorzugt genau einer Schicht aus einem Papierwerkstoff besteht. Vorzugsweise ist der materielle Griffkörper einteilig gefertigt. Der Papierwerkstoff kann dabei einlagig oder mehrlagig ausgebildet sein. Mehrlagiger Papierwerkstoff kann aus mehreren Lagen von Papier unterschiedlicher Dicke und/oder unterschiedlicher Eigenschaften und/oder zumindest teilweise aus unterschiedlichem Material bestehen, es ist aber auch denkbar, dass die aufgeführten Themen (Dicke, Eigenschaften, Material) der einzelnen Lagen zumindest teilweise identisch sind. Vorzugsweise können mehrere Lagen von Papier eine Schicht ausbilden, wobei der Körper aus einer oder mehreren Schichten bestehen kann. Dabei können äußere Lagen über von inneren Lagen verschiedene wasserabweisende Eigenschaften verfügen. Äußere Lagen können über eine von inneren Lagen verschiedene Farbe verfügen. Einzelne oder alle Lagen können im Wesentlichen aus Frischfaser bzw. Primärfaser bestehen. Einzelne oder alle Lagen können im Wesentlichen aus Sekundärfaser bestehen. Der Papierwerkstoff kann über zwischen 3 und 15 Lagen, bevorzugt zwischen 5 und 12 Lagen verfügen. Die Lagen können dabei durch Zusammenkleben, Laminieren oder Zusammenpressen, insbesondere ohne Einsatz von Klebstoff, hergestellt werden.

Dabei müssen insbesondere nicht alle Lagen aus demselben Werkstoff bestehen. So ist beispielsweise denkbar, dass äußere bedruckte Lagen aus dünnerem Papier sein könnten und/oder Decklagen andere Eigenschaften als mittlere Lagen haben könnten. So kann beispielsweise eine äußere Lage bereits vor einer Verarbeitung über eine Beschichtung, Lackierung oder eine Bedruckung verfügen. Alternativ oder zusätzlich sind auch unterschiedliche Farben der Lagen im Sandwich denkbar, sodass die Lagen klar sichtbar sind.

Das fertige Körperpflegeprodukt kann zumindest in einzelnen Bereichen aus mehreren Schichten des Papierwerkstoffs gebildet sein. Beispielsweise können zwei, drei, vier, fünf oder sechs Schichten gebildet werden. Die Anzahl Lagen werden entsprechend multipliziert.

Eine Faserausrichtung des Papierwerkstoffs ist vorzugsweise in Längsrichtung der Griffeinheit, sodass ein Knicken vermindert wird. Die Grammatur des Papierwerkstoffs, insbesondere der gesamten Schicht, beträgt insbesondere zwischen 1000 g/m² und 2500 g/m², vorzugsweise zwischen 1200 g/m² und 1700 g/m². Gemäß DIN 6730 kann zwischen verschiedenen Papierwerkstoffen hinsichtlich der Grammatur unterschieden werden. So handelt es sich bei bis 225g/mm² um Papier, während man ab 225g/mm² von Pappe spricht. Umgangssprachlich wird bis 250g/m² von Papier, von 150g/m² bis 600g/m² von Karton und ab 500 g/m² von Pappe gesprochen. Für Karton ist beispielsweise Vollpappe oder Wellpappe denkbar. Es gibt insbesondere verschiedene Varianten von Karton, insbesondere auch auf Recyclingbasis. Vorzugsweise besteht der Papierwerkstoff, insbesondere bei Karton, besonders bevorzugt bei Vollpappe, aus Frischfaser und/oder Sekundärfaser. Die Frischfaser bzw. Primärfaser ist dabei von Neumaterial, wie beispielsweise FSC Holz, gebildet. Die Sekundärfaser ist insbesondere von einem Recycling-Papier bzw. -Karton gebildet. Alternativ oder zusätzlich sind auch noch weitere Füllstoffe wie Gras für Graspapier denkbar. Papier wird aus Faserstoffen hergestellt, die heute vor allem aus dem Rohstoff Holz gewonnen werden. Es besteht insbesondere auch die Möglichkeit von holzfreier Cellulose. Bevorzugte Basis des Papierwerkstoffs ist jedoch Cellulose bzw. Zellstoff auf Basis von Holz. Mögliche Basis-Holzarten sind beispielsweise Pappel, Buche, Fichte, Kiefer oder Eukalyptus. Alternativ kann der Papierwerkstoff auch aus Gras, Bambus oder Papyrus gewonnen werden. Die wichtigsten Faserstoffe sind Zellstoff, Holzstoffe und Altpapierstoff. Gewisses Altpapier kann aufgrund von Druckerschwärze für den Einsatz in der Mundhygiene eher weniger geeignet sein. Außer dem Faserstoff oder einer Faserstoffmischung kann der Werkstoff häufig auch Füllstoffe und weitere Zusatzstoffe enthalten. Der Papierwerkstoff als Ganzes oder zumindest einzelne Lagen können einen Recycling-Anteil von zumindest 80% aufweisen, wobei der Papierwerkstoff als Ganzes oder zumindest einzelne Lagen einen Anteil an Primärfasern enthalten kann/können. Durch den Einsatz von Neumaterial bzw. Primärfaser können die Eigenschaften des Papierwerkstoffs in engerer Bandbreite gehalten werden und es kann u.a. eine bessere Reißfestigkeit und dadurch Stabilität erreicht werden. Es sind verschiedene, einem Fachmann als sinnvoll erscheinende Papierwerkstoffe denkbar. Zudem ist denkbar, dass der Papierwerkstoff gestrichen, das heißt beschichtet, oder ungestrichen und/ oder strukturiert ist, wie beispielsweise durch eine 3D-Kontur. Mittels einer Strukturierung und/oder Beschichtung kann insbesondere eine Griffigkeit des Körperpflegeprodukts verbessert werden. Insbesondere kann die Strukturierung sowohl im Papierwerkstoff selbst, wie beispielsweise durch eine Oberflächenbearbeitung und/oder eine Prägung, als auch in einer Bedruckung realisiert werden. Vorzugsweise erfolgt eine Herstellung des Papierwerkstoffs frei von einem Spritzguss- oder Extrusionsprozess, ausgenommen Faserguss oder Faserspritzguss, der im Vergleich zur Verarbeitung von Thermoplasten bei tieferen Temperaturen bzw. niedrigerem Druck durchgeführt wird. Die Temperatur liegt im Bereich von maximal 200°C und der Druck beträgt zwischen 10 bar und 40 bar, vorzugsweise zwischen 20 bar und 35 bar. Insbesondere ist bei der Herstellung des Papierwerkstoffs kein Schmelzprozess von Thermoplasten involviert.

Der Papierwerkstoff kann insbesondere auf verschiedene, einem Fachmann als sinnvoll erscheinende Arten bereitgestellt werden wie insbesondere in Form von Bögen, in Form von Nutzen, ab Rolle, in Block-Form oder als Granulat oder Grundstoff. Der Block kann dabei beispielsweise aus einem Kartonmaterial bestehen und beispielsweise zum mechanischen Bearbeiten wie z.B. Fräsen vorgesehen sein.

Der Papierwerkstoff kann abhängig von einer Anwendung verschiedene Dichten bzw. Stärken aufweisen. Mögliche Grammaturen sind beispielsweise ein Karton für eine Zahnbürste, einen Rasierer oder einen Pinsel. Mögliche Dichten bzw. Stärken pro Schicht können 1000 g/m² bis 2500 g/m², vorzugsweise 1200 g/m² bis 1700 g/m². Bei Verwendung mehrerer Schichten bewegt sich die Summer der Grammaturen im entsprechenden Bereich. Es können eine, zwei, drei, vier oder fünf Papierwerkstoffschichten verwendet werden. Die Schichten können unterschiedliche Grammaturen und Papierwerkstoffe aufweisen. Die Schichten können Kunststofffolien aufweisen. Vorzugsweise sind die Kunststofffolien ein- oder beidseitig von Schichten aus Papierwerkstoffen abgedeckt. Die Schichten können mittels Falten eines flächigen Papierwerkstoffs erzeugt werden. Es können zwei oder mehrere Faltungen erzeugt werden. Die Faltungen können sequentiell bzw. rollend sein oder die Seite jeweils alternierend wechseln (Handorgel).

Vorzugsweise kann der Papierwerkstoff von einem holzfreien gestrichenen Karton gebildet sein. Insgesamt kann mindestens eine Oberfläche des Papierwerkstoffs jeweils in Abhängigkeit von einer Anwendung gestrichen, bedruckt, geprägt oder dergleichen sein. Für den Papierwerkstoff ist insbesondere eine hohe Reißfestigkeit und zumindest anwendungsseitig eine hohe Wasserbarriere vorteilhaft. Die Reißfestigkeit beträgt zwischen 20 N und 100 N, vorzugsweise von 30 N bis 60 N.

Um die Wasserbarriere bzw. Wasserfestigkeit von Papierwerkstoffen zu prüfen, können diese einem Tauchtest unterzogen werden. Dazu wird der Papierwerkstoff zu einem vordefinierten Streifen geschnitten, beispielsweise mit einer Länge von rund 100 mm und einer Breite von rund 30 mm. Von diesem Streifen wird das Gewicht gemessen. Anschließend wird der Streifen in ein temperiertes Wasserbad getaucht. Das Wasserbad hat eine Temperatur im Bereich der Körpertemperatur des Menschen und liegt entsprechend zwischen 36°C und 38°C. Der Streifen wird 50 mm tief eingetaucht für eine vordefinierte Zeitspanne. Die Zeitspanne liegt zwischen 5 Sekunden und 180 Sekunden, 180 Sekunden steht für die empfohlene Putzzeit mit einer Zahnbürste. Anschließend wird der Streifen aus dem Wasserbad entfernt und das Wasser wird abgeklopft. Direkt nach dem Abklopfen wird der Streifen gewogen. Die Gewichtsdifferenz zeigt dabei, wieviel Wasser im Papierwerkstoff aufgenommen wurde. Die Gewichtsdifferenzen zwischen trockenen und nassen Papierwerkstoffen hängen von den verschiedensten Faktoren ab.

Der Aufbau des Papierwerkstoffs, dessen Beschichtung und Behandlung, Wassertemperatur, Form der Schnittflächen etc. tragen zu den Werten bei.

Die besten Papierwerkstoffe haben eine Wasseraufnahme bei 60s Einweichen in Wasser mit einer Wärme von 36° bis 38° von 5% bis 12%, vorzugsweise von 6,5 % bis 9,5%, direkt nach dem Einweichen.

Der Nicht-Papieranteil am Gesamtgewicht des Körperpflegeprodukts beträgt weniger als 10%, vorzugsweise weniger als 5%, am meisten bevorzugt weniger als 3%. Der Nicht-Papieranteil setzt sich beispielsweise zusammen aus Kunststofffolie und/oder Borsten und/oder Spritzgussteilen und/oder Metallteilen wie Draht oder Metallankern.

Ferner kann der Papierwerkstoff insbesondere zu einer Erreichung zusätzlicher Eigenschaften zusätzlich behandelt sein. Es kann beispielsweise eine Beschichtung auf einer Oberfläche des Papierwerkstoffs in Form eines Lacks, eines Klebstoffs, einer physikalischen und/oder chemischen Behandlung für die Wasserresistenz oder einer Veredelung vorgesehen sein.

Des Weiteren sind Füllstoffe für den Papierwerkstoff denkbar. Es sind insbesondere unlösliche Füllstoffe und lösliche Füllstoffe denkbar. Mögliche Füllstoffe sind für antibakterielle Additive, beispielsweise Silberpartikel , Polymere für eine verbesserte Wasserfestigkeit, wie beispielsweise bei der Papierbecherherstellung, abrasive Mittel (Erhöhen des Reibungskoeffizienten), beispielsweise zur Verbesserung der Reinigungsleistung, falls der Papierwerkstoff in der Reinigungseinheit eingesetzt wird, oder zur Verbesserung des Haltens bzw. der Haptik falls der Papierwerkstoff in der Griffeinheit eingesetzt wird, und/oder magnetische Partikel für ein magnetisches Papier, beispielsweise zur Halterung bzw. Lagerung bzw. Fixierung des Körperpflegeprodukts.

Weitere Füllstoffe, die sich im Gebrauch lösen, können folgende Wirksubstanzen sein, welche in einen entsprechenden Träger eingebunden sind und freigesetzt werden:
a) Wirkstoffe mit zahnpastaähnlicher Wirkung: Sorbitol, Aromen, Hydrated Silica, Natrium-Laurylsulfat, Natrium-Monofluorophosphat, Kreatin, Zinksulfate, Triclosan, Glycerin, Natrium-Saccharin, Propylenglycol, Dinatriumphosphat, Aluminiumoxid, Trinatriumphosphat, Natriumfluorid, Betain, Titandioxid, Carboxymethylcellulose, Tetranatrium-<Pyrophosphat etc.
b) Wirkstoffe mit antibakterieller Wirkung: Natriumbicarbonat, Citronensäure, Phosphorsäure, Natriumcarbonat, Kaliumcarbonat, Natriumperborat, NatriumHexametaphosphat, Natriumbenzoat, Natriumstearat etc.
c) Wirkstoffe zum Anzeigen des Putzerfolges mittels Einfärben des Plaques auf der Zahnoberfläche: Glucose, Maltodextrin, Magnesiumstearat, Aroma, Saccharin, Mikrokristalline Cellulose etc.
d) Wirkstoffe, die Geschmack abgeben
e) Wirkstoffe, die Geruch abgeben

Entsprechende Papierwerkstoffe sind beispielsweise bereits von Karton für Trinkbecher der Firma Storaenso bekannt, welcher lebensmitteltauglich, wasserdicht, kompostierbar und bedruckbar ist. Ferner ist von der Firma Kotkamills Karton bekannt, welcher mittels Dispersion wasserdicht gemacht wird und voll recycel- und kompostierbar bleibt. Ferner sind bereits Kartonröhren bekannt.

Der Papierwerkstoff kann vorbehandelt, z.B. veredelt sein. Unter einer "Veredelung" soll dabei eine Oberflächenbehandlung des Papierwerkstoffs verstanden werden, die beispielsweise dazu führt, dass der Papierwerkstoff einen hochwertigeren Eindruck erzielt. Eine Veredelung kann während oder nach dem Druckvorgang des Papierwerkstoffs aufgebracht werden. Eine Veredelung kann während oder nach dem Montagevorgang des Papierwerkstoffs mit anderen Elementen des Körperpflegeprodukts aufgebracht werden. Die Veredelung kann jeweils partiell an einer oder mehreren Stellen oder auch vollflächig aufgebracht/realisiert werden. Durch die Veredelung können beispielsweise metallische Effekte, optische Unterscheidungen oder auch Produktbeschriftungen realisiert werden. Durch die entsprechende Beschichtung kann auch eine Verminderung der Wasseraufnahme des Papierwerkstoffs erreicht werden. Die Beschichtung kann dabei beispielsweise nach einer Verarbeitung der Griffeinheit, wie beispielsweise einem Stanzen und/oder Formen, in einem Bad mit dem entsprechenden Beschichtungsmedium durch Tauchen aufgebracht werden. Weitere Möglichkeiten sind beispielsweise das Aufsprühen einer Beschichtung und/oder dass beispielsweise eine oberste Lage des materiellen Griffkörpers bereits außen über eine Beschichtung bzw. einen Lack verfügt. Die Beschichtung und/oder der Lack muss dabei insbesondere lebensmitteltauglich sein. Damit kann sich der Papierwerkstoff bzw. das daraus hergestellte Körperpflegeprodukt auch für einen Mehrfacheinsatz eignen. Veredelung kann beispielsweise eine Lackierung sein, dabei kann beispielsweise UV-Hochglanzlack, Strukturlack, Softtouch-Lack oder Glitzerlack verwendet werden. Die Veredelung kann auch eine mindestens partiell aufgebrachte Beflockung sein. Die Veredelung kann eine Prägung, beispielsweise eine Silberfolienprägung oder eine Hologramm-Prägung, sein. Die Veredelung kann ein Duftlack sein. Als eine weitere Möglichkeit der Vorbehandlung ist beispielsweise das Prägen des Papierwerkstoffs vorgesehen.

Vorzugsweise wird der materielle Griffkörper ab Bogen, Rolle oder Nutzen geschichtet. Die Schicht des materiellen Griffkörpers kann dabei insbesondere durch Zusammenführen mehrerer Bögen und/oder Rollen zu einem großen Bogen oder zu einer großen Schicht erfolgen. Anschließend kann die Schicht geformt werden. Die Schicht kann dabei aus der Ebene verformt werden, wobei insbesondere Verformungswinkel von zumindest 10°, vorzugsweise zumindest 20° und besonders bevorzugt zumindest 30° gegenüber einer Haupterstreckungsebene der Schicht möglich sind. Zu große Winkel führen insbesondere dazu, dass sich der Papierwerkstoff an den geschnittenen und/oder gestanzten Rändern auffächert und/oder dass einzelne Lagen an der Biegestelle reißen. Aus den gleichen Gründen werden bei der Verformung aus der Ebene bevorzugt an entstehenden Kanten auch Radien vorgesehen. Diese werden bevorzugt nicht zu klein gewählt. Aufwändige Versuche haben gezeigt, dass minimale Radien zwischen 0.1 mm-3.00 mm aufgeführte Probleme minimieren.

Größere Radien bis zu 20 mm, bevorzugt bis zu 10 mm können natürlich bei der Verformung auch verwendet werden. Sie werden v.a. eingesetzt, um ein rundes konkaves bzw. konvexes (Teil-) Profil der Griffeinheit in Erstreckungsrichtung zu bilden. Mit der Verformung aus der Ebene wird die Stabilität des Papierwerkstoffes in diesem Bereich erhöht und ein U, V, T, oder ein L Profil gebildet. Je größer die Radien bzw. je kleiner der Winkel gewählt wird umso stärker verschwindet der stabilisierende Effekt.

Der Papierwerkstoff kann auch mittels Pressen mindestens teilweise bzw. partiell mehr oder weniger verdichtet werden, das heißt, dass das Verformen der Schicht oder der Schichten gleichzeitig eine Komprimierung des Volumens bringen kann. Eine höhere Komprimierung bringt eine höhere Dichte, eine höhere Stabilität und auch weniger Risiko für Wassereintritt. Kombiniert mit einer Verformung kann eine Komprimierung die Stabilität wesentlich verbessern. Eine Komprimierung erfolgt mindestens um 20%, vorzugsweise mindestens um 35%, besonders bevorzugt bis zu 50% gegenüber der Ausgangsdicke. Die Verdichtung wird vorzugsweise an Stellen der Griffeinheit ausgeführt, welche eine andere bzw. zusätzliche Funktion ausführen. Beispielsweise können damit Zonen am Papierwerkstoff verstärkt werden, an welchen andere Elemente angebracht werden (z.B. Verbindungseinheit, Anwendungseinheit, Rasierklinge, Bürstenkopf, etc.). Es können auch Zonen an der Griffeinheit verstärkt werden, welche eine Funktion ausüben, z.B. einer Kratzkante, Schabkante, Schneidekante, Zahnstocherspitze, haptische Elemente z.B. für Fingerauflagen etc.

Ferner ist denkbar, dass die zumindest eine Schichte vor einer Verarbeitung vorbehandelt, bedruckt oder veredelt wird (siehe dazu die zusätzlichen Ausführungen in dieser Schrift). Die Vorbehandlung, Bedruckung und/oder Veredelung kann nur eine Lage betreffen, sie kann auch mit einer ganzen Schicht ein- oder beidseitig ausgeführt werden. Als Vorbehandlung ist beispielsweise ein Prägen denkbar, mit dem Ziel, eine Struktur auf der Oberfläche des Endprodukts zu erzeugen. Alternativ oder zusätzlich ist als Vorbehandlung ein Beschichten denkbar. Beispielsweise könnte eine Innenseite jeder Schicht zu einem Kleben vorgesehen sein, während eine Außenseite einen Schutz gegen Feuchtigkeit aufweist. Jede Schicht kann daher zwei Seiten mit unterschiedlichen Beschichtungen aufweisen. Alternativ oder zusätzlich ist als Vorbehandlung eine Bedruckung denkbar. Des Weiteren ist denkbar, dass die zumindest zwei Schichten nach einer Herstellung nachbehandelt werden. Als Nachbehandlung ist beispielsweise ein Beschichten, ein Bedrucken, ein Beschichten der Anwendungseinheit und/oder ein Laminieren denkbar. Vorzugsweise ist die Anwendungseinheit direkt mit dem materiellen Griffkörper verbunden. Die Anwendungseinheit wird insbesondere zwischen die Schichten des materiellen Griffkörpers eingelegt und zusammen mit den Schichten aus Papierwerkstoff verbunden, bzw. während des Verbindens der Schichten dazwischen verankert.

Vorzugsweise kann der Griffkörper mit einem Dekor und/oder einer Beschichtung versehen sein. Das Dekor und/oder die Beschichtung kann sich dabei über den kompletten Griffkörper erstrecken oder sich nur über Bereiche erstrecken. Dabei sind auch verschiedene Dekors oder Beschichtungen auf verschiedenen Teilen des Griffkörpers denkbar. Das Dekor und/oder die Beschichtung kann sowohl auf der Vorderseite als auch auf der Rückseite aufgebracht sein. Das Dekor und/oder die Beschichtung kann vollflächig oder nur partiell aufgebracht werden. So wäre auch lediglich eine partielle Beschichtung für Wasserfestigkeit in entsprechend exponierten Bereichen denkbar. So können beispielsweise lediglich Schnittkanten oder bei Zahnbürsten oder Rasierern kann lediglich der Bereich nahe der Verbindungseinheit bzw. der Anwendungseinheit partiell beschichtet werden. Das Dekor und/oder die Beschichtung kann die Eigenschaften des Griffkörpers aus Papierwerkstoff beeinflussen. Neben der primären Funktion der Beschichtung und/oder des Dekors wird auch der gesamte Griffkörper steifer. So kann weiches Papier durch die Behandlung stabiler und steifer ausgebildet werden. Sinnvollerweise wird für eine erhöhte Stabilität der Griffeinheit kombiniert mindestens partiell verformt, verdichtet und beschichtet.

Das Dekor kann ferner beispielsweise durch Kalt- / Heißprägen, Bedrucken, wie beispielsweise Inkjet, Laser oder Digitaldruck, durch Auflaminieren, part. Verbrennen/Verkohlen z.B. mittels heißem Stempel oder Laser oder durch eine Kombination von Drucken und Beschichten aufgebracht werden. Bei einer Kombination ist das Bedrucken beispielsweise nicht wasserfest, kratzfest oder dergleichen, was wiederum durch eine zusätzliche Beschichtung behoben werden kann.

Das Dekor und/oder die Beschichtung kann vor der Montage, insbesondere vor dem Stanzen und/oder vor einem Verformen, auf eine Lage oder eine Schicht aufgebracht werden. Dies hat insbesondere den Vorteil, dass eine Bedruckung und/oder Beschichtung ggf. bereits bei der Papierherstellung erfolgen kann. Zudem kann ein Auftrag im flachen Zustand vor einer Verformung erfolgen. Zu einer Vermeidung von Beschädigungen des Dekors oder der Beschichtung wäre denkbar, dass Stanzränder, Biegekanten, Faltkanten oder potentielle Rissstellen nicht mit dem Auftrag versehen, bzw. ausgespart sind.

Alternativ kann das Dekor und/oder die Beschichtung an den späteren Biegekanten, Faltkanten nach der Applikation und vor der Montage insbesondere vor dem Verformen mindestens teilweise gerillt werden. Dadurch sollte das Dekor und/oder die Beschichtung weniger reißen, wenn die effektive Verformung passiert.

Weiter kann das Dekor und/oder die Beschichtung an den späteren Biegekanten, Faltkanten nach der Applikation und vor der Montage insbesondere vor dem Verformen mindestens teilweise geschlitzt werden. Dadurch wird das Dekor und/oder die Beschichtung an der entsprechenden Stelle geschnitten und die Beschichtung reißt bei der Verformung weniger. Nachteil dabei ist, dass innere Lagen des Papierwerkstoffs freigelegt werden und dies sowohl optisch (andere Farbe sichtbar) als auch funktional (Unterbrechung der Beschichtung) Nachteile bringen kann. Dies könnte mit einer weiteren Beschichtung nach dem Verformen jedoch mindestens teilweise überbrückt werden.

Die Beschädigung des Dekors kann ebenfalls vermindert werden, wenn das Dekor auf einer flexiblen Schicht auf den Papierwerkstoff aufgebracht wird. Das heißt die flexible Schicht aus einer Kunststoffkomponente wie z.B. Polyethylen, Polyamid, oder ein nachhaltiger Kunststoff (beispielsweise in Form einer Folie) wird auf den Papierwerkstoff aufgebracht bzw. laminiert und dieser wird danach verformt. Durch die Flexibilität der Schicht wird die Beschädigung vermieden oder zumindest stark vermindert.

Alternativ wäre auch denkbar, dass ein Auftrag zumindest teilweise nach dem Verformen des Griffkörpers erfolgt, wobei ein Auftrag dann auf eine 3D-Geometrie erfolgen muss. Hierbei könnte jedoch ein Auftrag über mehrere Bauteile erfolgen, wobei wenn sich Teile gegenseitig verdecken, verdeckte Stellen nicht beschichtet sein können.

Vorzugsweise ist an dem Griffkörper eine Fläche für die Anbringung von Kennzeichnungen vorgesehen. Die Kennzeichnungen können beispielsweise in Form einer Bedruckung oder einer Heiß- /Kaltprägung, Laserbeschriftung eingebracht werden. Eine Prägung kann dabei beispielsweise direkt beim Formen der restlichen Struktur eingebracht werden. Es ist daher keine spezielle Prägefläche nötig. Eine Anordnung der Kennzeichnungen erfolgt insbesondere in einem Bereich mit einer überlaufenden Form ohne Kontursprünge und damit mit einer stetigen Geometrie. Vorzugsweise ist die Kennzeichnung beispielsweise in einer Vertiefung, insbesondere direkt in der Struktur des Griffkörpers integriert, oder auf einem Podest abgesetzt angeordnet.

In einer bevorzugten Ausführungsform wird der materielle Griffkörper insbesondere durch Zusammenführen mehrerer Papierlagen zu einer Schicht, anschließendes Aufbringen einer Veredelung wie z.B. Bedruckung/Lackierung, darauffolgendes Stanzen, Formen, Verdichten des Griffkörpers und anschließendes Aufbringen einer Schutzschicht wie z.B. eines Schutzlackes z.B. mittels Tauchen, Sprayendes Griffkörpers mit einem Beschichtungsmedium für eine Wasserbeständigkeit hergestellt. Die einzelnen Prozessschritte sind mindestens teilweise optional und können wie oben beschrieben auch in unterschiedlicher Reihenfolge vorgenommen werden.

Ferner kann eine Folienlaminierung, Folienkaschierung und/oder Heißfolienprägung genutzt werden. Es können dabei Papierwerkstoffe u.a. aus mehreren Lagen mit einer Folie hergestellt werden. Dabei können Mattfolien, Glanzfolien oder auch Strukturfolien genutzt werden. Dadurch werden die Oberflächen insbesondere langlebiger. Prägungen/Prägefoliendruck können/kann verwendet werden, dabei ist insbesondere ein Plan- oder Strukturprägen möglich. Auch eine Laminierung oder eine Einsiegelung kann zur Veredelung genutzt werden.

Dabei sind insbesondere verschiedene, einem Fachmann als sinnvoll erscheinende Ausgestaltungen des Körperpflegeprodukts denkbar. Der materielle Griffkörper kann insbesondere aus einem oder mehreren Bögen eines Papierwerkstoffs und/oder von Rollen abgerollt hergestellt werden und/oder als eine Rolle aus Streifen bzw. einem Band gerollt sein. Bei mehreren Schichten können die Schichten des materiellen Griffkörpers beispielsweise mittels Kleben, Siegeln, Schweißen und/oder Laminieren miteinander verbunden sein. Ferner ist denkbar, dass die zumindest eine Schicht vor einer Verarbeitung vorbehandelt wird. Als Vorbehandlung ist beispielsweise ein mindestens teilweises Prägen des Papierwerkstoffs denkbar, mit dem Ziel, eine Struktur auf der Oberfläche des Endprodukts und/oder einen Hohlraum im Griffkörper zu erzeugen. Alternativ oder zusätzlich ist als Vorbehandlung des Papierwerkstoffs ein Beschichten denkbar. Beispielsweise könnte eine Innenseite jeder Schicht des Papierwerkstoffs zu einem Kleben vorgesehen sein, während eine Außenseite einen Schutz gegen Feuchtigkeit aufweist. Jede Schicht kann daher zwei Seiten mit unterschiedlichen Behandlungen bzw. Beschichtungen aufweisen. Alternativ oder zusätzlich ist als Vorbehandlung eine Bedruckung bzw. Veredelung denkbar. Des Weiteren ist denkbar, dass die zumindest eine Schicht nach einer Herstellung nachbehandelt wird. Als Nachbehandlung ist beispielsweise ein Beschichten, ein Bedrucken und/oder ein Laminieren denkbar. Alternativ oder zusätzlich ist als Nachbehandlung ein Verformen und/oder Prägen und/oder Verdichten denkbar, wie insbesondere mittels Druck und Wärme, beispielsweise für eine Greifgeometrie.

Optional wäre denkbar, dass zumindest ein Teil der Oberfläche des Körperpflegeprodukts nachbehandelt wird. Mögliche Verfahren sind Bedrucken, Tauchen, Sprayen etc. Dabei wäre denkbar, dass das Körperpflegeprodukt für eine geringere Wasseraufnahme lackiert wird oder das Körperpflegeprodukt bedruckt wird. Eine vorteilhafte Wasserfestigkeit ist dabei insbesondere sinnvoll, da die Anwendungseinheit aus Papier besteht. Es können auch Körperpflegeprodukte aufgebracht werden. Selbstverständlich können auch andere in dieser Schrift beschriebene Verfahren zu Veredelungen, zum Einsatz von Füllstoffen oder Behandlungen des Papierwerkstoffs angewendet werden.

Die Nachbehandlung kann auch die Applikation von Körperpflegemitteln, insbesondere Zahnpflegemittel wie z.B. Zahnpasta auf die Anwendungseinheit beinhalten. Weiter kann durch die Nachbehandlung auch eine für die Reinigung optimierte Oberfläche der Anwendungseinheit geschaffen werden, beispielsweise durch die Applikation von Schleifmittel. Um Schleifmittel zu fixieren, werden mundhygienetaugliche Klebstoffe eingesetzt.

Die Nachbehandlung kann insbesondere durch Eintauchen einer entsprechenden Partie des Produkts in einem Fluid passieren.

Ferner wäre denkbar, dass der materielle Griffkörper der Griffeinheit zumindest zwei Schichten aus einem Papierwerkstoff aufweist, die geschichtet miteinander verbunden sind. Dazu können einzelne Schichten als separate Elemente vorbereitet sein und/oder mittels Biegen von Schichten erzeugt werden. Dadurch kann insbesondere vorteilhaft ein Griffkörper hergestellt werden. Es kann insbesondere ein vorteilhaft stabiler und leicht herzustellender Griffkörper bereitgestellt werden. Es kann insbesondere eine vorteilhafte Griffform bereitgestellt werden. Vorzugsweise besteht der materielle Griffkörper, insbesondere die gesamte Griffeinheit, aus zumindest einer Schicht. Alternativ sind bei zumindest zwei Schichten diese vorzugsweise mindestens teilweise übereinander angeordnet und miteinander verbunden. Vorzugsweise sind von 2 bis 6, vorzugsweise von 2 bis 4, Schichten vorgesehen. Es können aber auch 7 bis 15, vorzugsweise 8 bis 12 Schichten vorgesehen sein. Die Schichtdicke hängt von der Anzahl der Lagen ab, somit hängt die Anzahl Schichten auch davon ab, um die Stabilität optimal zu gestalten. Die Schichten können aus unterschiedlichen Papierwerkstoffen, unterschiedlichen Dichten bzw. Grammaturen von Papierwerkstoffen bestehen. Der/die Papierwerkstoffe können zusätzliche Schichten aus Folien, insbesondere Kunststofffolien, enthalten. Bevorzugt sind die zumindest zwei Schichten senkrecht zu einer Haupterstreckungsebene der Schichten mindestens teilweise übereinander angeordnet und miteinander verbunden. Die zumindest zwei Schichten grenzen insbesondere mit einer größten Fläche aneinander an. Die Schichten können beispielsweise mittels Kleben und/oder Siegeln und/oder Pressen und/oder Schweißen und/oder Laminieren miteinander verbunden werden. Dabei wird Druck und/oder Wärme und/oder Wasser(dampf) eingesetzt. Die Schichten werden insbesondere bei einer Herstellung des Körperpflegeprodukts miteinander verbunden. Während des Verbindens der Schichten kann die Anwendungseinheit zwischen den Schichten fixiert werden.

Die Verbindung, bzw. Siegelung von Schichten kann unter Anwendung von verschiedenen Parametern durchgeführt werden. Die bevorzugten Parameter beim Siegeln von Schichten sind wie nachfolgend aufgeführt. Die Temperatur bewegt sich im Bereich vom 150°C bis 210°C. Der Druck beträgt zwischen 4000 N/m² und 6000 N/m², vorzugsweise zwischen 4400 N/m² und 5100 N/m². Die Siegelungszeit beträgt zwischen 0,5 Sekunden und 5 Sekunden, vorzugsweise 1,5 Sekunden und 3,5 Sekunden.

Weiter kann beim Siegeln der Siegelstempel beispielsweise nicht eben ausgeführt sein. Dabei kann eine stetige oder nicht stetige Fläche und/oder eine Struktur des Stempels bereitgestellt werden. Mit einem derartigen Aufbau kann im Zusammenspiel mit der Gegenfläche eine Siegelstruktur geschaffen werden, die verschieden stark oder auch verschieden ausgeprägt gesiegelt ist. Es kann damit auch ein Druckverlauf über die Siegelfläche entstehen, der Druck variiert über verschiedene Bereiche des materiellen Griffkörpers der Griffeinheit.

Beim Siegeln mehrerer Schichten kann weiter eine Siegelreihenfolge festgelegt werden. Es werden beispielsweise nicht alle Schichten gleichzeitig miteinander versiegelt. Wenn beispielsweise drei Schichten versiegelt werden, können zuerst die ersten beiden Schichten gesiegelt werden und im Anschluss wird die dritte Schicht an die anderen beiden Schichten gesiegelt. Bei vier Schichten können beispielsweise die ersten beiden Schichten gesiegelt werden und im Anschluss entweder zweimal je eine Schicht oder die beiden weiteren Schichten im Anschluss auf einmal. Mit dem schrittweisen Siegeln kann eine optimale Siegelung der einzelnen Schichten erreicht werden. Beim schrittweisen Siegeln können zudem die Siegelparameter wie Temperatur, Druck und Zeit für jede Siegelung individuell angepasst werden.

Vorzugsweise wird der materielle Griffkörper ab Bogen, Rolle oder Nutzen geschichtet. Ferner ist denkbar, dass die zumindest zwei Schichten vor einer Verarbeitung vorbehandelt, geprägt, bedruckt oder veredelt werden (siehe dazu die zusätzlichen Ausführungen in dieser Schrift). Die Vorbehandlung, wie z.B. Prägung Bedruckung, Veredelung, kann auch nur eine Schicht betreffen. Als Vorbehandlung ist beispielsweise auch ein Verformen und/oder Prägen und/oder Verdichten denkbar, mit dem Ziel, eine Struktur und/oder eine Verstärkungssicke auf der Oberfläche des Endprodukts zu erzeugen. Ferner können wie beschrieben mittels Verformen und/oder Prägung und/oder Verdichten des Papierwerkstoffs auch Hohlräume zwischen den Schichten des Papierwerkstoffs/der Papierwerkstoffe gebildet werden. Dabei werden z.B. bei zwei Schichten an gleicher Position des Griffkörpers vor dem Verbinden der Papierwerkstoffschichten zwei entgegengesetzte Prägungen vorgesehen, um einen mindestens in Teilbereichen 3-dimensionalen statt flächigen Griffkörper zu erzeugen. Statt einem Hohlraum kann mit der beschriebenen Technik auch eine Zone bzw. eine Ausnehmung für eine später zu montierende Verbindungseinheit zwischen den vorgesehenen Schichten antizipiert werden.

Das Verformen, und/oder Prägen, und/oder Verdichten des Griffkörpers ist wie besprochen mit einer einzelnen Schicht oder auch nach dem Verbinden mehrerer Schichten möglich. Dadurch können ebenfalls Oberflächenstrukturen oder eine Sicke zur Verstärkung angebracht werden. Ein Hohlraum kann damit nicht mehr erzeugt werden. Es kann aber ebenfalls eine einseitige Zone, bzw. Vertiefung, bzw. Ausnehmung für eine Verbindungseinheit gebildet werden. Die Verformung entsteht dann jeweils einseitig konvex und auf der anderen Seite konkav.

Alternativ oder zusätzlich ist als Vorbehandlung ein Beschichten denkbar. Beispielsweise könnte eine Innenseite jeder Schicht des flächigen Papierwerkstoffs zu einem Kleben vorgesehen sein, während eine Außenseite einen Schutz gegen Feuchtigkeit aufweist. Jede Schicht kann daher zwei Seiten mit unterschiedlichen Beschichtungen bzw. Eigenschaften aufweisen. Alternativ oder zusätzlich ist als Vorbehandlung eine Bedruckung denkbar. Des Weiteren ist denkbar, dass die zumindest zwei Schichten des flächigen Papierwerkstoffs nach der Verbindung nachbehandelt werden. Als Nachbehandlung ist beispielsweise ein Beschichten, ein Lackieren, ein Bedrucken und/oder ein Laminieren denkbar. Vorzugsweise ist die Verbindungseinheit bzw. die Anwendungseinheit direkt mit dem materiellen Griffkörper verbunden. Die Verbindungseinheit bzw. Anwendungseinheit kann insbesondere zwischen die Schichten des materiellen Griffkörpers eingelegt und zusammen mit den Schichten aus Papierwerkstoff verbunden werden. Die Anwendungseinheit wird insbesondere quasi zwischen die zu verbindenden Schichten geklemmt. Beim Verbinden der beiden flächigen Papierwerkstoffschichten verdrängt der überlappende Teil der Verbindungseinheit bzw. Anwendungseinheit den Papierwerkstoff mindestens teilweise. Damit wird der Halt verstärkt. Der überlappende Teil der Verbindungseinheit bzw. Anwendungseinheit kann deformiert werden, um den Halt im Papierwerkstoff zu erhöhen. Wie oben beschrieben kann auch in diesem Teil eine Struktur und/oder eine Sicke mittels Prägung hergestellt werden, um den Halt der Anwendungseinheit zu erhöhen.

Alternativ wird die Verbindungseinheit bzw. die Anwendungseinheit einseitig auf die vorgesehene Schicht(en) aufgebracht. Dabei kommen z.B. Schweißen, Siegeln, Laminieren, Kleben, Nieten oder andere Verbindungstechniken in Frage.

Es ist insbesondere eine Option denkbar, bei welcher die Anzahl der Schichten von Papierwerkstoff variiert. Beispielsweise können im Bereich der Verankerung, wo eine bestimmte Stabilität erreicht werden muss, mehr Schichten vorhanden sein als am restlichen Griff - oder auch umgekehrt. Nicht jede Schicht muss zwingend über den ganzen Bereich der angrenzenden Schicht reichen. Eine reduzierte Schicht kann außen liegen oder zwischen größeren Schichten angeordnet sein. Durch das Anordnen zwischen den Schichten kann eine Beschädigung durch Wegreißen vermieden werden. Es bestehen insbesondere keine außenliegenden störenden Kanten, von welchen eine Verletzungsgefahr ausgehen kann.

Wie bereits beschrieben können die Schichten generell ab demselben Bogen bzw. Nutzen geformt werden, indem sie gefaltet und anschließend gesiegelt werden. Dadurch werden scharnierartige Faltkanten gebildet. Die gefaltete Schicht kann identisch aber auch kleiner als die nicht gefaltete Schicht des Bogens bzw. des Nutzens ausgebildet sein.

Mit einer Rille oder einer Perforation kann das Falten vereinfacht bzw. die Lage definiert werden. Vorzugsweise bleibt die Außenseite der Falte aber unverletzt. Vorzugsweise werden Rille oder Perforation auf der Innenseite des Falts angebracht. Gefaltete Schichten haben den Vorteil, dass weniger Schnittkanten des Papierwerkstoffes der Feuchtigkeit exponiert sind und dadurch die Wasseraufnahme an dieser Stelle reduziert wird.

Eine weitere Möglichkeit, die Schnittkanten des Papierwerkstoffs zu schützen, ist das Bördeln. Einzelne oder mehrere Schichten der Schnittkante können im Randbereich mindestens teilweise gebördelt werden. Dies verleiht dem Körperpflegeprodukt zusätzliche Stabilität und reduziert die Verletzungsgefahr, insbesondere beim Einsatz in der Mundhöhle. Eine weitere Möglichkeit besteht darin, die Schnittkanten nachträglich zu bearbeiten. Dabei werden die scharfen Kanten möglichst verrundet. Dies kann mittels abtragenden Verfahren (z.B. Schneiden, Schleifen etc.) und/oder mittels Verdichten gemacht werden.

Es wird weiter vorgeschlagen, dass die Verbindungseinheit zumindest eine Aufnahmenut aufweist, welche dazu vorgesehen ist, eine der Anwendungseinheit zugewandte Vorderkante des materiellen Griffkörpers der Griffeinheit aufzunehmen. Die Aufnahmenut ist insbesondere zu einer formschlüssigen bzw. klemmenden Aufnahme zumindest einer Kante des materiellen Griffkörpers vorgesehen. Vorzugsweise erstreckt sich die Aufnahmenut insbesondere zumindest im Wesentlichen senkrecht zu einer Längsachse der Griffeinheit. Bei Verwendung mehrerer Aufnahmenuten sind diese bevorzugt in einem Winkel zueinander angeordnet, wobei die Aufnahmenuten dadurch dennoch symmetrisch zur Längsachse angeordnet sind. Bevorzugt ist die Aufnahmenut insbesondere dazu vorgesehen, eine Vorderkante des materiellen Griffkörpers aufzunehmen. Durch eine mögliche Anordnung in einem Winkel wird parallel zur Aufnahme auch eine Ausrichtung der Griffeinheit gegenüber der Verbindungseinheit erreicht. Es ist denkbar, dass in der Aufnahmenut beispielsweise Widerhaken vorgesehen sind, welche eine Klemmung verbessern. Die Aufnahmenut ist insbesondere zu einer Bereitstellung einer Klemmverbindung mit der Griffeinheit, insbesondere mit dem materiellen Griffkörper vorgesehen. Bei einer Klemmverbindung wird insbesondere die Griffeinheit in einer Aufnahme, insbesondere der Aufnahmenut, der Verbindungseinheit eingeklemmt. Die Aufnahme ist dabei vorzugsweise aus Kunststoff hergestellt, sodass außen ein Kunststoff der Verbindungseinheit angeordnet ist, welche innen einen Papierwerkstoff der Griffeinheit aufnimmt. Dabei kann der Papierwerkstoff beispielsweise in ein U-förmiges Element der Verbindungseinheit, welches die Aufnahmenut begrenzt, eingeschoben werden. Die Klemmverbindung kann insbesondere vorteilhaft mit weiteren in dieser Schrift genannten Verbindungsmöglichkeiten kombiniert werden, damit ein Ausfahren aus der Aufnahme verhindert werden kann. Der Ausdruck "im Wesentlichen senkrecht" soll hier insbesondere eine Ausrichtung einer Richtung relativ zu einer Bezugsrichtung definieren, wobei die Richtung und die Bezugsrichtung, insbesondere in einer Projektionsebene betrachtet, einen Winkel von 90° einschließen und der Winkel eine maximale Abweichung von insbesondere kleiner als 8°, vorteilhaft kleiner als 5° und besonders vorteilhaft kleiner als 2° aufweist. Dadurch kann insbesondere eine besonders einfach herzustellende Verbindung bereitgestellt werden.

Ferner wird vorgeschlagen, dass der materielle Griffkörper der Griffeinheit zumindest eine Stabilisierungswand ausbildet, welche zu einer Haupterstreckungsebene der Griffeinheit abgewinkelt ist und sich zumindest im Wesentlichen parallel zu einer Längsachse der Griffeinheit erstreckt. Die Stabilisierungswand ist vorzugsweise an einer im Wesentlichen parallel zu der Längsachse verlaufenden Außenkante des materiellen Griffkörpers angeordnet. Die Stabilisierungswand ist senkrecht zu der Längsachse der Griffeinheit insbesondere in einem Winkel von zumindest 10°, vorzugsweise zumindest 20° und besonders bevorzugt zumindest 30° gegenüber einer Haupterstreckungsebene des Griffkörpers abgewinkelt. Vorzugsweise bildet die Stabilisierungswand eine freie Außenkante des materiellen Griffkörpers aus. Es wäre jedoch auch denkbar, dass die Stabilisierungswand mittig in dem materiellen Griffkörper ausgebildet ist. Ferner können weitere Stabilisierungsstrukturen in dem materiellen Griffkörper vorgesehen sein. Die Stabilisierungsstrukturen können vorzugsweise überwiegend in Längsrichtung geformt sein, insbesondere zur Verbesserung der Stabilität des Griffkörpers. Die Stabilisierungswand kann zudem zumindest eine Haltefläche ausbilden. Die Stabilisierungswand kann dafür mit Strukturen versehen werden. Die zumindest eine Haltefläche kann seitlich an dem Griffkörper gestaltet werden. Die Halteflächen sind insbesondere dazu vorgesehen, das Halten zu vereinfachen. Im Bereich der Stabilisierungswand sind die Flächen im Querschnitt insbesondere tiefer gezogen als der Querschnitt des Griffkörpers an anderer Stelle. Vorzugsweise weist der materielle Griffkörper im Bereich der Stabilisierungswand senkrecht zur Längsachse der Griffeinheit einen im Wesentlichen U-förmigen Querschnitt auf, vorzugsweise mit Öffnung gegen hinten oder vorne. Dadurch kann insbesondere eine vorteilhaft stabile Griffeinheit bereitgestellt werden.

Des Weiteren wird vorgeschlagen, dass die Verbindungseinheit zumindest eine Begrenzungswand aufweist, welche zu einer zumindest teilweisen Begrenzung und Stabilisierung der Stabilisierungswand des materiellen Griffkörpers vorgesehen ist. Die Begrenzungswand dient insbesondere dazu, die Stabilisierungswand der Griffeinheit in Position zu halten. Die Begrenzungswand erstreckt sich insbesondere zumindest im Wesentlichen parallel zu einer Längsachse der Griffeinheit. Die Begrenzungswand ist vorzugsweise an einer im Wesentlichen parallel zu der Längsachse verlaufenden Außenkante der Verbindungseinheit angeordnet. Die Halteflächen an der Griffeinheit können sich in Halteflächen an der Begrenzungswand der Verbindungseinheit fortsetzen. Die Begrenzungswand an der Verbindungseinheit kann als Stütze für die Stabilisierungswand der Griffeinheit dienen. Dabei ist beispielsweise denkbar, dass die Stabilisierungswand der Griffeinheit auf der Innenseite an der Begrenzungswand der Verbindungseinheit anliegt. Alternativ wäre denkbar, dass die Stabilisierungswand der Griffeinheit in eine Geometrie direkt an der Begrenzungswand der Verbindungseinheit eingefahren wird. Selbstverständlich kann auch mit Hilfsmitteln eine fixe Verbindung zwischen der Begrenzungswand der Verbindungseinheit und der Stabilisierungswand der Griffeinheit geschaffen werden, beispielsweise mittels Kleben, Siegeln, Schweißen Klemmen, Rasten, Nieten bzw. anderen in dieser Schrift genannten Verbindungsverfahren etc. Dadurch kann insbesondere eine vorteilhaft stabile Griffeinheit bereitgestellt werden. Ferner kann eine vorteilhaft hohe Ergonomie bereitgestellt werden. Es kann insbesondere ein zuverlässiges Greifen des Körperpflegeprodukts ermöglicht werden.

Die Verbindungseinheit kann vorzugweise über Haltepunkte, Auflagepunkte oder Auflageflächen verfügen, die den Benutzer des Produkts in der Anwendung unterstützen. So können beispielsweise bei Zahnbürsten oder Pinseln Finger- bzw. Daumenauflagen vorgesehen sein. Die Verbindungseinheit kann dazu entlang der Griffeinheit in die Länge gezogen werden, wobei der verlängerte Teil eine Auflage, insbesondere eine Daumenauflage, ausbildet. Alternativ können bei einem Rasierer die oben ausgeführten Halteflächen vorgesehen sein, welche seitlich auf der schmalen Seite der Griffeinheit zusätzliche Greifflächen bereitstellen

Ferner kann die Verbindungseinheit ein oder mehrere Stützelemente wie die Begrenzungswand enthalten, welche als Teil der Verbindungseinheit geformt sind. Der gebogene Griffkörper kann hierbei entsprechend an den Stützelementen, insbesondere einer entsprechenden Begrenzungswand, anliegen. Die Stützelemente stützen dabei und sorgen dafür, dass sich der Griff nicht aufbiegt. So kann die Struktur des Griffkörpers gehalten werden. Die Stützelemente, insbesondere die Begrenzungswand, können/kann dabei insbesondere mit Haltepunkten, Auflagepunkten oder Auflageflächen kombiniert werden, sodass beispielsweise das Stützelement innen als Stützelement dienen kann, während es außen zum Halten dient.

Die Begrenzungswand kann bezüglich der Auflagefläche des Griffkörpers am Verbindungselement in Richtung der Höhenachse mittig oder außermittig angeordnet sein. Das heißt die Begrenzungswand kann auf der Vorderseite wie auf der Rückseite über den Griffkörper hervorstehen.

Weiter kann die Begrenzungswand mit Formschlusselementen ausgestattet sein. Die Formschlusselemente können in Form von Rastelementen aufgebaut sein. Diese Formschlusselemente sind auf der Vorderseite der Verbindungseinheit angeordnet und in Form von vorstehenden Elementen vorzugsweise in Form von vorstehenden Kanten in Richtung der Breitenachse gestaltet. Die Elemente sind an beiden Begrenzungswänden angebracht und gegeneinander gerichtet bzw. in Richtung gegen die Griffeinheit gerichtet. Sie klemmen im montierten Zustand die Griffeinheit bzw. den Griffkörper gegenüber der Verbindungseinheit. Die Formschlusselemente drücken dabei den Griffkörper gegen die Auflagefläche des Grundkörpers der Anwendungseinheit an der Verbindungseinheit. Vorzugsweise sind die Rastelemente dazu vorgesehen, bei einem Befestigungsvorgang elastisch ausgelenkt zu werden, um anschließend durch eine innere Spannkraft hinter einer Kante der Griffeinheit einzurasten. Der Vorteil dieser Gestaltung der Verbindung zwischen der Griffeinheit und der Verbindungseinheit liegt darin, dass der Griff nicht durch eine zusätzliche Stanzung mit einem Durchgangsloch versehen werden muss. Die Stanzung erfolgt lediglich entlang der Außenkontur.

Des Weiteren sind auch Halteelemente in Kombinationen von Griffeinheit und Verbindungseinheit möglich. So kann beispielsweise ein Halteelement in der Kombination von Elementen der Griffeinheit und der Verbindungseinheit erreicht werden. Es wäre beispielsweise denkbar, dass das Formschlusselement der Verbindungseinheit pilzförmig ausgeführt ist und sich durch eine Durchgangsausnehmung in der Griffeinheit erstreckt, wobei das pilzförmige Formschlusselement derart geformt ist, dass das frei durch die Griffeinheit ragende Ende beispielsweise auch als Daumenauflage genutzt werden kann. Alternativ wäre denkbar, dass das Formschlusselement als Ring ausgebildet ist, der seitlich an einer Durchgangsausnehmung rastet, sodass die Durchgangsausnehmung teilweise geöffnet bleibt. Die Durchgangsausnehmung kann dann zusammen mit dem ringförmigen Formschlusselement als Daumenauflage genutzt werden.

Es wird ferner vorgeschlagen, dass der materielle Griffkörper der Griffeinheit entlang einer Längsrichtung der Griffeinheit im Wesentlichen S-förmig geschwungen ausgebildet ist. Vorzugsweise ist der materielle Griffkörper gegenüber einer ebenen Ausgestaltung verformt ausgebildet. Vorzugsweise weist der materielle Griffkörper in einem Längsschnitt und/oder in einer Längsansicht eine S-förmige Längsform auf. Das Profil der Griffeinheit ist insbesondere auf eine Anwendung sowie eine Ergonomie für den Benutzer optimiert. Der materielle Griffkörper weist in Längsrichtung insbesondere eine kontinuierliche, stetige, gerundete Form auf. Besonders bevorzugt weist der materielle Griffkörper in Längsrichtung eine S-förmig geschwungene Form auf. Dadurch kann insbesondere eine vorteilhaft ergonomische und stabile Griffeinheit bereitgestellt werden.

Ferner weist der materielle Griffkörper der Griffeinheit in einem Querschnitt in Querrichtung insbesondere eine vorteilhaft geformte Querform auf. Die Querform kann insbesondere generell Strukturen aufweisen, die die Stabilität positiv beeinflussen und sich in Längsrichtung kontinuierlich fortsetzen. Zudem wäre denkbar, dass Formen in Längsrichtung ineinander übergehen können. Der materielle Griffkörper ist insbesondere in einer Ebene parallel zu der Längsachse und senkrecht zur der Querachse bevorzugt symmetrisch ausgebildet. Bevorzugt ziehen sich die Formen der Querform von unten nach oben vorzugsweise bis in den Halsbereich zu der Verbindungseinheit. Beispielhafte Querschnittsformen können beispielsweise eine U-Form, eine T-Form, eine Ω-Form, eine W-Form, eine V-Form, eine L-Form oder auch eine Kombination mehrerer Formen sein. Die Ecken dieser Querformen bzw. die daraus entstehenden Längskanten sind wie weiter vorn beschrieben vorzugsweise verrundet. Der materielle Griffkörper kann beispielsweise eine wellenförmige Querschnittsform aufweisen. Ferner können mehrere Ringsegmente zu einer Querschnittsform kombiniert werden. In einer Draufsicht sind insbesondere beliebige Formen für den materiellen Griffkörper denkbar, die auf die Anwendung abgestimmt sind. So ist beispielsweise eine rechteckige, eine quadratische, eine ovale, eine runde und/oder eine dreieckige Form für den materiellen Griffkörper in einer Draufsicht denkbar. Mittels der Form des Griffkörpers kann insbesondere eine Stabilität verbessert werden, sodass damit die Kräfte bei einer Belastung der Anwendungseinheit entstehenden aufgenommen werden können. Die Griffeinheit kann insbesondere elastisch Kräfte bis zu einer Belastung der Anwendungseinheit von 1500g, vorzugsweise bis 2000 g, aufnehmen. Dabei wird die Anwendungseinheit fixiert und die Kraft am hinteren Ende der Griffeinheit gemessen - die Griffeinheit darf dabei nicht knicken und muss im Wesentlichen die Ursprungsform elastisch wieder einnehmen...

Vorzugsweise kann die Griffeinheit, insbesondere der materielle Griffkörper, eine Aufhängausnehmung aufweisen. Die Aufhängausnehmung ist insbesondere derart gestaltet, dass sie in der Anwendung das Greifen der Griffeinheit nicht stört und als Feature für das Aufhängen des Produkts genutzt werden kann. Die Aufhängausnehmung ist vorzugsweise so gestaltet, dass sie an dem Griffende liegt, welches der Anwendungseinheit abgewandt ist. Ferner wäre auch denkbar, dass die Griffeinheit, insbesondere der materielle Griffkörper, ein ausdrückbares Element zum Überstülpen aufweist; das Element kann dabei so gestaltet sein, dass es teilweise aus einer Grundform des Griffkörpers herausgedrückt werden kann. Hierfür wird insbesondere eine zu brechende oder bereits gebrochene, also bereits gestanzte, Struktur geschaffen. Hierbei kann insbesondere lediglich eine Linie eingestanzt werden oder eine Fläche, insbesondere eine sichelmondförmige Fläche, ausgestanzt werden, welche das Element teilweise freistellt. Die zu brechende Struktur kann insbesondere beispielsweise durch eine Perforation oder eine anderweitige Schwächung realisiert werden, sodass das Element manuell herausgedrückt werden kann. Mittels des Elements kann das Produkt beispielsweise an ein Zahnglas gehängt werden.

Vorzugsweise weist der materielle Griffkörper der Griffeinheit zumindest eine Schicht aus einem Papierwerkstoff auf, wobei die Schicht eine Prägung und/oder Verformung aufweist. Bei der Prägung wird der Papierwerkstoff mittels Druck mindestens teilweise verformt. Damit ist auf der Oberfläche des Papierwerkstoffs in Teilbereichen der Oberfläche eine dreidimensionale Gestaltung und/oder eine Oberflächenstruktur zu erreichen. Es ist ferner möglich, mittels Prägung und/oder Verdichtung Hohlräume bzw. Ausnehmungen innerhalb der Schichten von Papierwerkstoffen zu erzielen. Damit wird eine bessere Griffigkeit bzw. Rutschfestigkeit des Griffkörpers erreicht und damit die Handhabung des Mundhygieneprodukts erleichtert. Geprägt bzw. verdichtet werden können grundsätzlich alle Papierwerkstoffe, seien diese in einem u.a. flächigen, gerollten, laminierten, gesiegelten und/oder geschweißten Zustand. Eine Prägung bzw. eine Verdichtung kann mit einem Prägewerkzeug (Negativform) mittels Druck und/oder Wärme und/oder Wasser(dampf) etc. unterstützt werden. Eine Prägung bzw. Verdichtung kann im flächigen Zustand beim Ausstanzen des Papierwerkstoffs gemacht werden. Es ist aber auch möglich, dass eine Prägung an einem bereits gerollten Papierwerkstoff durchgeführt wird. Das Prägen bzw. Verdichten kann auch eine Art Tiefziehen sein, welches ebenfalls die oben erwähnten Gestaltungsmöglichkeiten mit sich bringt. Das Tiefziehen des Papierwerkstoffs kann mit einem Tiefziehwerkzeug ebenfalls mittels Druck und/oder Wärme und/oder Wasser(dampf) etc. unterstützt werden. Ein Prägen oder Tiefziehen kann dem Papierwerkstoff auch eine Form verleihen, welche eine Stabilität verleiht, beispielsweise eine Vertiefung in Längsrichtung des Griffteils einer Zahnbürste, welche den Griffkörper stabiler macht. Weiter können spezifisch gewählte Beschichtungen, wie Lacke etc., die Formstabilität und die Stabilität im Generellen beeinflussen und auch verbessern.

Ferner wäre denkbar, dass das Körperpflegeprodukt, insbesondere der materielle Griffkörper, zumindest eine Schicht aufweist, die zumindest teilweise verformt und/oder verpresst und/oder tiefgezogen ist. Die zumindest eine Schicht kann insbesondere sowohl aus einem oder mehreren Bögen bzw. Nutzen eines Papierwerkstoffs und/oder von Rollen abgerollt hergestellt werden. Das Körperpflegeprodukt weist insbesondere mehrere Schichten auf. Die Schichten, insbesondere des materiellen Griffkörpers, können beispielsweise mittels Kleben, Schweißen und/oder Laminieren miteinander verbunden sein. Ferner ist denkbar, dass die zumindest eine Schicht vor einer Verarbeitung vorbehandelt wird. Als Vorbehandlung ist beispielsweise ein Prägen denkbar, mit dem Ziel, eine Struktur auf der Oberfläche des Endprodukts zu erzeugen. Alternativ oder zusätzlich ist als Vorbehandlung ein Beschichten denkbar. Beispielsweise könnte eine Innenseite jeder Schicht zu einem Kleben vorgesehen sein, während eine Außenseite einen Schutz gegen Feuchtigkeit aufweist. Jede Schicht kann daher zwei Seiten mit unterschiedlichen Beschichtungen aufweisen. Alternativ oder zusätzlich ist als Vorbehandlung eine Bedruckung denkbar. Des Weiteren ist denkbar, dass die zumindest eine Schicht nach einer Herstellung nachbehandelt wird. Als Nachbehandlung ist beispielsweise ein Beschichten, ein Bedrucken und/oder ein Laminieren denkbar. Es ist jedoch auch eine alternative generelle Grundform für eine Griffeinheit entsprechend denkbar. Die zumindest eine Schicht des Körperpflegeprodukts wird insbesondere verformt, bevorzugt tiefgezogen, insbesondere mittels Drucks und Wärme. Hierdurch wird insbesondere beispielsweise eine Greifgeometrie für eine bessere Ergonomie erzeugt. Es kann insbesondere ein Körperpflegeprodukt mit einer voluminösen Griffeinheit bereitgestellt werden. Es wäre auch denkbar, dass die Griffeinheit aus zwei zusammengesetzten, tiefgezogenen Halbschalen besteht, die in einem miteinander verbundenen Zustand einen Hohlraum einschließen. Die Verbindung der Halbschalen kann beispielsweise mittels Kleben, Schweißen, Siegeln und/oder Stecken erfolgen. So kann insbesondere beispielsweise die Griffeinheit einer Zahnbürste hergestellt werden.

Ferner ist denkbar, dass an dem Körperpflegeprodukt Trennhilfen und oder Sollbruchstellen vorgesehen sind, an welchen das Körperpflegeprodukt zu einer Entsorgung auseinandergenommen, mit oder ohne einfache Hilfsmittel (wie z.B. Schere, Messer, Schraubenzieher etc.) aufgetrennt oder separiert werden kann, damit die verschiedenen Komponenten bzw. Materialien zwecks Entsorgung mindestens teilweise sortenrein getrennt werden können. Es können beispielsweise Greiflaschen, beispielsweise zwischen zu trennenden Schichten, Perforationen, entfernbare Fortsätze realisiert sein, über welche die Materialien bzw. Schichten getrennt werden können. Alternativ oder zusätzlich können nicht verbundene, nicht gesiegelte oder nicht geschweißte Abschnitte zu einer Trennung vorgesehen sein. Ferner können unterstützende Perforationen vorgesehen sein, beispielsweise zu einem Trennen von Bürste und Papier. Vorzugsweise sind insbesondere Trennhilfen an dem Körperpflegeprodukt vorgesehen, wie insbesondere Zuglaschen, Greiflaschen und/oder Perforationen.

Vorzugsweise können an der Verbindungseinheit bzw. an der Anwendungseinheit Mittel bzw. Teile oder Teilbereiche gestaltet werden, die ein Trennen des Verbindungselements, welches vorzugsweise aus Kunststoff besteht, von der Griffeinheit, welche vorzugsweise aus einem Papierwerkstoff besteht, ermöglichen. Hierdurch kann insbesondere eine getrennte Entsorgung ermöglicht werden. Die Verbindungseinheit kann wiederum mit der Anwendungseinheit untrennbar ausgeführt werden. Bei einem Rasierer muss dann entsprechend die Verbindungseinheit mit der Anwendungseinheit im Abfall entsorgt werden. Es wäre jedoch auch denkbar, dass eine Klinge von Kunststoffteilen trennbar ausgeführt ist, sodass die Kunststoffteile einer Kunststoffsammlung zugeführt werden können. Hierdurch kann zumindest ein partielles Recycling ermöglicht werden. Beispielsweise kann an der Verbindungseinheit eine Lasche, Kante, Vorsprung etc. vorgesehen sein, welche gut gegriffen werden kann, die normale Anwendung aber nicht stört. Vorzugsweise kann an der Lasche gezogen und die Griffeinheit kann gegengleich bewegt werden, um die Teile, insbesondere die Griffeinheit und die Verbindungseinheit, auseinanderzureißen.

Das Ablösen von Teilbereichen bzw. von Elementen des Körperpflegeprodukts, bzw. das mindestens teilweise Separieren von Materialien (wie z.B. der Anwendungseinheit vom Griffteil; der gespritzten Borsten bzw. des Borstenplättchens vom Kopfteil) ist nur einmalig vorgesehen (z.B. zur getrennten Entsorgung von Materialien). Ein Wiederverbinden der getrennten Elemente ist vorzugsweise nicht vorgesehen.

Ferner wäre denkbar, dass der materielle Griffkörper der Griffeinheit zumindest eine Schicht aus einem Papierwerkstoff aufweist, die zumindest teilweise gefaltet ausgebildet ist. Dadurch kann insbesondere vorteilhaft ein Griffkörper hergestellt werden. Es kann insbesondere ein vorteilhaft stabiler und leicht herzustellender Griffkörper bereitgestellt werden. Es kann insbesondere eine vorteilhafte Griffform bereitgestellt werden. Vorzugsweise ist die Schicht aus einem zusammenhängenden, insbesondere zugeschnittenen, Papier und/oder Karton hergestellt. Die Schicht umfasst vorteilhaft zumindest eine Faltachse und/oder zumindest eine Faltkante. Die Schicht ist in einem Gebrauchszustand vorzugsweise an der zumindest einen Faltachse und/oder der zumindest einen Faltkante gefaltet. Vorteilhaft wird durch die zumindest eine Faltachse und/oder Faltkante ein Falten der Schicht in einen Gebrauchszustand erleichtert oder das Produkt erhält durch die Faltung erst seine Form, die nötig ist, um den Gebrauch zu ermöglichen. Es wäre denkbar, dass die Schicht in der Faltkante und/oder Faltachse perforiert und/oder gerillt und/oder geprägt ausgebildet ist, um ein definiertes Falten zu ermöglichen.

Vorzugsweise ist die Schicht durch die zumindest eine Faltachse in zwei Seiten, insbesondere Teilschichten, unterteilt. Vorzugsweise verläuft die zumindest eine Faltachse und/oder Faltkante parallel zur Haupterstreckungsrichtung des Körperpflegeprodukts. Denkbar ist auch, dass zumindest eine Faltachse senkrecht zur Haupterstreckungsrichtung des Körperpflegeprodukts verläuft. Die Faltkante kann stetig oder nicht stetig ausgeführt sein, weiter kann eine Kombination von Perforationen, Rillungen, Schnitten die Faltkante bilden. Die Elemente können beispielsweise hintereinander angeordnet sein. Die Faltkante kann gerade oder gebogen ausgeführt sein. Dadurch können auch taillierte Formen gestaltet werden, wenn der Körper montiert ist. Der materielle Griffkörper kann dabei insbesondere aus Papierbögen oder ab Rolle gefaltet werden. Ferner kann eine Verbindung von Faltseiten insbesondere mittels Siegelung oder Klebung oder Montage (wie Stecken, Rasten) erfolgen. Als Vorbehandlung ist beispielsweise ein Prägen bzw. Verdichten denkbar, mit dem Ziel eine Struktur auf der Oberfläche des Endprodukts zu erzeugen. Alternativ oder zusätzlich ist als Vorbehandlung ein Beschichten denkbar. Beispielsweise könnte eine Innenseite einer Schicht zu einem Kleben vorgesehen sein, während eine Außenseite einen Schutz gegen Feuchtigkeit aufweist. Die Schicht kann daher zwei Seiten mit unterschiedlichen Vorbehandlungen, wie beispielsweise Beschichtungen, aufweisen. Alternativ oder zusätzlich ist als Vorbehandlung eine Bedruckung denkbar. Des Weiteren ist denkbar, dass die zumindest eine Schicht nach einer Herstellung nachbehandelt wird. Als Nachbehandlung ist beispielsweise ein Beschichten, ein Bedrucken und/oder ein Laminieren denkbar. Vorzugsweise wird die Schicht des materiellen Griffkörpers geschnitten oder gestanzt.

Als Körperpflegeprodukt mit einem entsprechenden materiellen Griffkörper wäre insbesondere eine Zahnbürste denkbar. Das Körperpflegeprodukt ist insbesondere flach hergestellt, wobei insbesondere Rillen, Prägungen, Perforation oder dergleichen zu einem Falten vor dem ersten Gebrauch vorgesehen sind. Die Rillen sind insbesondere in der Faltachse angeordnet. Zudem wäre eine Kombination aus Falten und einem anschließenden Verbinden, wie beispielsweise mittels Kleben, Siegeln, Schweißen, Leimen, Laminieren denkbar. Bei einem als Zahnbürste ausgebildeten Körperpflegeprodukt könnte das Körperpflegeprodukt mit Origami-Anlehnung ein Fachwerk aus Papier bilden. Alternativ oder zusätzlich könnte das Körperpflegeprodukt bei einer Ausbildung als Zahnbürste Reinigungselemente aus einem Papierwerkstoff umfassen. Die Reinigungselemente könnten auf einem Borstenplättchen aus Kunststoff, Holz oder einem Papierwerkstoff angeordnet sein, wobei das Borstenplättchen auf dem Papierwerkstoff des Griffkörpers angeordnet bzw. fixiert wird.

Das Körperpflegeprodukt ist insbesondere aus einem flächigen Papierwerkstoff hergestellt, wobei insbesondere Rillen zu einem Falten vor dem ersten Gebrauch vorgesehen sind. Die Rillen sind insbesondere in der Faltachse angeordnet. Das Körperpflegeprodukt kann im Gebrauch insbesondere zu einem Dreieck bzw. zu einem pyramidenförmigen oder kegelförmigen Gebilde gefaltet werden. Optional könnte bei einem gefalteten Körperpflegeprodukt zudem innen ein Fachwerk aus Papierwerkstoff vorgesehen sein, während außen eine Hülle das Fachwerk umgibt.

Als weiteres Körperpflegeprodukt mit einem entsprechenden materiellen Griffkörper wäre eine Zahnbürste denkbar (anlog stehen diese Möglichkeiten u.a. auch für andere Körperpflegeprodukte wie Nassrasierer zur Verfügung). Der Papierwerkstoff wird als Schicht mit entsprechender Rillung, Perforation oder dergleichen vorbereitet, damit ein entsprechendes Produkt anschließend gefaltet werden kann. Die Zahnbürste, die aus einem Papierwerkstoff in dieser Art hergestellt ist, kann mit verschiedenen Querschnittsprofilen erstellt sein. Beispielsweise kann das Produkt im Gebrauchszustand ein geschlossenes Profil z.B. ein dreieckiges, ein viereckiges oder generell ein n-eckiges Profil, ein offenes Profil z.B. ein U- Profil, V-Profil, ein W-Profil T- Profil, L-Profil oder ein daraus kombiniertes Profil ausbilden. Das Profil bildet schlussendlich einen Körper, der den Griffkörper der Zahnbürste bildet. Dabei kann das Profil auch dazu dienen, beispielsweise eine Standfläche auf der Rückseite der Zahnbürste zu bilden, damit die Zahnbürste auf eine Fläche abgelegt werden kann und dann nicht um die eigene Achse rollt. Die für die Faltungen vorbereiteten Rillungen, Perforationen oder dergleichen müssen nicht zwingend über den ganzen Körper geführt sein, sie können auch in einer Fläche enden, sodass sich beispielsweise im Anschluss eine Rundung ergibt. Die Montage des Produkts kann auf verschiedene Arten erfolgen. Beispielsweise kann das Produkt bereits fertig montiert sein oder der Endkunde (Benutzer) montiert das Produkt nach dem Kauf bzw. vor dem Gebrauch. Die zweite Variante kann verschiedene Vorteile in Bezug auf den in der Verpackung benötigten Platz bringen, da das Körperpflegeprodukt im flachen Zustand weniger voluminös ist als montiert. Beim Montieren werden verschiedene Stellen der Schicht miteinander verbunden, um den voluminösen Körper bzw. den Griffteil zu formen. Die Schichten können mit Klebestreifen, Kleber oder einem anderen für die Verbindung geeigneten Mittel direkt verbunden werden. Das Borstenfeld kann bereits am Griff montiert sein oder es kann ebenfalls erst mit der Montage des Griffs fixiert werden, beispielsweise durch Kleben. Vor der Montage kann es auch möglich sein, gewisse Stellen der Schicht aus Papierwerkstoff abzutrennen. Weiter ist es möglich, dass Elemente gestaltet sind, die es ermöglichen, dass der Körper des Produkts im gebrauchsfertigen Zustand einen geschlossenen Hohlraum bildet. So sind gewisse Laschen geformt, die so in Öffnungen gesteckt und darin fixiert werden, dass die Öffnungen hinterher verschlossen sind. Die Faltungen können in verschiedene Richtungen gestaltet sein, vorzugsweise findet der größte Teil der Faltungen um Faltachsen in Längsrichtung statt. Es können aber auch Faltungen in Querrichtungen vorbereitet sein, beispielsweise, wenn Elemente zum Verpacken ineinander gefaltet werden. Weiter können Elemente vorgesehen sein, die so zu falten sind, dass beispielsweise der Griffkörper auf der Seite der Anwendungseinheit (z.B. Borstenfeld) verschlossen wird.

Weiter ist es möglich, die Schichten aus Papierwerkstoff im flachen Zustand (vor der Faltung) mit Strukturen oder anderen Oberflächenbeschichtungen etc. zu versehen, um im Endprodukt Oberflächenelemente zu schaffen, die bestimmte Eigenschaften erfüllen, z.B. eine höhere Reibung aufweisen.

Der materielle Griffkörper kann mit einer Oberflächenstruktur versehen werden zu einem Verbessern der Haptik bzw. des Haltens. So können auf einer Oberfläche des Griffkörpers beispielsweise Noppen vorgesehen sein, welche mit dem Prägen des restlichen Körpers geformt werden. Die Oberflächenstruktur kann in den verschiedensten Bereichen des Griffkörpers integriert sein, wie beispielsweise auf den genannten Halteflächen oder auf den weiteren Oberflächen.

Die Prägung erfolgt wie bereits beschrieben mittels Druck und/oder Wärme. Die Papierschichten können dazu etwas befeuchtet werden, z.B. mit Wasser(dampf), damit die Umformung vereinfacht wird. Auf diese Weise kann durch die Behandlung mit Wärme, Feuchte und Druck das Produkt eine Form annehmen, welche sich in eine weitere Dimension erstreckt, z.B. Griffkörper mit einem Knick bzw. Winkel darin.

Alternativ oder zusätzlich kann der Verbindungsabschnitt der Anwendungseinheit als eine Schnittstelle für ein Wechselteil, wie beispielsweise einen Wechselkopf für eine manuelle Zahnbürste, ausgebildet sein.

Optional kann der materielle Griffkörper bereits aus einem bedruckten Papierwerkstoff hergestellt sein. Hierdurch könnte ein farbiges Endprodukt ohne Nachbearbeitung geschaffen werden.

Des Weiteren wird vorgeschlagen, dass der materielle Griffkörper der Griffeinheit zumindest eine Schicht aus einem Papierwerkstoff aufweist, die zumindest teilweise gefaltet ausgebildet ist, wobei der Griffkörper eine Verstaustellung, in welcher eine Innenseite der Schicht nach außen gerichtet ist, und eine Betriebsstellung aufweist, in welcher eine Außenseite der Schicht nach außen gerichtet ist. So kann die Griffeinheit insbesondere verpackt nach innen gefaltet sein und zum Gebrauch nach außen gefaltet werden. Es ist dabei insbesondere denkbar, dass der Griffkörper mit einer Verpackung kombiniert ist. Vorzugsweise bildet die Schicht in einem Darbietungszustand zumindest einen Teil einer Verpackung des Körperpflegeprodukts. Vorzugsweise ist das Verbringen des Griffkörpers von der Verstaustellung in die Betriebsstellung irreversibel, da möglicherweise gewisse Elemente abgetrennt werden müssen, um von der Verstaustellung zur Betriebsstellung zu gelangen.

Ferner wäre denkbar, dass der Grundkörper der Anwendungseinheit ein gespritztes Borstenfeld umfasst. Es sind insbesondere direkt angespritzte Borsten denkbar. Diese können insbesondere während eines Mehrkomponentenspritzgießens gemeinsam mit der Anwendungseinheit, der Griffeinheit und/oder der Kopplungseinheit gefertigt sein, oder sie können nachträglich an einen Grundkörper der Anwendungseinheit angespritzt sein. Im Gegensatz zu den konventionellen Borsten werden gespritzte Borsten nicht extrudiert, sondern mittels Spritzgussverfahren geformt. Es werden besondere Materialien dafür eingesetzt. Gespritzte Borsten können direkt am Papierwerkstoff oder Holz angespritzt werden, d.h. die Werkstoffe werden ins Spritzgusswerkzeug eingelegt und anschließend umspritzt.

Des Weiteren wäre denkbar, dass das Körperpflegeprodukt zumindest teilweise aus einem Papierwerkstoff gefräst ist. Das Körperpflegeprodukt wird dabei insbesondere zumindest teilweise aus Vollpappe hergestellt, welche in eine definierte Form gefräst ist. Dabei ist insbesondere denkbar, dass die zumindest eine Vollpappe nach einer Fräsung nachbehandelt wird. Die Vollpappe kann auch aus mehreren Schichten eines Papierwerkstoffs gebildet werden. Als Nachbehandlung ist beispielsweise ein Beschichten, ein Bedrucken und/oder ein Laminieren denkbar. Vorzugsweise besteht insbesondere zumindest die Griffeinheit aus Vollpappe. Es sind verschiedene, einem Fachmann als sinnvoll erscheinende Vollpappen denkbar, wie insbesondere Finnpappe. Als Körperpflegeprodukt wäre in diesem Zusammenhang insbesondere eine gestanzte Zahnbürste denkbar. Hierzu wird insbesondere ein Block aus einem Papierwerkstoff in eine Kontur gefräst, wobei im späteren Bürstenkopf Löcher gebohrt werden. Bei einer Herstellung werden insbesondere in einem ersten Schritt die Griffeinheit und Teile der Anwendungseinheit aus einem Block aus einem Papierwerkstoff gefräst und gebohrt. Anschließend kann die Griffeinheit und/oder die Anwendungseinheit optional bedruckt und lackiert werden.

Des Weiteren wäre denkbar, dass das Körperpflegeprodukt zumindest teilweise aus einem Papierwerkstoff gegossen ist. Der Papierwerkstoff wird dabei insbesondere in einem spritzgussähnlichen Prozess verarbeitet. Die Verarbeitung des Papierwerkstoffs wird jedoch bei deutlich tieferen Temperaturen und Spritzdrucken umgesetzt. Entsprechende Verfahren sind beispielsweise bei der PurePaperPak, einer konstruktiven Papierverpackung, bekannt. Dabei ist insbesondere ein ökologischer Rohstoffeinsatz möglich, wobei die Inhaltsstoffe ihren Ursprung in der Natur finden und insbesondere zu 100% aus natürlichen Ressourcen bestehen. Die Rohprodukte setzen sich insbesondere aus Industriestärke, vorzugsweise aus Kartoffeln, Papierfasern, Wasser, inklusive Zumischung, zusammen. Die dadurch entstehende Papiermischung wird anschließend für die Weiterverarbeitung mittels Spritzgusstechnik verwendet. Der Papierspritzguss erfolgt insbesondere in drei Schritten. In einem ersten Schritt erfolgt eine Einspritzung, bei der die Papiermixtur in ein Aluminium-Werkzeug gespritzt wird. In einem zweiten Schritt erfolgt ein Backprozess, bei dem die Papiermixtur im Werkzeug gebacken, insbesondere erhitzt, wird. In einem dritten Schritt erfolgt eine Entformung, bei der die fertige Verpackung entnommen werden kann. Ferner sind bei Verpackungen sogenannte PAPACKS^{®}-Verpackungen bekannt. Die Verpackungen bestehen insbesondere aus lebensmittelzertifiziertem PAPACKS^{®}-Faserguss. Entsprechende Produkte sind insbesondere kompostierbar und auch in den Altpapier- oder Biomüll-Kreislauf einbringbar. Dabei ist insbesondere denkbar, dass das gegossene Körperpflegeprodukt nach einem Guss nachbehandelt wird. Als Nachbehandlung ist beispielsweise ein Beschichten, ein Bedrucken und/oder ein Laminieren denkbar. Als Körperpflegeprodukt wäre in diesem Zusammenhang insbesondere eine gestanzte Zahnbürste denkbar. Ein Zahnbürstenkörper, insbesondere die Griffeinheit, wird dafür insbesondere aus einem Papierwerkstoff gegossen. Bei einer Herstellung wird insbesondere in einem ersten Schritt die Griffeinheit aus einem Papierwerkstoff gegossen. Anschließend kann die Griffeinheit und/oder die Anwendungseinheit optional bedruckt und lackiert werden.

Für Spritzguss mit einem Papierwerkstoff können als Materialien insbesondere Papier mit Stärke (Industriestärke) und einem Lösungsmittel eingesetzt werden. Die Komponenten werden gemischt und durch entsprechende Spritzgießmaschinen verarbeitet. Die Materialien werden in einem Verhältnis von 25% bis 70%, vorzugsweise 30% bis 40%, Papier, 20% bis 40%, vorzugsweise 25% bis 35%, Stärke und 5% bis 40%, vorzugsweise 10% bis 35%, Lösungsmittel gemischt. Als Stärke kann eine Industriestärke beispielsweise aus Mais oder Kartoffeln eingesetzt werden. Als Lösungsmittel dient insbesondere Polyvinyl-Alkohol. Für das Mischen der Komponenten werden diese in Wasser gelöst, gemischt und die entstehende Flüssigkeit/Masse wird getrocknet und anschließend zu einem Granulat verarbeitet.

Der Papierwerkstoff des Körperpflegeprodukts, insbesondere der Griffeinheit, kann auch mit einem Spritzgussteil mit zumindest einem funktionalen Teil auch separat gespritzt werden, um dann in einem nachgeordneten Prozess mit dem Papierwerkstoff verbunden zu werden. Dies bietet sich für bereits genannte Beispiele an (z.B. Bürstenkopf mit gespritzten Borsten oder Borstenplättchen mit konventionellen/extrudierten Filamenten als Zahnbürste, Interdentalreiniger, Flosser, etc.). Dabei sind an dem mindestens einen Spritzgussteil mit zumindest einem funktionalen Teil Mittel zur Verbindung mit dem Papierwerkstoff vorgesehen. Die Verbindung erfolgt dann mittels Leimen, Kleben, Siegeln, Nieten, Verformen etc.

Falls eine oder mehrere Schichten aus Papierwerkstoff verwendet werden, können diese natürlich, wie in dieser Schrift an anderen Stellen beschrieben, vor oder nach dem Anspritzen bzw. Verbinden mit diversen in dieser Schrift erwähnten Methoden (gefaltet, verformt, geprägt, gerillt, veredelt, beschichtet etc.) bearbeitet werden.

Alternativ zu einem Rollen von Papierwerkstoff wäre insbesondere auch ein Extrudieren von Papierwerkstoff zum Schaffen einer zylindrischen Struktur denkbar. Alternativ zu einem Fräsen von Papierwerkstoff wäre insbesondere auch das Drehen von Papierwerkstoff zu einem Schaffen von rotationssymmetrischen Geometrien denkbar.

Des Weiteren kann eine spezielle Oberfläche des Papierwerkstoffs geschaffen werden. Es können insbesondere auf verschiedenen Seiten unterschiedliche Oberflächen geschaffen werden, beispielsweise in Bezug auf Farbe oder Oberflächenbeschaffenheit.

Eine Verbindung mittels Schweißung muss gemäß der Offenbarung der Erfindung nicht über die ganze berührende Fläche erfolgen. So können beispielsweise Aufreißlaschen und/oder gewisse Randpositionen bzw. Randzonen nicht geschweißt werden. Ferner kann eine Schweißung beispielsweise auch nur am Rand erfolgen, während Innenflächen ausgelassen werden. Des Weiteren wäre auch denkbar, dass Schweißungen ausgelassen werden, beispielsweise für das Schaffen von Beweglichkeit. Alternativ wäre auch denkbar, dass bewusst zur Schaffung von Beweglichkeit geschweißt wird, insbesondere im Bereich einer Faltkante. Alternativ oder zusätzlich kann auch nur im Bereich der Schnittstelle zur Anwendungseinheit geschweißt werden, beispielsweise nur bei einem Verbindungsabschnitt des Grundkörpers.

Es wird ferner vorgeschlagen, dass der Papierwerkstoff eine wasserabweisende Beschichtung und/oder Imprägnierung aufweist. Durch die entsprechende Beschichtung und/oder Imprägnierung kann auch eine Verminderung der Wasseraufnahme des Papierwerkstoffs erreicht werden. Damit kann sich der Papierwerkstoff auch für einen Mehrfacheinsatz eignen. Eine Imprägnierung kann dabei beispielsweise mittels eines Kunststoffs und/oder eines Wachses oder dergleichen erfolgen.

Es wird weiter vorgeschlagen, dass der Papierwerkstoff lackiert und/oder bedruckt ausgebildet ist. Dadurch kann insbesondere eine vorteilhafte Individualisierung des Papierwerkstoffs erreicht werden. Auf einem Papierwerkstoff kann insbesondere eine vorteilhaft einfache Bedruckung ermöglicht werden. Es kann insbesondere eine Veredelung erreicht werden. Eine Veredelung kann beispielsweise eine Lackierung sein, dabei kann beispielsweise UV-Hochglanzlack, Strukturlack, Softtouch-Lack oder Glitzerlack verwendet werden. Alternativ oder zusätzlich kann der Papierwerkstoff eine Bedruckung aufweisen. Der Papierwerkstoff kann beispielsweise eine Bedruckung mit wasserbasierten Lacken aufweisen, welche gegenüber konventionellen Lacken ökologisch sind.

Der Schwerpunkt des gesamten Körperpflegeprodukts verschiebt sich gegenüber konventionell hergestellten Körperpflegeprodukten in Richtung der Anwendungseinheit. Die Griffeinheit, welche aus Papierwerkstoff hergestellt ist, hat ein geringeres Gewicht bei der erfindungsgemäßen Herstellung, während die Elemente im Bereich der Anwendungseinheit ihr Gewicht behalten.

Ein Schwerpunkt des gesamten Körperpflegeprodukts kommt insbesondere gemessen vom oberen Ende im Bereich von 25% bis 45%, vorzugsweise von 30% bis 40% der Gesamtlänge des Körperpflegeprodukts. Das Gesamtgewicht des Körperpflegeprodukts beträgt insbesondere von 0.5 g bis 15 g, vorzugsweise von 1 g bis 8 g. Bei einer Ausgestaltung des Körperpflegeprodukts als Zahnbürste beträgt ein Gewicht insbesondere von 4 g bis 15 g vorzugsweise von 5 g bis 8 g. Bei einer Ausgestaltung des Körperpflegeprodukts als Rasierer beträgt ein Gewicht der Griffeinheit insbesondere von 5 g bis 8 g, vorzugsweise von 3 g bis 12 g.

Ferner sind verschiedene, einem Fachmann als sinnvoll erscheinende Verpackungen für das Körperpflegeprodukt denkbar. Beispielsweise sind konventionelle Kunststoff-Blister oder Verpackungen aus Papierwerkstoff denkbar. Bei einer Verpackung aus einem Papierwerkstoff kann insbesondere der Einsatz von Kunststoff stark vermindert werden.

Ferner geht die Erfindung aus von einem Verfahren zur Herstellung des Körperpflegeprodukts.

Das erfindungsgemäße Körperpflegeprodukt soll hierbei nicht auf die oben beschriebenen Anwendungen und Ausführungsformen beschränkt sein. Insbesondere kann das erfindungsgemäße Körperpflegeprodukt zu einer Erfüllung einer hierin beschriebenen Funktionsweise eine von einer hierin genannten Anzahl von einzelnen Elementen, Bauteilen und Einheiten abweichende Anzahl und/oder eine beliebige sinnvolle Kombination derselben aufweisen. Zudem sollen bei den in dieser Offenbarung angegebenen Wertebereichen auch innerhalb der genannten Grenzen liegende Werte als offenbart und als beliebig einsetzbar gelten.

Selbstverständlich sind die in dieser Schrift gezeigten Ausgestaltungsvarianten beispielhaft. Im Rahmen der Erfindung können die einzelnen Ausprägungen und Elemente dieser Ausgestaltungsvarianten mit anderen Ausgestaltungsvarianten kombiniert werden, ohne den Rahmen dieser Erfindung zu verlassen.

### Zeichnungen

Weitere Vorteile ergeben sich aus der folgenden Zeichnungsbeschreibung. In den Zeichnungen sind sechs Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1A: eine Griffeinheit eines erfindungsgemäßen, als Zahnbürste ausgebildeten Körperpflegeprodukts in einer schematischen 3D-Ansicht von vorne,
- Fig. 1B: die Griffeinheit des erfindungsgemäßen Körperpflegeprodukts in einer schematischen 3D-Ansicht von hinten,
- Fig. 1C: die Griffeinheit des erfindungsgemäßen Körperpflegeprodukts in einer Draufsicht von vorne,
- Fig. 1D: die Griffeinheit des erfindungsgemäßen Körperpflegeprodukts in einer Draufsicht von hinten,
- Fig. 1E: die Griffeinheit des erfindungsgemäßen Körperpflegeprodukts in einer schematischen Schnittdarstellung entlang der Schnittlinie A-A,
- Fig. 1F: eine Anwendungseinheit und eine Verbindungseinheit des erfindungsgemäßen Körperpflegeprodukts in einer schematischen 3D-Ansicht von vorne,
- Fig. 1G: die Anwendungseinheit und die Verbindungseinheit des erfindungsgemäßen Körperpflegeprodukts in einer schematischen 3D-Ansicht von hinten,
- Fig. 1H: die Anwendungseinheit und die Verbindungseinheit des erfindungsgemäßen Körperpflegeprodukts in einer schematischen Schnittdarstellung entlang der Schnittlinie B-B,
- Fig. 1I: das erfindungsgemäße Körperpflegeprodukt mit der Griffeinheit, der Anwendungseinheit und der Verbindungseinheit in einer schematischen 3D-Ansicht von vorne,
- Fig. 1J: das erfindungsgemäße Körperpflegeprodukt mit der Griffeinheit, der Anwendungseinheit und der Verbindungseinheit in einer schematischen 3D-Ansicht von hinten,
- Fig. 1K: das erfindungsgemäße Körperpflegeprodukt mit der Griffeinheit, der Anwendungseinheit und der Verbindungseinheit in einer schematischen Schnittdarstellung entlang der Schnittlinie C-C,
- Fig. 2A: eine Griffeinheit eines alternativen erfindungsgemäßen als Zahnbürste, insbesondere Reisezahnbürste, ausgebildeten Körperpflegeprodukts in einer schematischen 3D-Ansicht von vorne,
- Fig. 2B: die Griffeinheit des alternativen erfindungsgemäßen Körperpflegeprodukts in einer schematischen 3D-Ansicht von hinten,
- Fig. 2C: die Griffeinheit des alternativen erfindungsgemäßen Körperpflegeprodukts in einer Draufsicht von vorne,
- Fig. 2D: die Griffeinheit des alternativen erfindungsgemäßen Körperpflegeprodukts in einer Draufsicht von hinten,
- Fig. 2E: die Griffeinheit des alternativen erfindungsgemäßen Körperpflegeprodukts in einer schematischen Schnittdarstellung entlang der Schnittlinie D-D,
- Fig. 2F: eine Anwendungseinheit und eine Verbindungseinheit des alternativen erfindungsgemäßen Körperpflegeprodukts in einer schematischen 3D-Ansicht von vorne,
- Fig. 2G: die Anwendungseinheit und die Verbindungseinheit des alternativen erfindungsgemäßen Körperpflegeprodukts in einer schematischen 3D-Ansicht von hinten,
- Fig. 2H: das alternative erfindungsgemäße Körperpflegeprodukt mit der Griffeinheit, der Anwendungseinheit und der Verbindungseinheit in einer schematischen 3D-Ansicht von vorne,
- Fig. 2I: das alternative erfindungsgemäße Körperpflegeprodukt mit der Griffeinheit, der Anwendungseinheit und der Verbindungseinheit in einer schematischen 3D-Ansicht von hinten,
- Fig. 2J: das alternative erfindungsgemäße Körperpflegeprodukt mit der Griffeinheit, der Anwendungseinheit und der Verbindungseinheit in einer schematischen Schnittdarstellung entlang der Schnittlinie E-E,
- Fig. 3A: ein weiteres alternatives erfindungsgemäßes Körperpflegeprodukt mit einer Griffeinheit in einer ersten alternativen Ausgestaltung, mit einer Anwendungseinheit und einer Verbindungseinheit in einer schematischen 3D-Ansicht von vorne,
- Fig. 3B: das weitere alternative erfindungsgemäße Körperpflegeprodukt mit der Griffeinheit in einer zweiten alternativen Ausgestaltung, mit einer Anwendungseinheit und der Verbindungseinheit in einer schematischen 3D-Ansicht von vorne,
- Fig. 3C: das weitere alternative erfindungsgemäße Körperpflegeprodukt mit der Griffeinheit in einer dritten alternativen Ausgestaltung, einer Anwendungseinheit und der Verbindungseinheit in einer schematischen 3D-Ansicht von vorne,
- Fig. 4A: eine Griffeinheit eines weiteren alternativen erfindungsgemäßen, als Rasierer ausgebildeten Körperpflegeprodukts, in einer schematischen 3D-Ansicht von vorne,
- Fig. 4B: die Griffeinheit des weiteren alternativen erfindungsgemäßen Körperpflegeprodukts in einer schematischen 3D-Ansicht von hinten,
- Fig. 4C: die Griffeinheit des weiteren alternativen erfindungsgemäßen Körperpflegeprodukts in einer Draufsicht von vorne,
- Fig. 4D: die Griffeinheit des weiteren alternativen erfindungsgemäßen Körperpflegeprodukts in einer Seitenansicht,
- Fig. 4E: die Griffeinheit des weiteren alternativen erfindungsgemäßen Körperpflegeprodukts in einer Draufsicht von hinten,
- Fig. 4F: eine Anwendungseinheit und eine Verbindungseinheit des weiteren alternativen erfindungsgemäßen Körperpflegeprodukts in einer schematischen 3D-Ansicht von vorne,
- Fig. 4G: die Anwendungseinheit und die Verbindungseinheit des weiteren alternativen erfindungsgemäßen Körperpflegeprodukts in einer schematischen 3D-Ansicht von hinten,
- Fig. 4H: die Anwendungseinheit und die Verbindungseinheit des weiteren alternativen erfindungsgemäßen Körperpflegeprodukts in einer Draufsicht von vorne,
- Fig. 4I: die Anwendungseinheit und die Verbindungseinheit des weiteren alternativen erfindungsgemäßen Körperpflegeprodukts in einer Draufsicht von der Seite,
- Fig. 4J: die Anwendungseinheit und die Verbindungseinheit des weiteren alternativen erfindungsgemäßen Körperpflegeprodukts in einer Draufsicht von hinten,
- Fig. 4K: das weitere alternative erfindungsgemäße Körperpflegeprodukt mit der Griffeinheit, der Anwendungseinheit und der Verbindungseinheit in einer schematischen 3D-Ansicht von vorne,
- Fig. 4L: das weitere alternative erfindungsgemäße Körperpflegeprodukt mit der Griffeinheit, der Anwendungseinheit und der Verbindungseinheit in einer schematischen 3D-Ansicht von hinten,
- Fig. 4M: das weitere alternative erfindungsgemäße Körperpflegeprodukt mit der Griffeinheit, der Anwendungseinheit und der Verbindungseinheit in einer Draufsicht von vorne,
- Fig. 4N: das weitere alternative erfindungsgemäße Körperpflegeprodukt mit der Griffeinheit, der Anwendungseinheit und der Verbindungseinheit in einer Seitenansicht,
- Fig. 4O: das weitere alternative erfindungsgemäße Körperpflegeprodukt mit der Griffeinheit, der Anwendungseinheit und der Verbindungseinheit in einer schematischen Schnittdarstellung entlang der Schnittlinie F-F,
- Fig. 5A: eine Griffeinheit eines weiteren alternativen erfindungsgemäßen, als Applikationspinsel ausgebildeten Körperpflegeprodukts in einer schematischen 3D-Ansicht von vorne,
- Fig. 5B: die Griffeinheit des weiteren alternativen erfindungsgemäßen Körperpflegeprodukts in einer schematischen 3D-Ansicht von hinten,
- Fig. 5C: eine Anwendungseinheit und eine Verbindungseinheit des weiteren alternativen erfindungsgemäßen Körperpflegeprodukts in einer schematischen 3D-Ansicht von vorne,
- Fig. 5D: die Anwendungseinheit und die Verbindungseinheit des weiteren alternativen erfindungsgemäßen Körperpflegeprodukts in einer schematischen 3D-Ansicht von hinten,
- Fig. 5E: die Anwendungseinheit und die Verbindungseinheit des weiteren alternativen erfindungsgemäßen Körperpflegeprodukts in einer schematischen Schnittdarstellung entlang der Schnittlinie G-G,
- Fig. 5F: das weitere alternative erfindungsgemäße Körperpflegeprodukt mit der Griffeinheit, der Anwendungseinheit und der Verbindungseinheit in einer schematischen 3D-Ansicht von vorne,
- Fig. 5G: das weitere alternative erfindungsgemäße Körperpflegeprodukt mit der Griffeinheit, der Anwendungseinheit und der Verbindungseinheit in einer schematischen 3D-Ansicht von hinten und
- Fig. 5H: das weitere alternative erfindungsgemäße Körperpflegeprodukt mit der Griffeinheit, der Anwendungseinheit und der Verbindungseinheit in einer schematischen Schnittdarstellung entlang der Schnittlinie H-H
- Fig. 6A: eine Griffeinheit eines weiteren alternativen erfindungsgemäßen, als Rasierer ausgebildeten Körperpflegeprodukts, in einer schematischen 3D-Ansicht von vorne,
- Fig. 6B: eine Anwendungseinheit und eine Verbindungseinheit des weiteren alternativen erfindungsgemäßen Körperpflegeprodukts in einer schematischen 3D-Ansicht von vorne und
- Fig. 6C: das weitere alternative erfindungsgemäße Körperpflegeprodukt mit der Griffeinheit, der Anwendungseinheit und der Verbindungseinheit in einer schematischen 3D-Ansicht von vorne.

### Beschreibung der Ausführungsbeispiele

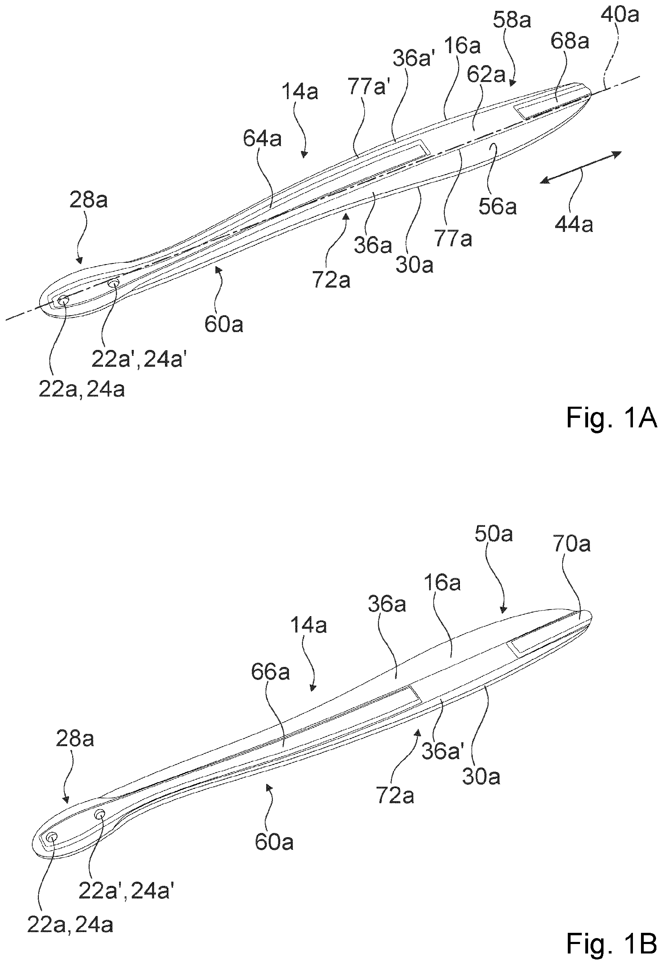

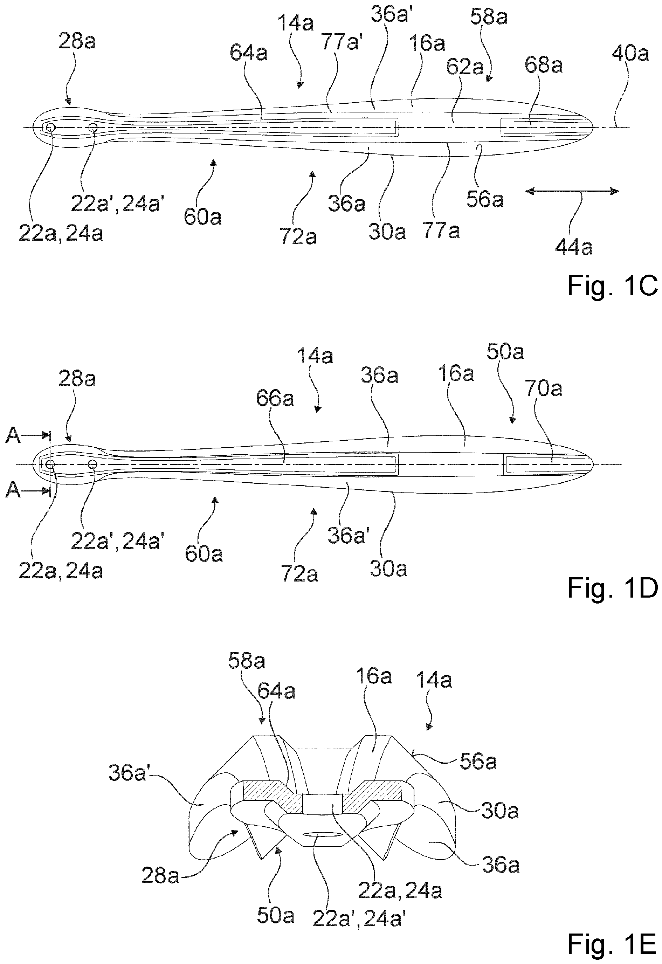

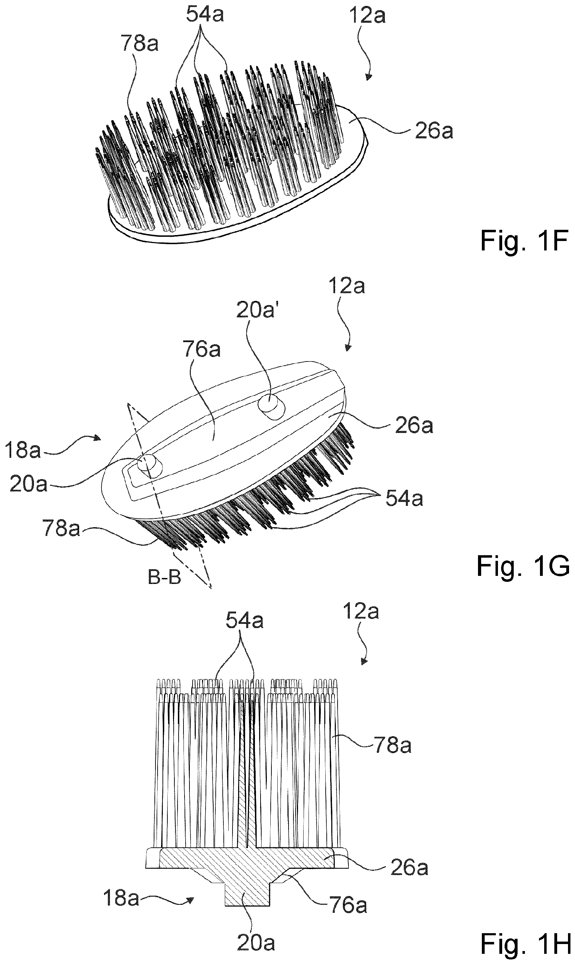

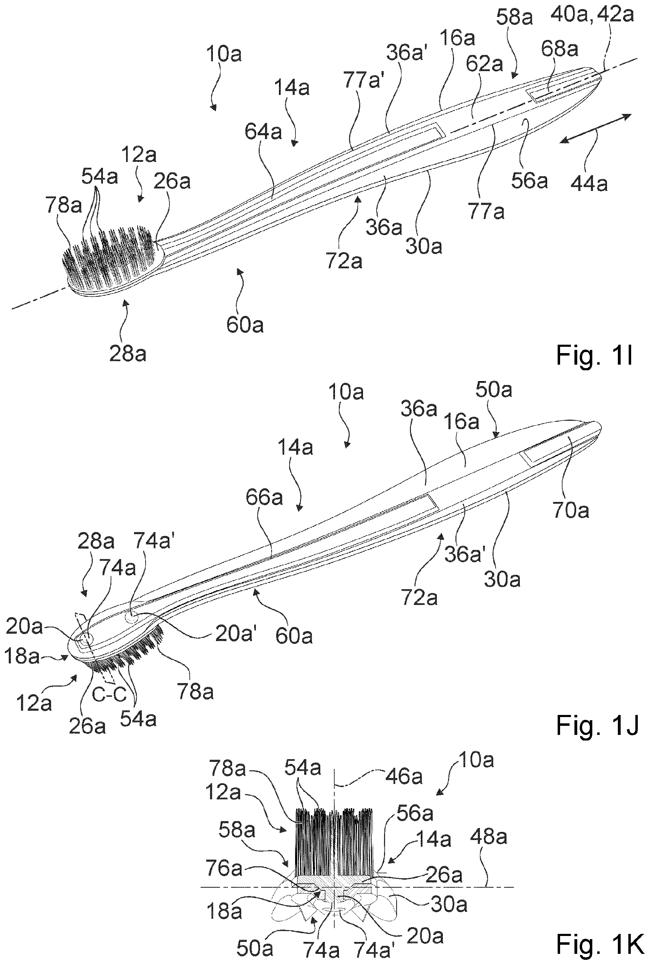

Im Folgenden wird auf die Figuren 1A bis 1K Bezug genommen, welche unterschiedliche Ansichten und Fertigungsstufen eines Körperpflegeprodukts 10a zeigen. Aufgrund der unterschiedlichen Ansichten sind einige Elemente nicht in sämtlichen Figuren dargestellt und entsprechend nicht in sämtlichen Figuren mit Bezugszeichen versehen.

Die Figur 1A zeigt eine Griffeinheit 14a des Körperpflegeprodukts 10a in einer schematischen perspektivischen Darstellung. Das Körperpflegeprodukt 10a ist von einem Mundhygienemittel gebildet. Das Körperpflegeprodukt 10a ist im vorliegenden Fall als eine Zahnbürste ausgebildet.

Das Körperpflegeprodukt 10a weist eine Anwendungseinheit 12a auf. Ferner weist das Körperpflegeprodukt 10a die Griffeinheit 14a auf, welche einen materiellen Griffkörper 16a aufweist. Des Weiteren weist das Körperpflegeprodukt 10a eine Verbindungseinheit 18a auf, welche die Anwendungseinheit 12a mit der Griffeinheit 14a verbindet.

Das Körperpflegeprodukt 10a weist eine Längsachse 42a, eine Höhenachse 46a und eine Breitenachse 48a auf. Die Längsachse 42a ist parallel zu einer

Haupterstreckungsrichtung 44a des Körperpflegeprodukts 10a angeordnet. Ist das Körperpflegeprodukt 10a mit einer Rückseite 50a auf eine ebene Oberfläche gelegt, sodass die Längsachse 42a parallel zu der Oberfläche angeordnet ist, ist die Höhenachse 46a senkrecht zu der Längsachse 42a und senkrecht zu der Oberfläche und der Breitenachse 48a angeordnet. Die Breitenachse 48a ist senkrecht zu der Längsachse 42a und senkrecht zu der Höhenachse 46a angeordnet. Im vorliegenden Fall weist das Körperpflegeprodukt 10a eine Länge, insbesondere parallel zu der Längsachse 42a und gemessen parallel zur Auflagefläche, von 140 mm bis 210 mm und vorzugsweise von 165 mm bis 185 mm auf. Ferner weist das Körperpflegeprodukt 10a eine maximale Höhe, insbesondere parallel zu der Höhenachse 46a gemessen senkrecht zur Auflagefläche, von 12 mm bis 25 mm und vorzugsweise von 15 mm bis 19 mm auf. Zudem weist das Körperpflegeprodukt 10a an einer breitesten Stelle eine Breite, insbesondere parallel zu der Breitenachse 48a, von 10 mm bis 25 mm und vorzugsweise von 13 mm bis 18 mm auf.

Die Anwendungseinheit 12a bildet einen Bürstenkopf des Körperpflegeprodukts 10a. Im vorliegenden Fall ist die Anwendungseinheit 12a als ein Zahnbürstenkopf ausgebildet. Die Anwendungseinheit 12a weist insbesondere einen als Borstenträger ausgebildeten Grundkörper 26a auf. Der Grundkörper 26a der Anwendungseinheit 12a ist vollständig aus einer Kunststoff-Komponente ausgebildet, hier aus Material für gespritzte Borsten. Es wäre jedoch auch denkbar, dass der Grundkörper 26a der Anwendungseinheit 12a aus einer Hartkomponente und einer Weichkomponente gebildet ist. Der Grundkörper 26a der Anwendungseinheit 12a bildet einen Borstenträger aus. Die Anwendungseinheit 12a weist ferner mehrere mit dem Grundkörper 26a zusammen ausgebildete Borsten 78a auf. Die Anwendungseinheit 12a ist im Spritzgussverfahren hergestellt und es bildet sich sowohl der Grundkörper 26a wie auch ein Borstenbündel 54a aus, welche aus Borsten 78a bestehen. Die Borstenbündel 54a können alternativ auch im Grundkörper 26a aufgenommen sein. Alternativ können auf einer Vorderseite 58a des Grundkörpers 26a Aussparungen ausgeformt sein. Die Aussparungen sind zur Aufnahme der Borstenbündel 54a vorgesehen, welche im konventionellen Ankerstanz-Verfahren fixiert werden. Der Grundkörper 26a weist in dem Fall eine Vielzahl von Aussparungen auf. Die Aussparungen sind von Borstenlöchern gebildet. Als Borsten für die Borstenbündel 54a kommen beliebige geeignete Borsten infrage, die, wie oben beschrieben, beispielsweise im Ankerstanz-Verfahren angestanzt sein können. Grundsätzlich wäre auch der Einsatz von AFT denkbar. Im Falle von AFT ist in dem Grundkörper 26a eine Ausnehmung vorgesehen, welche zur Aufnahme eines Borstenplättchens parallel zur Griffeinheit 14a vorgesehen ist. Das Plättchen ist insbesondere mittels eines Spritzgussverfahrens hergestellt, wobei das Plättchen bereits vor der Befestigung beborstet ist. Das Plättchen ist dazu vorgesehen, in der Ausnehmung des Grundkörpers 26a, insbesondere mittels Verschweißens, verankert zu werden oder auch mittels anderer Verbindungstechniken und/oder außerhalb einer Ausnehmung angeordnet zu werden. Die Borstenbündel 54a können sich hinsichtlich ihrer Länge, ihrer Zusammensetzung, ihrer Borstenanzahl, eines Borstenmaterials, einer Farbe, einer Oberflächenstrukturierung und dergleichen mehr unterscheiden.

Die Anwendungseinheit 12a ist an der Oberseite des Körperpflegeprodukts 10a angeordnet. Die Anwendungseinheit 12a bildet einen obersten Punkt des Körperpflegeprodukts 10a. Die Anwendungseinheit 12a ist vollständig aus einer Kunststoff-Komponente, hier einem Material für gespritzte Borsten ausgebildet. Es wäre jedoch auch denkbar, dass die Anwendungseinheit 12a teilweise aus einer Weichkomponente und teilweise aus einer Hartkomponente ausgebildet ist. Bezüglich geeigneter Weich- und/oder Hartkomponenten wird auf obenstehende Beschreibung verwiesen.

Die Griffeinheit 14a ist zu einem Greifen durch den Bediener vorgesehen. Die Griffeinheit 14a weist den materiellen Griffkörper 16a auf. Der materielle Griffkörper 16a der Griffeinheit 14a weist einen Kopfbereich 28a auf. Ferner weist der materielle Griffkörper 16a der Griffeinheit 14a einen Griffbereich 72a und einen zwischen dem Griffbereich 72a und dem Kopfbereich 28a angeordneten Halsbereich 60a auf. Der Kopfbereich 28a ist zu einer Anbindung der Anwendungseinheit 12a vorgesehen. Die Griffeinheit 14a weist zumindest eine Grifffläche 56a auf, welche eine Oberfläche des materiellen Griffkörpers 16a ausbildet. Die Grifffläche 56a verläuft auf der Vorderseite 58a von dem Halsbereich 60a des Griffkörpers 16a bis zum unteren Ende des Körperpflegeprodukts 10a an der Unterseite. Die Grifffläche 56a erstreckt sich über den Griffbereich 72a des materiellen Griffkörpers 16a. Die Grifffläche 56a bildet entlang der Längsachse 42a auf der Vorderseite 58a eine bombierte bzw. konvexe Form aus. Die bombierte Form ist entlang der Längsachse 42a auf der Rückseite 50a umgekehrt ausgebildet. Der Griffkörper 16a ist auf der Rückseite 50a in einem Bereich der Grifffläche 56a konkav geformt. Der Griffkörper 16a weist in einem Bereich der Grifffläche 56a eine ovale Grundform auf. Der Griffkörper 16a ist dreidimensional verformt, um die bombierte, konvexe Form auszubilden. Ferner weist der materielle Griffkörper 16a der Griffeinheit 14a zwei Stabilisierungswände 36a, 36a' auf, welche zu einer Haupterstreckungsebene der Griffeinheit 14a abgewinkelt sind und sich im Wesentlichen parallel zu einer Längsachse 40a der Griffeinheit 14a erstrecken. Die Stabilisierungswände 36a, 36a' sind jeweils über eine Faltkante 77a, 77a' gegenüber einem Rest des materiellen Griffkörpers 16a abgewinkelt. Zwischen den Stabilisierungswänden 36a, 36a' ist ein Mittenbereich des materiellen Griffkörpers 16a angeordnet. Die Stabilisierungswände 36a, 36a' erstrecken sich jeweils über den Griffbereich 72a und den Halsbereich 60a des materiellen Griffkörpers 16a. Die Stabilisierungswände 36a, 36a' sind an einer im Wesentlichen parallel zu der Längsachse 40a verlaufenden Außenkante des materiellen Griffkörpers 16a angeordnet. Die Stabilisierungswände 36a, 36a' sind senkrecht zu der Längsachse 40a der Griffeinheit 14a in einem Winkel von zumindest 10°, vorzugsweise zumindest 20°, gegenüber einer Haupterstreckungsebene des Griffkörpers 16a abgewinkelt. Die Stabilisierungswände 36a, 36a' bilden jeweils eine freie Außenkante des materiellen Griffkörpers 16a aus.

In einem mittleren oder hinteren Bereich der Grifffläche 56a könnte insbesondere ein Schriftzug und/oder ein Logo angeordnet sein. Der Griffkörper 16a bildet auf der Grifffläche 56a ein Plateau 62a aus. Das Plateau 62a dient zu einer Aufnahme eines Schriftzugs und/oder eines Logos. Das Plateau 62a ist zumindest teilweise mittig auf der Grifffläche 56a angeordnet. Das Plateau 62a ist in einem Bereich einer maximalen Breite des Körperpflegeprodukts 10a angeordnet.

Von einer Unterseite zu einer Oberseite ist eine maximale Breite des Körperpflegeprodukts 10a in den ersten 30 % der Haupterstreckung des Körperpflegeprodukts 10a angeordnet. Nach der maximalen Breite nimmt der Körper des Körperpflegeprodukts 10a nach hinten bis zum hinteren Ende in der Breite kontinuierlich ab. Nach der maximalen Breite nimmt der Körper des Körperpflegeprodukts 10a nach vorne zu der Anwendungseinheit 12a, der Breite der Griffeinheit 14a sowie des Halsbereichs 60a bis zum Kopfansatz des Kopfbereichs 28a in der Breite kontinuierlich ab. Die Griffeinheit 14a weist in der Höhe, insbesondere parallel zu der Höhenachse 46a, eine annähernd gleichmäßige Materialstärke auf.

Der Griffkörper 16a weist ferner eine im Wesentlichen konstante Materialstärke auf. Ferner weist der Griffkörper 16a eine erste Nut 64a auf, welche sich auf der Vorderseite 58a von der Anwendungseinheit 12a über den Halsbereich 60a über die Grifffläche 56a bis zu dem Plateau 62a erstreckt. Die erste Nut 64a verläuft mittig parallel zu der Längsachse 40a der Griffeinheit 14a. Die erste Nut 64a weist entlang einer Erstreckung parallel zu der Längsachse 40a einen zumindest annähernd konstanten Querschnitt auf, wobei sich die erste Nut 64a in dem Halsbereich 60a teilweise verjüngt. Die erste Nut 64a weist einen trapezförmigen Querschnitt auf, welcher sich zu der Vorderseite 58a hin aufweitet. Auf der Rückseite 50a des materiellen Griffkörpers 16a ist eine zu der ersten Nut 64a korrespondierende erste stegförmige Erhebung 66a ausgebildet. Die erste stegförmige Erhebung 66a entsteht dabei insbesondere durch die konstante Materialstärke bei einer Herstellung der ersten Nut 64a.

Des Weiteren weist der Griffkörper 16a eine zweite Nut 68a auf, welche sich auf der Vorderseite 58a von einem der Anwendungseinheit 12a angewandten unteren Ende der Griffeinheit 14a über die Grifffläche 56a bis zu dem Plateau 62a erstreckt. Die zweite Nut 68a verläuft mittig parallel zu der Längsachse 40a der Griffeinheit 14a. Die zweite Nut 68a weist entlang einer Erstreckung parallel zu der Längsachse 40a einen zumindest annähernd konstanten Querschnitt auf. Die zweite Nut 68a weist einen trapezförmigen Querschnitt auf, welcher sich zu der Vorderseite 58a hin aufweitet. Die zweite Nut 68a verläuft im Wesentlichen fluchtend zu der ersten Nut 64a, wobei die erste Nut 64a und die zweite Nut 68a durch das Plateau 62a unterbrochen sind. Auf der Rückseite 50a des materiellen Griffkörpers 16a ist eine zu der zweiten Nut 68a korrespondierende zweite stegförmige Erhebung 70a ausgebildet. Die zweite stegförmige Erhebung 70a entsteht dabei insbesondere durch die konstante Materialstärke bei einer Herstellung der zweiten Nut 68a.

Der materielle Griffkörper 16a besteht zumindest zu einem Großteil aus einem Papierwerkstoff. Der materielle Griffkörper 16a besteht vollständig aus einem Papierwerkstoff. Der materielle Griffkörper 16a der Griffeinheit 14a weist zumindest eine Schicht 30a aus einem Papierwerkstoff auf, welche dreidimensional geformt ausgebildet ist. Der materielle Griffkörper 16a besteht aus genau einer Schicht 30a aus einem Papierwerkstoff, es wäre jedoch auch ein mehrschichtiger Aufbau denkbar.

Der materielle Griffkörper 16a der Griffeinheit 14a ist entlang einer Längsrichtung der Griffeinheit 14a im Wesentlichen S-förmig geschwungen ausgebildet. Der materielle Griffkörper 16a weist in einem Längsschnitt und/oder in einer Längsansicht eine S-förmige Längsform auf. Das Profil der Griffeinheit 14a ist auf eine Anwendung sowie eine Ergonomie für den Benutzer optimiert. Der materielle Griffkörper 16a weist in Längsrichtung eine kontinuierliche, stetige, gerundete Form auf.

Ferner weist das Körperpflegeprodukt 10a die Verbindungseinheit 18a auf. Die Verbindungseinheit 18a ist zu einer formschlüssigen Verbindung mit dem materiellen Griffkörper 16a der Griffeinheit 14a vorgesehen. Zusätzlich zu der formschlüssigen Verbindung kann zudem eine kraft- und/oder stoffschlüssige Verbindung vorgesehen sein. Es ist insbesondere denkbar, dass die Verbindungseinheit 18a zusätzlich zumindest teilweise mit dem materiellen Griffkörper 16a der Griffeinheit 14a verklebt, verschweißt oder vergossen ist. Ferner wäre denkbar, dass eine Verbindung mittels Anspritzen und/oder Umspritzen oder mittels Klemmen hergestellt ist.

Die Verbindungseinheit 18a weist zumindest ein Formschlusselement 20a, 20a' auf. Die Verbindungseinheit 18a weist beispielhaft zwei Formschlusselemente 20a, 20a' auf. Die Formschlusselemente 20a, 20a' sind dazu vorgesehen, jeweils in eine Ausnehmung 22a, 22a' der Anwendungseinheit 12a und/oder der Griffeinheit 14a einzugreifen. Die Formschlusselemente 20a, 20a' sind dazu vorgesehen, jeweils in eine Ausnehmung 22a, 22a' der Griffeinheit 14a einzugreifen. Die Ausnehmungen 22a, 22a' der Griffeinheit 14a sind von Durchgangsausnehmungen 24a, 24a' gebildet. Der materielle Griffkörper 16a begrenzt die zwei Durchgangsausnehmungen 24a, 24a'. Die Durchgangsausnehmungen 24a, 24a' sind in dem Kopfbereich 28a des materiellen Griffkörpers 16a eingebracht. Die Durchgangsausnehmungen 24a, 24a' sind entlang der Längsachse 40a des Griffeinheit 14a hintereinander angeordnet. Die Durchgangsausnehmungen 24a, 24a' sind in der ersten Nut 64a angeordnet. Die Durchgangsausnehmungen 24a, 24a' sind dazu vorgesehen, die Formschlusselemente 20a, 20a' der Verbindungseinheit 18a aufzunehmen. Die Durchgangsausnehmungen 24a, 24a' sind jeweils von einer kreiszylindrischen Ausnehmung 22a, 22a' gebildet. Die Formschlusselemente 20a, 20a' weisen ebenfalls eine zylindrische Grundform auf. Die Formschlusselemente 20a, 20a' sind jeweils von einem Niet gebildet. Die Formschlusselemente 20a, 20a' sind jeweils von einem Kunststoffniet gebildet. Die Formschlusselemente 20a, 20a' weisen in einem mit der Griffeinheit 14a verbundenen Zustand jeweils einen Schaft sowie einen Formschlusskopf 74a, 74a' auf. Der Formschlusskopf 74a, 74a' der Formschlusselemente 20a, 20a' weist gegenüber den Durchgangsausnehmungen 24a, 24a' einen vergrößerten Durchmesser auf und ist auf einer der Anwendungseinheit 12a abgewandten Seite der Griffeinheit 14a angeordnet. Die Formschlussköpfe 74a, 74a' der Formschlusselemente 20a, 20a' verhindern ein Zurückziehen der Verbindungseinheit 18a und somit ein Entfernen der Verbindungseinheit 18a von der Griffeinheit 14a.

Bei einer Herstellung wird die Griffeinheit 14a sowie die Verbindungseinheit 18a bereitgestellt. Anschließend werden die Griffeinheit 14a und die Verbindungseinheit 18a zusammengeführt, wobei sich die Formschlusselemente 20a, 20a' durch die Durchgangsausnehmungen 24a, 24a' erstrecken. Darauffolgend werden die hindurchstehenden Schäfte der Formschlusselemente 20a, 20a' verformt. Dabei sind verschiedene, einem Fachmann als sinnvoll erscheinende Verformungsmöglichkeiten denkbar, wie beispielsweise durch Druck, wobei die Formschlusselemente 20a, 20a' verdrückt bzw. angestaucht werden, oder durch Druck und Wärme, wobei die Formschlusselemente 20a, 20a' mit einem geheizten Stempel verformt bzw. angestaucht werden. Der Druck ist dabei insbesondere so gewählt, dass ein kontrolliertes Stauchen erfolgt. Bei einem Aufbringen des Drucks wird insbesondere eine Ausbreitung der gestauchten Formschlusselemente 20a, 20a' gesteuert. Vorzugsweise ist ein Stempel dafür insbesondere nicht flach, sondern konkav gewölbt. Eine runde Form der Berührungsfläche steuert insbesondere das Fließen des Materials während der Verformung der Formschlusselemente 20a, 20a'.

Der materielle Griffkörper 16a der Griffeinheit 14a weist in dem Kopfbereich 28a ferner eine Vertiefung auf, welche zu einer Positionierung der Verbindungseinheit 18a relativ zu der Griffeinheit 14a vorgesehen ist. Die Vertiefung ist von einem Teilbereich der ersten Nut 64a gebildet. Die Vertiefung ist von einer länglichen Vertiefung in dem Kopfbereich 28a gebildet, die sich parallel zu der Längsachse 40a der Griffeinheit 14a erstreckt. Die Verbindungseinheit 18a weist einen zu der Vertiefung bzw. der ersten Nut 64a korrespondierenden Steg 76a auf, welcher sich parallel zu der Längsachse 42a des Körperpflegeprodukts 10a über einen Großteil einer Erstreckung der Verbindungseinheit 18a erstreckt. Der Steg 76a weist einen trapezförmigen Querschnitt auf. Ferner ist der Steg 76a in einem Mittenbereich verbreitert. Eine Form des Stegs 76a ist an eine Form der ersten Nut 64a in dem Kopfbereich 28a angepasst. Die Formschlusselemente 20a, 20a' sind auf dem Steg 76a angeordnet. Die Vertiefung sowie der Steg 76a sind zu einer Vorpositionierung der Verbindungseinheit 18a an der Griffeinheit 14a vorgesehen und dienen zudem zu einer teilweisen Aufnahme von Querkräften. Die Verbindung der Vertiefung mit dem Steg 76a bildet eine formschlüssige Verbindung der Verbindungseinheit 18a aus. Die Formschlusselemente 20a, 20a' bilden eine weitere formschlüssige Verbindung der Verbindungseinheit 18a mit der Griffeinheit 14a aus.

Die Anwendungseinheit 12a weist den Grundkörper 26a auf. Der Grundkörper 26a ist fest mit der Verbindungseinheit 18a verbunden. Der Grundkörper 26a ist einstückig mit der Verbindungseinheit 18a verbunden. Der Grundkörper 26a ist einteilig mit der Verbindungseinheit 18a ausgebildet. Die Verbindungseinheit 18a ist in den Grundkörper 26a integriert. Die Formschlusselemente 20a, 20a' sind einstückig mit dem Grundkörper 26a der Anwendungseinheit 12a ausgebildet. Die Formschlusselemente 20a, 20a' sind einteilig mit dem Grundkörper 26a der Anwendungseinheit 12a ausgebildet. Der Steg 76a ist ebenfalls einteilig mit dem Grundkörper 26a der Anwendungseinheit 12a ausgebildet. Am Grundkörper 26a bzw. mit dem Grundkörper 26a zusammen sind auch die Borstenbündel 54a ausgeformt.

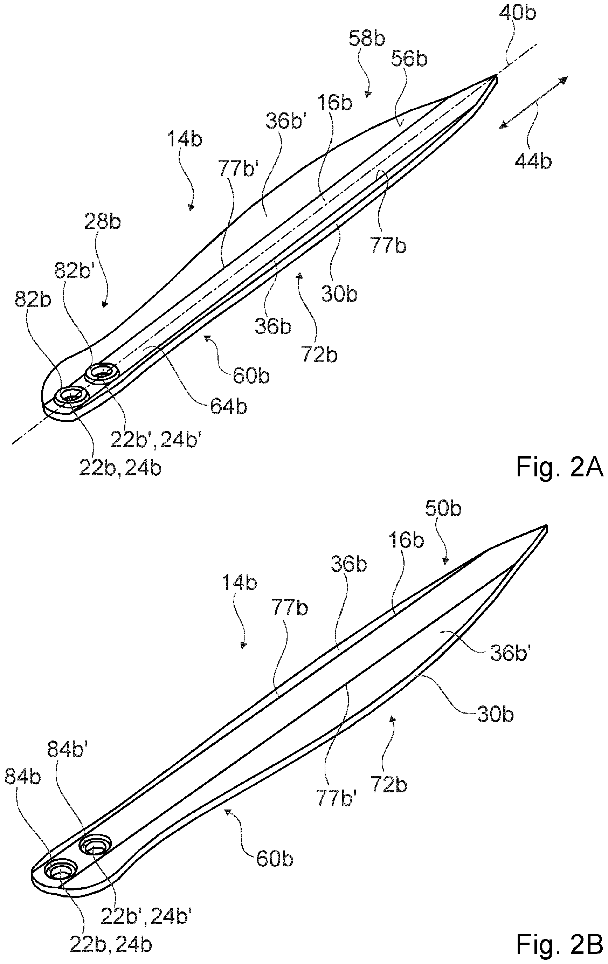

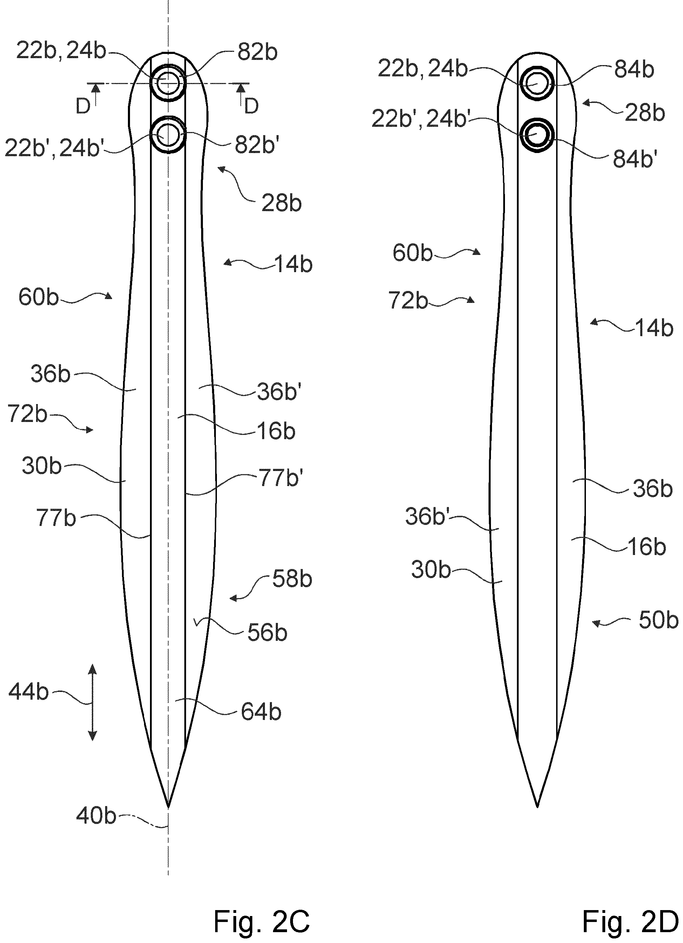

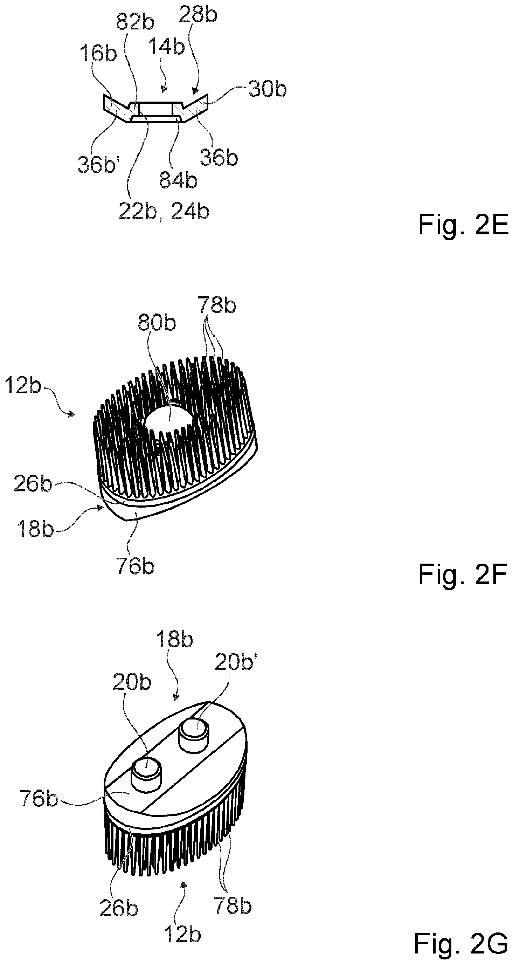

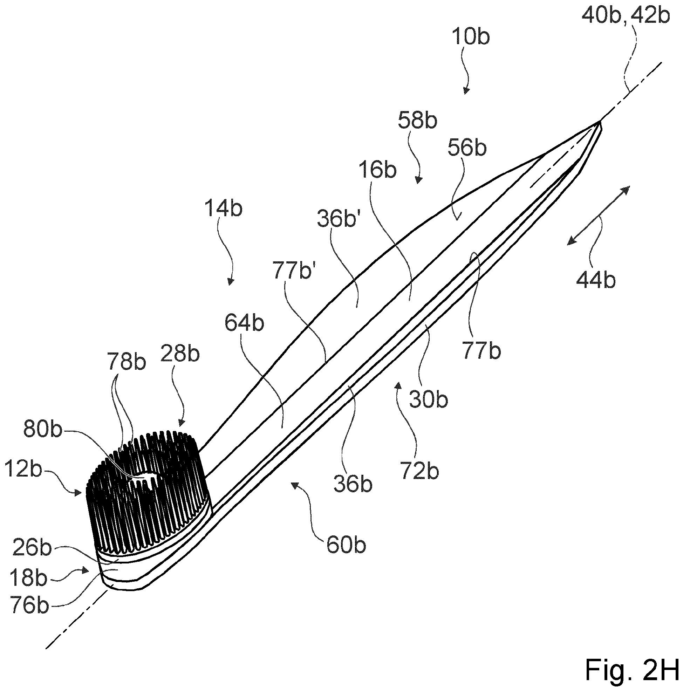

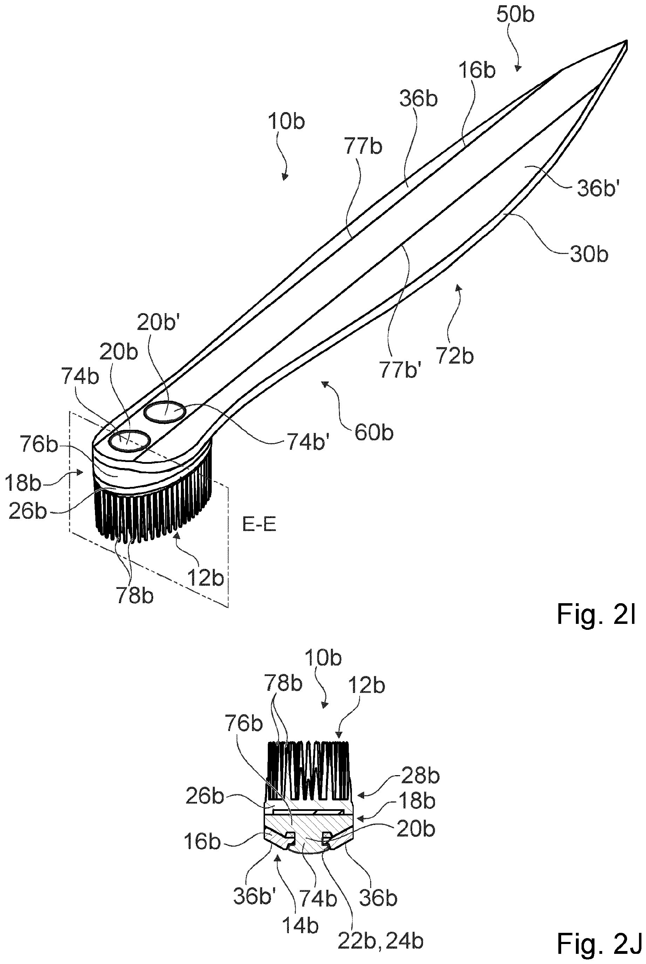

In den Figuren 2A bis 6C sind vier weitere Ausführungsbeispiele der Erfindung gezeigt. Die nachfolgenden Beschreibungen beschränken sich im Wesentlichen auf die Unterschiede zwischen den Ausführungsbeispielen, wobei bezüglich gleichbleibender Bauteile, Merkmale und Funktionen auf die Beschreibung der anderen Ausführungsbeispiele, insbesondere der Figuren 1A bis 1K, verwiesen werden kann. Zur Unterscheidung der Ausführungsbeispiele ist der Buchstabe a in den Bezugszeichen des Ausführungsbeispiels der Figuren 1A bis 1K durch die Buchstaben b bis e in den Bezugszeichen der Ausführungsbeispiele der Figuren 2A bis 6C ersetzt. Bezüglich gleich bezeichneter Bauteile, insbesondere in Bezug auf Bauteile mit gleichen Bezugszeichen, kann grundsätzlich auch auf die Zeichnungen und/oder die Beschreibung der anderen Ausführungsbeispiele, insbesondere der Figuren 1A bis 1K, verwiesen werden.

Im Folgenden wird auf die Figuren 2A bis 2J Bezug genommen, welche unterschiedliche Ansichten und Fertigungsstufen des Körperpflegeprodukts 10b zeigen. Aufgrund der unterschiedlichen Ansichten sind einige Elemente nicht in sämtlichen Figuren dargestellt und entsprechend nicht in sämtlichen Figuren mit Bezugszeichen versehen.

Die Figur 2A zeigt eine Griffeinheit 14b eines Körperpflegeprodukts 10b in einer schematischen perspektivischen Darstellung. Das Körperpflegeprodukt 10b ist von einem Mundhygienemittel gebildet. Das Körperpflegeprodukt 10b ist im vorliegenden Fall als eine Zahnbürste ausgebildet. Das Körperpflegeprodukt 10b ist als Einweg-Zahnbürste ausgebildet. Das Körperpflegeprodukt 10b ist als Reise- und/oder Kompaktzahnbürste ausgebildet.

Das Körperpflegeprodukt 10b weist eine Anwendungseinheit 12b auf. Ferner weist das Körperpflegeprodukt 10b eine Griffeinheit 14b auf, welche einen materiellen Griffkörper 16b aufweist. Des Weiteren weist das Körperpflegeprodukt 10b eine Verbindungseinheit 18b auf, welche die Anwendungseinheit 12b mit der Griffeinheit 14b verbindet.

Die Anwendungseinheit 12b bildet einen Bürstenkopf des Körperpflegeprodukts 10b. Im vorliegenden Fall ist die Anwendungseinheit 12b als ein Zahnbürstenkopf ausgebildet. Die Anwendungseinheit 12b weist insbesondere einen als Borstenträger ausgebildeten Grundkörper 26b auf. Der Grundkörper 26b bildet eine Verbindung mit der Verbindungseinheit 18b. Der Grundkörper 26b der Anwendungseinheit 12b bildet einen Borstenträger aus. Die Anwendungseinheit 12b weist ferner mehrere an dem Grundkörper 26b angeordnete Borsten 78b auf. Die Borsten 78b sind an den Grundkörper 26b angespritzt. Die Borsten 78b bestehen aus Material für gespritzte Borsten, dieses bildet auch den Grundkörper 26d. Die Anwendungseinheit 12b weist des Weiteren ein auf dem Grundkörper 26b, zwischen den Borsten 78b angeordnetes Funktionselement 80b auf. Das Funktionselement 80b ist beispielsweise von einer Kapsel zur Aufnahme von Mundwasser und/oder Zahncreme gebildet, welche dazu vorgesehen ist, bei einer Benutzung aufzuplatzen und einen Inhalt freizugeben.

Die Griffeinheit 14b ist zu einem Greifen durch den Bediener vorgesehen. Die Griffeinheit 14b weist den materiellen Griffkörper 16b auf. Der materielle Griffkörper 16b der Griffeinheit 14b weist einen Kopfbereich 28b auf. Ferner weist der materielle Griffkörper 16b der Griffeinheit 14b einen Griffbereich 72b und einen zwischen dem Griffbereich 72b und dem Kopfbereich 28b angeordneten Halsbereich 60b auf. Der Kopfbereich 28b ist zu einer Anbindung der Anwendungseinheit 12b vorgesehen. Die Griffeinheit 14b weist zumindest eine Grifffläche 56b auf, welche eine Oberfläche des materiellen Griffkörpers 16b ausbildet. Die Grifffläche 56b bildet entlang der Längsachse 42b auf einer Vorderseite 58b eine konkave Form aus. Der Griffkörper 16b ist auf einer Rückseite 50b in einem Bereich der Grifffläche 56b konvex geformt. Der Griffkörper 16b weist in einem Bereich der Grifffläche 56b eine ovale Grundform auf. Der Griffkörper 16b ist dreidimensional verformt ausgebildet. Ferner weist der materielle Griffkörper 16b der Griffeinheit 14b zwei Stabilisierungswände 36b, 36b' auf, welche zu einer Haupterstreckungsebene der Griffeinheit 14b abgewinkelt sind und sich im Wesentlichen parallel zu einer Längsachse 40b der Griffeinheit 14b erstrecken. Die Stabilisierungswände 36b, 36b' sind jeweils über eine Faltkante 77b, 77b' gegenüber einem Rest des materiellen Griffkörpers 16b zu der Vorderseite 58b hin abgewinkelt, sodass auf der Vorderseite 58b eine sich über den gesamten Griffkörper 16b erstreckende Vertiefung entsteht. Zwischen den Stabilisierungswänden 36b, 36b' ist ein Mittenbereich des materiellen Griffkörpers 16b angeordnet. Die Stabilisierungswände 36b, 36b' erstecken sich jeweils über den Griffbereich 72b, den Halsbereich 60b und den Kopfbereich 28b des materiellen Griffkörpers 16b. Die Stabilisierungswände 36b, 36b' sind an einer im Wesentlichen parallel zu der Längsachse 40b verlaufenden Außenkante des materiellen Griffkörpers 16b angeordnet. Die Stabilisierungswände 36b, 36b' sind senkrecht zu der Längsachse 40b der Griffeinheit 14b in einem Winkel von zumindest 10°, vorzugsweise zumindest 20°, gegenüber einer Haupterstreckungsebene des Griffkörpers 16b abgewinkelt. Die Stabilisierungswände 36b, 36b' bilden jeweils eine freie Außenkante des materiellen Griffkörpers 16b aus.

Der materielle Griffkörper 16b besteht zumindest zu einem Großteil aus einem Papierwerkstoff. Der materielle Griffkörper 16b besteht vollständig aus einem Papierwerkstoff. Der materielle Griffkörper 16b der Griffeinheit 14b weist zumindest eine Schicht 30b aus einem Papierwerkstoff auf, welche dreidimensional geformt ausgebildet ist. Der materielle Griffkörper 16b besteht aus genau einer Schicht 30b aus einem Papierwerkstoff.

Ferner weist das Körperpflegeprodukt 10b die Verbindungseinheit 18b auf. Die Verbindungseinheit 18b ist zu einer formschlüssigen Verbindung mit dem materiellen Griffkörper 16b der Griffeinheit 14b vorgesehen.

Die Verbindungseinheit 18b weist zumindest ein Formschlusselement 20b, 20b' auf. Die Verbindungseinheit 18b weist beispielhaft zwei Formschlusselemente 20b, 20b' auf. Die Formschlusselemente 20b, 20b' sind dazu vorgesehen, jeweils in eine Ausnehmung 22b, 22b' der Anwendungseinheit 12b und/oder der Griffeinheit 14b einzugreifen. Die Formschlusselemente 20b, 20b' sind dazu vorgesehen, jeweils in eine Ausnehmung 22b, 22b' der Griffeinheit 14b einzugreifen. Die Ausnehmungen 22b, 22b' der Griffeinheit 14b sind von Durchgangsausnehmungen 24b, 24b' gebildet. Der materielle Griffkörper 16b begrenzt die zwei Durchgangsausnehmungen 24b, 24b'. Die Durchgangsausnehmungen 24b, 24b' sind in dem Kopfbereich 28b des materiellen Griffkörpers 16b eingebracht. Die Durchgangsausnehmungen 24b, 24b' sind entlang der Längsachse 40b der Griffeinheit 14b hintereinander angeordnet. Die Durchgangsausnehmungen 24b, 24b' sind jeweils in eine zylindrische Erhebung des materiellen Griffkörpers 16b eingebracht, welche auf der Vorderseite 58b jeweils eine ringförmige Erhebung 82b, 82b' um die Durchgangsausnehmungen 24b, 24b' ausbildet und auf der Rückseite 50b jeweils eine ringförmige Vertiefung 84b, 84b' um die Durchgangsausnehmungen 24b, 24b' ausbildet. Die Vertiefungen 84b, 84b' bilden jeweils eine Stufe in den Durchgangsausnehmungen 24b, 24b' aus. Die Durchgangsausnehmungen 24b, 24b' sind dazu vorgesehen, die Formschlusselemente 20b, 20b' der Verbindungseinheit 18b aufzunehmen. Die Durchgangsausnehmungen 24b, 24b' sind jeweils von einer kreiszylindrischen Ausnehmung 22b, 22b' gebildet. Die Formschlusselemente 20b, 20b' weisen ebenfalls eine zylindrische Grundform auf. Die Formschlusselemente 20b, 20b' sind jeweils von einem Niet gebildet. Die Formschlusselemente 20b, 20b' sind jeweils von einem Kunststoffniet gebildet. Die Formschlusselemente 20b, 20b' weisen in einem mit der Griffeinheit 14b verbundenen Zustand jeweils einen Schaft sowie einen verformten Formschlusskopf 74b, 74b' auf. Der Formschlusskopf 74b, 74b' der Formschlusselemente 20b, 20b' weist gegenüber den Durchgangsausnehmungen 24b, 24b' einen vergrößerten Durchmesser auf und ist auf einer der Anwendungseinheit 12b abgewandten Seite der Griffeinheit 14b angeordnet. Die Formschlussköpfe 74b, 74b' der Formschlusselemente 20b, 20b' verhindern ein Zurückziehen der Verbindungseinheit 18b. Die Formschlussköpfe 74b, 74b' der Formschlusselemente 20b, 20b' erstrecken sich jeweils in die Vertiefungen 84b, 84b' um die Durchgangsausnehmungen 24b, 24b' und stehen somit vorzugsweise nicht über die weitere Grifffläche 56b vor.

Der materielle Griffkörper 16b der Griffeinheit 14b weist in dem Kopfbereich 28b ferner die Vertiefung auf, welche zu einer Positionierung der Verbindungseinheit 18b relativ zu der Griffeinheit 14b vorgesehen ist. Die Vertiefung ist von einer länglichen Vertiefung in dem Kopfbereich gebildet, die sich parallel zu der Längsachse 40b der Griffeinheit 14b erstreckt. Die Verbindungseinheit 18b weist einen zu der Vertiefung bzw. der ersten Nut 64b korrespondierenden Steg 76b auf, welcher sich parallel zu einer Längsachse 42b des Körperpflegeprodukts 10b über einen Großteil einer Erstreckung der Verbindungseinheit 18b erstreckt. Der Steg 76b weist einen trapezförmigen Querschnitt auf. Eine Form des Stegs 76b ist an eine Form der ersten Nut 64b in dem Kopfbereich 28b angepasst. Die Formschlusselemente 20b, 20b' sind auf dem Steg 76b angeordnet. Die Vertiefung sowie der Steg 76b sind zu einer Vorpositionierung der Verbindungseinheit 18b an der Griffeinheit 14b vorgesehen und dienen zudem zu einer teilweisen Aufnahme von Querkräften. Die Verbindung der Vertiefung mit dem Steg 76b bildet eine formschlüssige Verbindung der Verbindungseinheit 18b aus. Ferner können die ringförmigen Erhebungen 82b, 82b' ebenfalls zu einem Formschluss dienen. Hierfür können in dem Steg 76b um die Formschlusselemente 20b, 20b' jeweils nicht weiter sichtbar ringförmige Vertiefungen angeordnet sein, in welche die ringförmigen Erhebungen 82b, 82b' eingreifen. Die Formschlusselemente 20b, 20b' bilden eine weitere formschlüssige Verbindung der Verbindungseinheit 18b mit der Griffeinheit 14b aus.

Die Anwendungseinheit 12b weist den Grundkörper 26b auf. Der Grundkörper 26b ist fest mit der Verbindungseinheit 18b verbunden. Der Grundkörper 26b ist einstückig mit der Verbindungseinheit 18b verbunden. Der Grundkörper 26b ist mittels einer Klebe- und/oder Schweißverbindung mit der Verbindungseinheit 18b, insbesondere mit dem Steg 76b, verbunden.

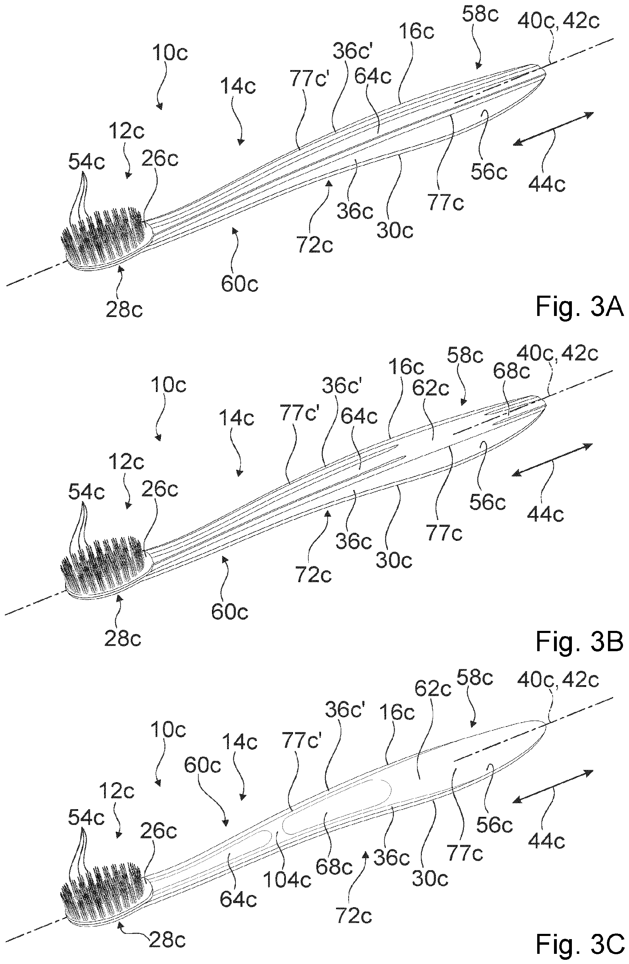

Die Figuren 3A, 3B und 3C zeigen jeweils ein weiteres alternatives erfindungsgemäßes Körperpflegeprodukt 10c. Das Körperpflegeprodukt 10c ist von einem Mundhygienemittel gebildet. Das Körperpflegeprodukt 10c ist im vorliegenden Fall als eine Zahnbürste ausgebildet.

Das Körperpflegeprodukt 10c weist eine Anwendungseinheit 12c auf. Ferner weist das Körperpflegeprodukt 10c eine Griffeinheit 14c auf, welche einen materiellen Griffkörper 16c aufweist. Des Weiteren weist das Körperpflegeprodukt 10c eine Verbindungseinheit 18c auf, welche die Anwendungseinheit 12c mit der Griffeinheit 14c verbindet.

Die Griffeinheit 14c ist zu einem Greifen durch den Bediener vorgesehen. Die Griffeinheit 14c weist den materiellen Griffkörper 16c auf. Der materielle Griffkörper 16c der Griffeinheit 14c weist einen Kopfbereich 28c auf. Ferner weist der materielle Griffkörper 16c der Griffeinheit 14c einen Griffbereich 72c und einen zwischen dem Griffbereich 72c und dem Kopfbereich 28c angeordneten Halsbereich 60c auf. Der Kopfbereich 28c ist zu einer Anbindung der Anwendungseinheit 12c vorgesehen.

Der materielle Griffkörper 16c besteht zumindest zu einem Großteil aus einem Papierwerkstoff. Der materielle Griffkörper 16c besteht vollständig aus einem Papierwerkstoff. Der materielle Griffkörper 16c der Griffeinheit 14c weist zumindest eine Schicht 30c aus einem Papierwerkstoff auf, welche dreidimensional geformt ausgebildet ist. Der materielle Griffkörper 16c besteht aus genau einer Schicht 30c aus einem Papierwerkstoff, es wäre jedoch auch ein mehrschichtiger Aufbau denkbar.

Der materielle Griffkörper 16c der Griffeinheit 14c ist entlang einer Längsrichtung der Griffeinheit 14c im Wesentlichen S-förmig geschwungen ausgebildet. Der materielle Griffkörper 16c weist in einem Längsschnitt und/oder in einer Längsansicht eine S-förmige Längsform auf. Das Profil der Griffeinheit 14c ist auf eine Anwendung sowie eine Ergonomie für den Benutzer optimiert. Der materielle Griffkörper 16c weist in Längsrichtung eine kontinuierliche, stetige, gerundete Form auf.

Die Figuren 3A, 3B und 3C zeigen jeweils Abwandlungen der Griffeinheit 14a des ersten Ausführungsbeispiels der Figuren 1A bis 1K. Im Folgenden werden daher insbesondere lediglich die Unterschiede der verschiedenen Ausgestaltungen der Griffeinheit 14c, insbesondere gegenüber der Griffeinheit 14a des ersten Ausführungsbeispiels der Figuren 1A bis 1K, beschrieben.

Die Griffeinheit 14c der Figur 3A weist zumindest eine Grifffläche 56c auf, welche eine Oberfläche des materiellen Griffkörpers 16c ausbildet. Die Grifffläche 56c bildet entlang einer Längsachse 42c auf einer Vorderseite 58c eine bombierte bzw. konvexe Form aus. Die bombierte Form ist entlang der Längsachse 42c auf der Rückseite umgekehrt ausgebildet. Der Griffkörper 16c ist auf der Rückseite in einem Bereich der Grifffläche 56c konkav geformt. Der Griffkörper 16c weist in einem Bereich der Grifffläche 56c eine ovale Grundform auf. Der Griffkörper 16c ist dreidimensional verformt, um die bombierte, konvexe Form auszubilden. Ferner weist der materielle Griffkörper 16c der Griffeinheit 14c zwei Stabilisierungswände 36c, 36c' auf, welche zu einer Haupterstreckungsebene der Griffeinheit 14c abgewinkelt sind und sich im Wesentlichen parallel zu einer Längsachse 40c der Griffeinheit 14c erstrecken. Die Stabilisierungswände 36c, 36c' sind jeweils über eine Faltkante 77c, 77c' gegenüber einem Rest des materiellen Griffkörpers 16c abgewinkelt. Zwischen den Stabilisierungswänden 36c, 36c' ist ein Mittenbereich des materiellen Griffkörpers 16c angeordnet. Die Stabilisierungswände 36c, 36c' erstrecken sich jeweils über den Griffbereich 72c und den Halsbereich 60c des materiellen Griffkörpers 16c. Der Griffkörper 16c weist ferner eine im Wesentlichen konstante Materialstärke auf. Ferner weist der Griffkörper 16c eine Nut 64c auf, welche sich auf der Vorderseite 58c von der Anwendungseinheit 12c über den Halsbereich 60c über die Grifffläche 56c bis einem unteren Ende der Griffeinheit 14c erstreckt. Die Nut 64c verläuft mittig parallel zu der Längsachse 40c der Griffeinheit 14c. Die Nut 64c weist entlang einer Erstreckung parallel zu der Längsachse 40c einen zumindest annähernd konstanten Querschnitt auf, wobei sich die Nut 64c in dem Halsbereich 60c teilweise verjüngt. Die Nut 64c weist einen trapezförmigen Querschnitt auf, welcher sich zu einer Vorderseite 58c hin aufweitet. Auf einer Rückseite des materiellen Griffkörpers 16c ist eine zu der Nut 64c korrespondierende stegförmige Erhebung ausgebildet. Die stegförmige Erhebung entsteht dabei insbesondere durch die konstante Materialstärke bei einer Herstellung der Nut 64c.

Die Griffeinheit 14c der Figur 3B weist zumindest eine Grifffläche 56c auf, welche eine Oberfläche des materiellen Griffkörpers 16c ausbildet. Die Grifffläche 56c bildet entlang der Längsachse 42c auf der Vorderseite 58c eine bombierte bzw. konvexe Form aus. Die bombierte Form ist entlang der Längsachse 42c auf der Rückseite umgekehrt ausgebildet. Der Griffkörper 16c ist auf der Rückseite in einem Bereich der Grifffläche 56c konkav geformt. Der Griffkörper 16c weist in einem Bereich der Grifffläche 56c eine ovale Grundform auf. Der Griffkörper 16c ist dreidimensional verformt um die bombierte, konvexe Form auszubilden. Ferner weist der materielle Griffkörper 16c der Griffeinheit 14c zwei Stabilisierungswände 36c, 36c' auf, welche zu einer Haupterstreckungsebene der Griffeinheit 14d abgewinkelt sind und sich im Wesentlichen parallel zu einer Längsachse 40c der Griffeinheit 14c erstreckt. Die Stabilisierungswände 36c, 36c' sind jeweils über eine Faltkante 77c, 77c' gegenüber einem Rest des materiellen Griffkörpers 16c abgewinkelt. Zwischen den Stabilisierungswände 36c, 36c' ist ein Mittenbereich des materiellen Griffkörpers 16c angeordnet. Die Stabilisierungswände 36c, 36c' erstrecken sich jeweils über den Griffbereich 72c und den Halsbereich 60c des materiellen Griffkörpers 16c. Der Griffkörper 16c bildet auf der Grifffläche 56c ein Plateau 62c aus. Das Plateau 62c dient zu einer Aufnahme eines Schriftzugs und/oder eines Logos. Das Plateau 62c ist zumindest teilweise mittig auf der Grifffläche 56c angeordnet. Das Plateau 62c ist in einem Bereich einer maximalen Breite des Körperpflegeprodukts 10c angeordnet. Der Griffkörper 16c weist ferner eine im Wesentlichen konstante Materialstärke auf. Ferner weist der Griffkörper 16c eine erste Nut 64c auf, welche sich auf der Vorderseite 58c von der Anwendungseinheit 12c über den Halsbereich 60c über die Grifffläche 56c bis zu dem Plateau 62c erstreckt, wobei die erste Nut 64c fließend in das Plateau 62c ausläuft. Die erste Nut 64c verläuft mittig parallel zu der Längsachse 40c der Griffeinheit 14c. Die erste Nut 64c weist entlang einer Erstreckung parallel zu der Längsachse 40c einen zumindest annähernd konstanten Querschnitt auf, wobei sich die erste Nut in dem Halsbereich 60c teilweise verjüngt. Die erste Nut 64c weist einen trapezförmigen Querschnitt auf, welcher sich zu einer Vorderseite 58c hin aufweitet. Auf einer Rückseite des materiellen Griffkörpers 16c ist eine zu der ersten Nut 64c korrespondierende erste stegförmige Erhebung ausgebildet. Die erste stegförmige Erhebung entsteht dabei insbesondere durch die konstante Materialstärke bei einer Herstellung der ersten Nut 64c. Des Weiteren weist der Griffkörper 16c eine zweite Nut 68c auf, welche sich auf der Vorderseite 58c von einem der Anwendungseinheit 12c angewandten unteren Ende der Griffeinheit 14c über die Grifffläche 56c bis zu dem Plateau 62c erstreckt, wobei die zweite Nut 68c fließend in das Plateau 62c ausläuft. Die zweite Nut 68c verläuft mittig parallel zu der Längsachse 40c der Griffeinheit 14c. Die zweite Nut 68c weist entlang einer Erstreckung parallel zu der Längsachse 40c einen zumindest annähernd konstanten Querschnitt auf. Die zweite Nut 68c weist einen trapezförmigen Querschnitt auf, welcher sich zu einer Vorderseite 58c hin aufweitet. Die zweite Nut 68c verläuft im Wesentlichen fluchtend zu der ersten Nut 64c, wobei die ersten Nut 64c und die zweite Nut 68c durch das Plateau 62c unterbrochen sind. Auf einer Rückseite des materiellen Griffkörpers 16c ist eine zu der zweiten Nut 68c korrespondierende zweite stegförmige Erhebung ausgebildet. Die zweite stegförmige Erhebung entsteht dabei insbesondere durch die konstante Materialstärke bei einer Herstellung der zweiten Nut 68c.

Die Griffeinheit 14c der Figur 3C weist zumindest eine Grifffläche 56c auf, welche eine Oberfläche des materiellen Griffkörpers 16c ausbildet. Die Grifffläche 56c bildet entlang der Längsachse 42c auf der Vorderseite 58c eine bombierte bzw. konvexe Form aus. Die bombierte Form ist entlang der Längsachse 42c auf der Rückseite umgekehrt ausgebildet. Der Griffkörper 16c ist auf der Rückseite in einem Bereich der Grifffläche 56c konkav geformt. Der Griffkörper 16c weist in einem Bereich der Grifffläche 56c eine ovale Grundform auf. Der Griffkörper 16c ist dreidimensional verformt um die bombierte, konvexe Form auszubilden. Ferner weist der materielle Griffkörper 16c der Griffeinheit 14c zwei Stabilisierungswände 36c, 36c' auf, welche zu einer Haupterstreckungsebene der Griffeinheit 14d abgewinkelt sind und sich im Wesentlichen parallel zu einer Längsachse 40c der Griffeinheit 14c erstreckt. Der materielle Griffkörper 16c geht fließend, insbesondere frei von Faltkanten von einem Mittenbereich in die

Stabilisierungswände 36c, 36c' über, wobei die Stabilisierungswände 36c, 36c' gegenüber einem Rest des materiellen Griffkörpers 16c abgewinkelt sind. Zwischen den Stabilisierungswände 36c, 36c' ist der Mittenbereich des materiellen Griffkörpers 16c angeordnet. Die Stabilisierungswände 36c, 36c' erstrecken sich jeweils über den Griffbereich 72c und den Halsbereich 60c des materiellen Griffkörpers 16c. Der Griffkörper 16c bildet auf der Grifffläche 56c ein Plateau 62c aus. Das Plateau 62c dient als Auflagefläche für eine Handfläche. Ferner weist der Griffkörper 16c einen Daumengriff 104c auf. Der Daumengriff 104c ist in einem Übergang von dem Griffbereich 72c in den Halsbereich 60c angeordnet. Der Griffkörper 16c weist ferner eine im Wesentlichen konstante Materialstärke auf. Ferner weist der Griffkörper 16c eine erste Nut 64c auf, welche sich auf der Vorderseite 58c von der Anwendungseinheit 12c über den Halsbereich 60c bis zu dem Daumengriff 104c erstreckt, wobei die erste Nut 64c fließend in den Daumengriff 104c sowie einen umlaufenden Rand der ersten Nut 64c ausläuft. Die erste Nut 64c verläuft mittig parallel zu der Längsachse 40c der Griffeinheit 14c. Die erste Nut 64c weist entlang einer Erstreckung parallel zu der Längsachse 40c einen zumindest annähernd konstanten Querschnitt auf, wobei sich die erste Nut in dem Halsbereich 60c teilweise verjüngt. Auf einer Rückseite des materiellen Griffkörpers 16c ist eine zu der ersten Nut 64c korrespondierende erste stegförmige Erhebung ausgebildet. Die erste stegförmige Erhebung entsteht dabei insbesondere durch die konstante Materialstärke bei einer Herstellung der ersten Nut 64c. Des Weiteren weist der Griffkörper 16c eine zweite Nut 68c auf, welche sich auf der Vorderseite 58c von einer Mitte des Griffbereichs 72c bis zu dem Daumengriff 104c erstreckt, wobei die zweite Nut 68c fließend in den Daumengriff 104c sowie einen umlaufenden Rand der zweiten Nut 68c ausläuft. Die zweite Nut 68c verläuft mittig parallel zu der Längsachse 40c der Griffeinheit 14c. Die zweite Nut 68c weist entlang einer Erstreckung parallel zu der Längsachse 40c einen zumindest annähernd konstanten Querschnitt auf. Die zweite Nut 68c verläuft im Wesentlichen fluchtend zu der ersten Nut 64c, wobei die erste Nut 64c und die zweite Nut 68c durch den Daumengriff 104c unterbrochen sind. Auf einer Rückseite des materiellen Griffkörpers 16c ist eine zu der zweiten Nut 68c korrespondierende zweite stegförmige Erhebung ausgebildet. Die zweite stegförmige Erhebung entsteht dabei insbesondere durch die konstante Materialstärke bei einer Herstellung der zweiten Nut 68c.

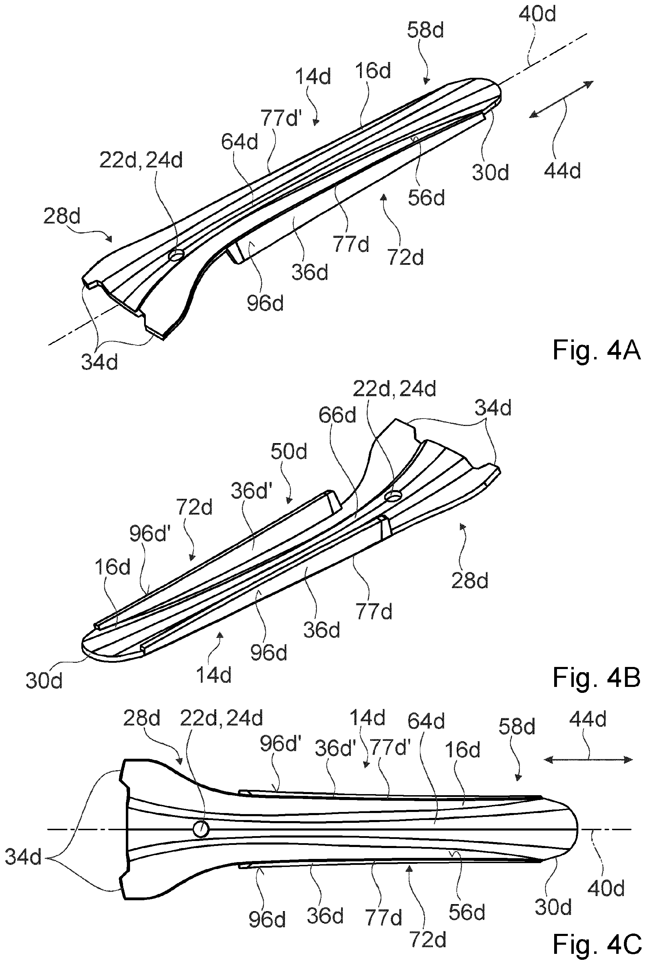

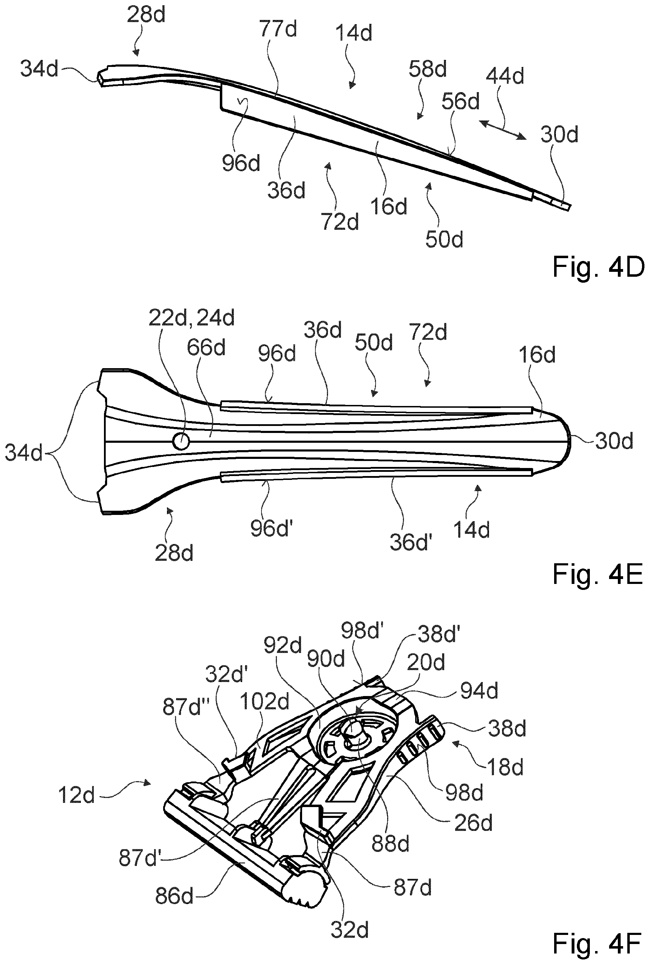

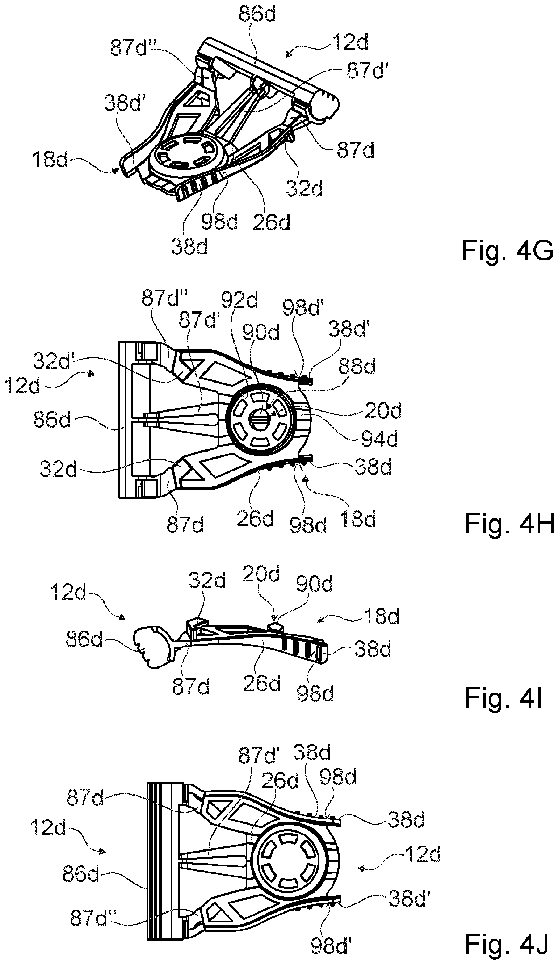

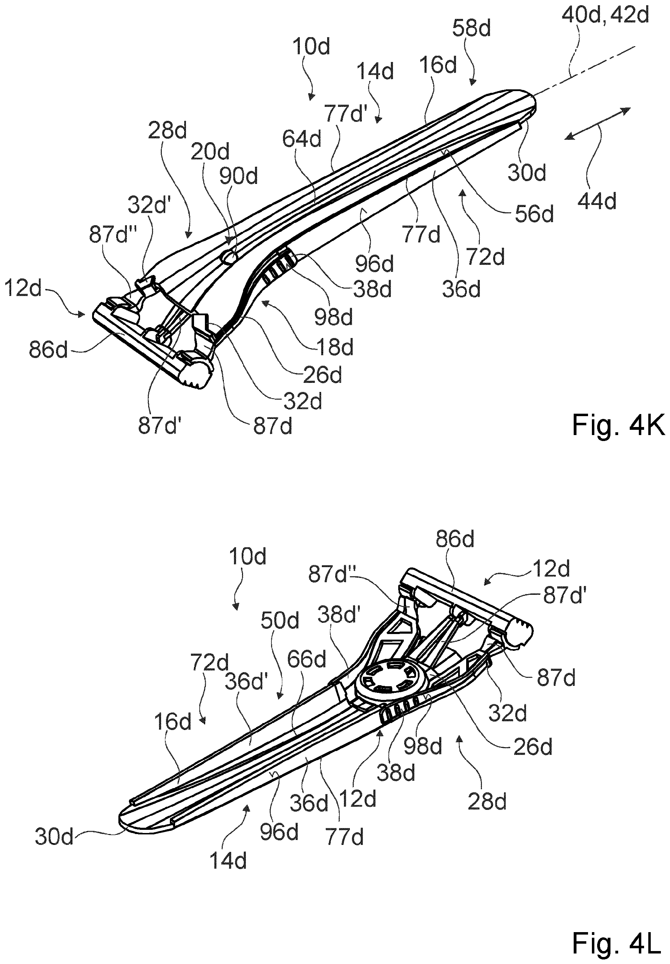

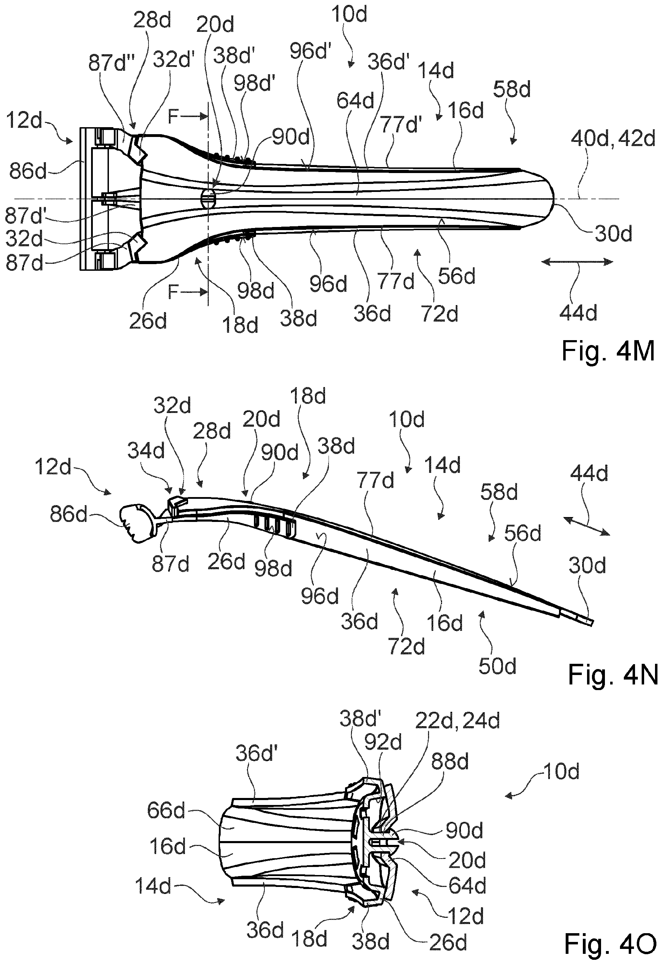

Im Folgenden wird auf die Figuren 4A bis 4O Bezug genommen, welche unterschiedliche Ansichten und Fertigungsstufen des Körperpflegeprodukts 10d zeigen. Aufgrund der unterschiedlichen Ansichten sind einige Elemente nicht in sämtlichen Figuren dargestellt und entsprechend nicht in sämtlichen Figuren mit Bezugszeichen versehen.

Die Figur 4A zeigt eine Griffeinheit 14d eines Körperpflegeprodukts 10d in einer schematischen perspektivischen Darstellung. Das Körperpflegeprodukt 10d ist im vorliegenden Fall als ein Rasierer ausgebildet.

Das Körperpflegeprodukt 10d weist eine Anwendungseinheit 12d auf. Ferner weist das Körperpflegeprodukt 10d eine Griffeinheit 14d auf, welche einen materiellen Griffkörper 16d aufweist. Des Weiteren weist das Körperpflegeprodukt 10d eine Verbindungseinheit 18d auf, welche die Anwendungseinheit 12d mit der Griffeinheit 14d verbindet.

Die Anwendungseinheit 12d bildet einen Rasierkopf des Körperpflegeprodukts 10d. Die Anwendungseinheit 12d weist insbesondere einen als Klingenträger ausgebildeten Grundkörper 26d auf. Der Grundkörper 26d der Anwendungseinheit 12d ist vollständig aus einer Hartkomponente ausgebildet. Es wäre jedoch auch denkbar, dass der Grundkörper 26d der Anwendungseinheit 12d aus einer Hartkomponente und einer Weichkomponente gebildet ist. Der Grundkörper 26d der Anwendungseinheit 12d bildet einen statischen Klingenträger aus, welcher zu einer beweglichen, insbesondere schwenkbaren Aufnahme eines Klingenkopfes 86d vorgesehen ist. Die Anwendungseinheit 12d weist den an dem Grundkörper 26d angeordneten Klingenkopf 86d auf. Der Grundkörper 26d weist drei Gelenkarme 87d, 87d', 87d" auf, welche zu einer schwenkbaren Aufnahme des Klingenkopfes 86d vorgesehen sind. Der Klingenkopf 86d kann insbesondere auswechselbar mit dem Grundkörper 26d verbunden sein.

Die Griffeinheit 14d ist zu einem Greifen durch den Bediener vorgesehen. Die Griffeinheit 14d weist den materiellen Griffkörper 16d auf. Der materielle Griffkörper 16d der Griffeinheit 14d weist einen Kopfbereich 28d auf. Ferner weist der materielle Griffkörper 16d der Griffeinheit 14d einen Griffbereich 72d auf. Der Kopfbereich 28d ist zu einer Anbindung der Anwendungseinheit 12d vorgesehen. Die Griffeinheit 14d weist zumindest eine Grifffläche 56d auf, welche eine Oberfläche des materiellen Griffkörpers 16d ausbildet. Die Grifffläche 56d verläuft auf einer Vorderseite 58d von dem Kopfbereich 28d des Griffkörpers 16d bis zum unteren Ende des Körperpflegeprodukts 10d an der Unterseite. Die Grifffläche 56d erstreckt sich über den Griffbereich 72d des materiellen Griffkörpers 16d. Die Grifffläche 56d bildet entlang der Längsachse 40d auf der Vorderseite 58d eine teilweise bombierte bzw. teilweise konvexe Form aus. Die bombierte Form ist entlang der Längsachse 40d auf der Rückseite 50d umgekehrt ausgebildet. Der Griffkörper 16d ist auf der Rückseite 50d in einem Bereich der Grifffläche 56d teilweise konkav geformt. Der Griffkörper 16d weist in der Draufsicht in einem Bereich der Grifffläche 56d eine I-förmige Grundform auf. Der gesamte Griffkörper 16d ist in der Draufsicht insbesondere teilweise Y-förmig oder T-förmig ausgebildet. Der Griffkörper 16d ist dreidimensional verformt, um die bombierte, konvexe Form auszubilden. Ferner weist der materielle Griffkörper 16d der Griffeinheit 14d zwei Stabilisierungswände 36d, 36d' auf, welche zu einer Haupterstreckungsebene der Griffeinheit 14d abgewinkelt sind und sich im Wesentlichen parallel zu einer Längsachse 40d der Griffeinheit 14d erstrecken. Die Stabilisierungswände 36d, 36d' sind jeweils über eine Faltkante 77d, 77d' gegenüber einem Rest des materiellen Griffkörpers 16d abgewinkelt. Zwischen den Stabilisierungswänden 36d, 36d' ist ein Mittenbereich des materiellen Griffkörpers 16d angeordnet. Die Stabilisierungswände 36d, 36d' erstecken sich jeweils über den Griffbereich 72d des materiellen Griffkörpers 16d. Die Stabilisierungswände 36d, 36d' sind an einer im Wesentlichen parallel zu der Längsachse 40d verlaufenden Außenkante des materiellen Griffkörpers 16d angeordnet. Die Stabilisierungswände 36d, 36d' sind senkrecht zu der Längsachse 40d der Griffeinheit 14d in einem Winkel von zumindest 60°, vorzugsweise zumindest 80°, gegenüber einer Haupterstreckungsebene des Griffkörpers 16d abgewinkelt. Die Stabilisierungswände 36d, 36d' bilden jeweils eine freie Außenkante des materiellen Griffkörpers 16d aus.

Die Stabilisierungswände 36d, 36d' bilden zudem jeweils eine Haltefläche 96d, 96d' aus. Die Halteflächen 96d, 96d' sind dazu seitlich außen an den Stabilisierungswänden 36d, 36d' gestaltet. Die Halteflächen 96d, 96d' sind insbesondere dazu vorgesehen, das Halten zu vereinfachen.

Der Griffkörper 16d weist ferner eine im Wesentlichen konstante Materialstärke auf. Ferner weist der Griffkörper 16d eine Nut 64d auf, welche sich auf der Vorderseite 58d von der Anwendungseinheit 12d über die Grifffläche 56d bis zu einer Unterseite der Griffeinheit 14d erstreckt. Die Nut 64d verläuft mittig parallel zu der Längsachse 40d der Griffeinheit 14d. Die Nut 64d weist entlang einer Erstreckung parallel zu der Längsachse 40d einen zumindest annähernd konstanten Querschnitt auf, wobei sich die Nut 64d zu der Anwendungseinheit 12d hin leicht verbreitert. Die Nut 64d weist einen trapezförmigen Querschnitt auf, welcher sich zu der Vorderseite 58d hin aufweitet. Auf der Rückseite 50d des materiellen Griffkörpers 16d ist eine zu der Nut 64d korrespondierende stegförmige Erhebung 66d ausgebildet. Die stegförmige Erhebung 66d entsteht dabei insbesondere durch die konstante Materialstärke bei einer Herstellung der Nut 64d.

Der materielle Griffkörper 16d besteht zumindest zu einem Großteil aus einem Papierwerkstoff. Der materielle Griffkörper 16d besteht vollständig aus einem Papierwerkstoff. Der materielle Griffkörper 16d der Griffeinheit 14d weist zumindest eine Schicht 30d aus einem Papierwerkstoff auf, welche dreidimensional geformt ausgebildet ist. Der materielle Griffkörper 16d besteht aus genau einer Schicht 30d aus einem Papierwerkstoff, es wäre jedoch auch ein mehrschichtiger Aufbau denkbar.

Ferner weist das Körperpflegeprodukt 10d die Verbindungseinheit 18d auf. Die Verbindungseinheit 18d ist zu einer formschlüssigen Verbindung mit dem materiellen Griffkörper 16d der Griffeinheit 14d vorgesehen.

Die Verbindungseinheit 18d weist ein Formschlusselement 20d auf. Das Formschlusselement 20d ist dazu vorgesehen, in eine Ausnehmung 22d der Anwendungseinheit 12d und/oder der Griffeinheit 14d einzugreifen. Das Formschlusselement 20d ist dazu vorgesehen, in eine Ausnehmung 22d der Griffeinheit 14d einzugreifen. Die Ausnehmung 22d der Griffeinheit 14d ist von einer Durchgangsausnehmung 24d gebildet. Der materielle Griffkörper 16d begrenzt die Durchgangsausnehmung 24d. Die Durchgangsausnehmung 24d ist in dem Kopfbereich 28d des materiellen Griffkörpers 16d eingebracht. Die Durchgangsausnehmung 24d ist in der Nut 64d angeordnet. Die Durchgangsausnehmung 24d ist dazu vorgesehen, das Formschlusselement 20d der Verbindungseinheit 18d aufzunehmen. Die Durchgangsausnehmung 24d ist jeweils von einer kreiszylindrischen Ausnehmung 22d gebildet. Das Formschlusselement 20d weist ebenfalls eine zylindrische Grundform auf. Das Formschlusselement 20d ist von einem Rastelement gebildet. Das Rastelement ist pilzförmig mit einem Stielabschnitt 88d und einem Kopfabschnitt 90d ausgebildet. Das Rastelement ist ferner geteilt, sodass zwei federnde Teilelemente entstehen. Das Formschlusselement 20d ist in einer zylindrischen Vertiefung 92d der Verbindungseinheit 18d angeordnet. Die Vertiefung 92d ermöglicht bei einer Verbindung der Verbindungseinheit 18d mit der Griffeinheit 14d ein elastisches Auslenken des Formschlusselements 20d. Das Formschlusselement 20d ist dazu vorgesehen, den Griffkörper 16d gegen eine Auflagefläche 102d des Grundkörpers 26d zu klemmen. Die Auflagefläche 102d ist insbesondere von einer zu der Unterseite des Kopfbereichs 28d des Griffkörpers 16d korrespondierenden Fläche gebildet, an welcher der Griffkörper 16d großflächig anliegen kann.

Der materielle Griffkörper 16d der Griffeinheit 14d weist in dem Kopfbereich 28d auf der Rückseite 50d die Erhebung 66d auf, welche zu einer Positionierung der Verbindungseinheit 18d relativ zu der Griffeinheit 14d vorgesehen ist. Die Erhebung 66d ist dazu vorgesehen, in eine Vertiefung 94d der Verbindungseinheit 18d einzugreifen.

Ferner weist die Verbindungseinheit 18d zumindest eine Aufnahmenut 32d, 32d' auf. Die die Verbindungseinheit 18d weist zwei Aufnahmenuten 32d, 32d' auf. Die Aufnahmenuten 32d, 32d' sind an den äußeren Gelenkarmen 87d, 87d" des Grundkörpers 26d der Anwendungseinheit 12d angeordnet. Die Aufnahmenuten 32d, 32d' sind jeweils dazu vorgesehen, eine der Anwendungseinheit 12d zugewandte Vorderkante 34d des materiellen Griffkörpers 16d der Griffeinheit 14d aufzunehmen. Der Griffkörper 16d weist insbesondere eine Y-förmige Grundform auf, wobei insbesondere die beiden äußeren Vorderkanten von den Aufnahmenuten 32d, 32d' aufgenommen werden.

Die Aufnahmenuten 32d, 32d' sind zu einer klemmenden Aufnahme zumindest einer Kante des materiellen Griffkörpers 16d vorgesehen. Die Aufnahmenuten 32d, 32d' erstrecken sich im Wesentlichen senkrecht zu einer Längsachse 40d der Griffeinheit 14d. Es ist denkbar, dass in den Aufnahmenuten 32d, 32d' beispielsweise Widerhaken vorgesehen sind, welche eine Klemmung verbessern. Die Aufnahmenuten 32d, 32d' sind zu einer Bereitstellung einer Klemmverbindung mit dem materiellen Griffkörper 16d vorgesehen. Die Aufnahmenuten 32d, 32d' sind dabei aus Kunststoff hergestellt, sodass außen ein Kunststoff der Verbindungseinheit 18d angeordnet ist, welche innen einen Papierwerkstoff der Griffeinheit 14d aufnimmt. Die Aufnahmenuten 32d, 32d' bilden jeweils eine U-förmige Nut aus, welche zu der Griffeinheit 14d hin geöffnet sind.

Bei einer Herstellung wird die Griffeinheit 14d sowie die Verbindungseinheit 18d bereitgestellt. Anschließend werden die Griffeinheit 14d und die Verbindungseinheit 18d zusammengeführt, wobei die Vorderkante des materiellen Griffkörpers 16d leicht angewinkelt in die Aufnahmenuten 32d, 32d' eingeschoben wird. Anschließend wird der materielle Griffkörper in eine Endposition verschwenkt, wobei sich das Formschlusselement 20d, von der Rückseite 50d durch die Durchgangsausnehmung 24d erstreckt und auf der Vorderseite 58d der Griffeinheit 14d mittels dem Kopfabschnitt 90d mit dem Griffkörper 16d verrastet.

Ferner weist die Verbindungseinheit 18d zumindest eine Begrenzungswand 38d, 38d' auf. Die Verbindungseinheit 18d weist zwei Begrenzungswände 38d, 38d' auf. Die Begrenzungswände 38d, 38d' sind zu einer Haupterstreckungsebene der Verbindungseinheit 18d abgewinkelt und erstrecken sich im Wesentlichen parallel zu einer Längsachse 40d der Griffeinheit 14d. Die Begrenzungswände 38d, 38d' erstecken sich von den Aufnahmenuten 32d, 32d' in Richtung der Griffeinheit 14d. Die Begrenzungswände 38d, 38d' sind an einer im Wesentlichen parallel zu der Längsachse 42d verlaufenden Außenkante der Verbindungseinheit 18d, insbesondere des Grundkörpers 26d der Anwendungseinheit 12d, angeordnet. Die Begrenzungswände 38d, 38d' sind senkrecht zu der Längsachse der Verbindungseinheit 18d in einem Winkel von zumindest 60°, vorzugsweise zumindest 80°, gegenüber einer Haupterstreckungsebene der Verbindungseinheit 18d abgewinkelt. Die Begrenzungswände 38d, 38d' bilden jeweils eine freie Außenkante des materiellen Griffkörpers 16d aus. Ferner kragen die Begrenzungswände 38d, 38d' jeweils in Richtung der Unterseite aus. Die Begrenzungswände 38d, 38d' sind zu einer zumindest teilweisen Begrenzung und Stabilisierung der Stabilisierungswände 36d, 36d' des materiellen Griffkörpers 16d vorgesehen. Die Stabilisierungswände 36d, 36d' liegen dazu in einem der Anwendungseinheit 12d zugewandten Bereich in einem montierten Zustand innen an den Begrenzungswänden 38d, 38d' an. Die Halteflächen 96d, 96d' an den Stabilisierungswänden 36d, 36d' setzen sich in Halteflächen 98d, 98d' an den Begrenzungswänden 38d, 38d' der Verbindungseinheit 18d fort.

Die Anwendungseinheit 12d weist den Grundkörper 26d auf. Der Grundkörper 26d ist fest mit der Verbindungseinheit 18d verbunden. Der Grundkörper 26d ist einstückig mit der Verbindungseinheit 18d verbunden. Der Grundkörper 26d ist einteilig mit der Verbindungseinheit 18d ausgebildet. Die Verbindungseinheit 18d ist in den Grundkörper 26d integriert. Das Formschlusselement 20d, die Aufnahmenuten 32d, 32d' und die Begrenzungswände 38d, 38d' sind einstückig mit dem Grundkörper 26d der Anwendungseinheit 12d ausgebildet. Das Formschlusselement 20d, die Aufnahmenuten 32d, 32d' und die Begrenzungswände 38d, 38d' sind einteilig mit dem Grundkörper 26d der Anwendungseinheit 12d ausgebildet. Die Vertiefung 94d ist ebenfalls einteilig mit dem Grundkörper 26d der Anwendungseinheit 12d ausgebildet.

Alternativ wäre auch denkbar, dass die Anwendungseinheit 12d zumindest einen Grundkörper 26d aufweist, welcher wechselbar mit der Verbindungseinheit 18d verbunden ist. Dabei wäre insbesondere denkbar, dass die gesamte Anwendungseinheit 12d beispielsweise als Wechselklinge ausgebildet ist.

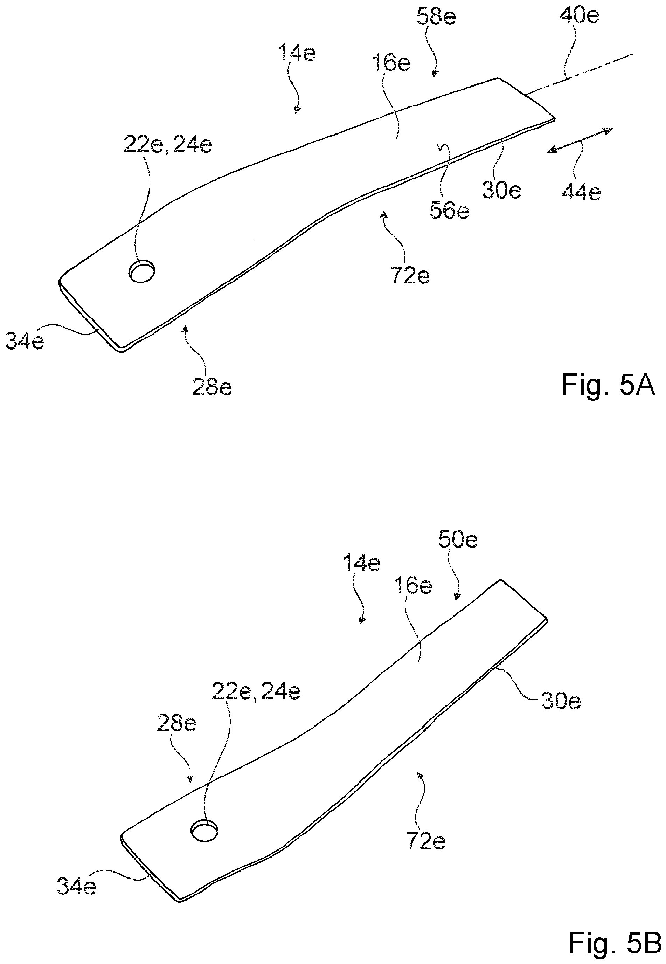

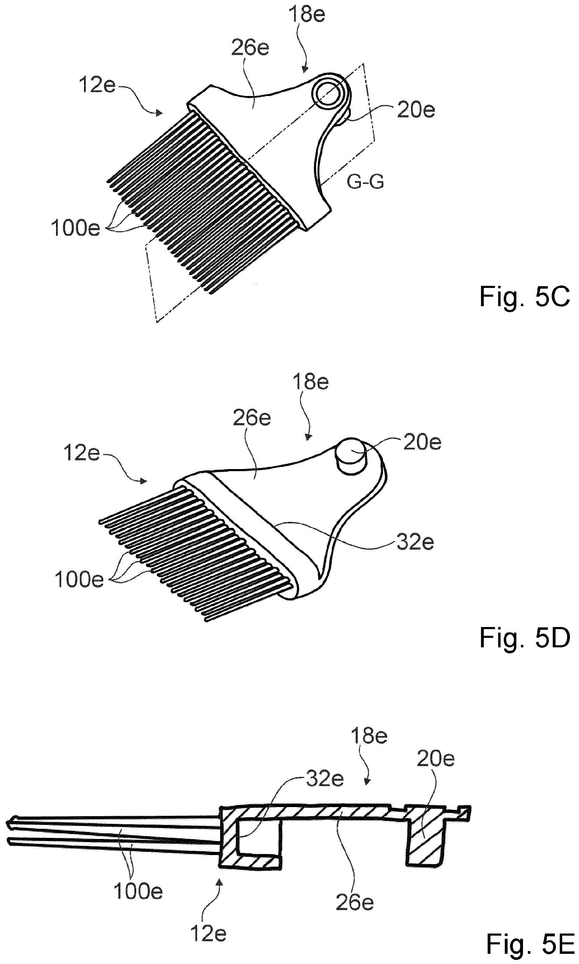

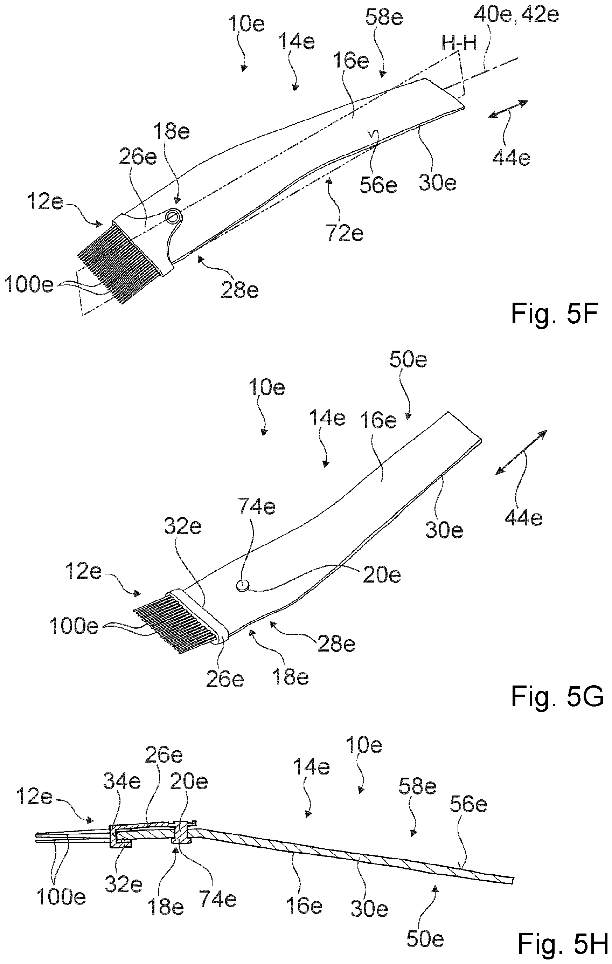

Im Folgenden wird auf die Figuren 5A bis 5H Bezug genommen, welche unterschiedliche Ansichten und Fertigungsstufen des Körperpflegeprodukts 10e zeigen. Aufgrund der unterschiedlichen Ansichten sind einige Elemente nicht in sämtlichen Figuren dargestellt und entsprechend nicht in sämtlichen Figuren mit Bezugszeichen versehen Die Figur 5A zeigt eine Griffeinheit 14e eines Körperpflegeprodukts 10e in einer schematischen perspektivischen Darstellung. Das Körperpflegeprodukt 10e ist im vorliegenden Fall als ein Pinsel, insbesondere als ein Applikationspinsel, ausgebildet.

Das Körperpflegeprodukt 10e weist eine Anwendungseinheit 12e auf. Ferner weist das Körperpflegeprodukt 10e eine Griffeinheit 14e auf, welche einen materiellen Griffkörper 16e aufweist. Des Weiteren weist das Körperpflegeprodukt 10e eine Verbindungseinheit 18e auf, welche die Anwendungseinheit 12e mit der Griffeinheit 14e verbindet.

Die Anwendungseinheit 12e bildet einen Pinselkopf des Körperpflegeprodukts 10e. Die Anwendungseinheit 12e weist insbesondere einen als Pinselhaarträger ausgebildeten Grundkörper 26e auf. Ein Grundkörper 26e der Anwendungseinheit 12e ist vollständig aus einer Hartkomponente ausgebildet. Es wäre jedoch auch denkbar, dass der Grundkörper 26e der Anwendungseinheit 12e aus einer Hartkomponente und einer Weichkomponente gebildet ist. Die Anwendungseinheit 12e weist ferner mehrere an dem Grundkörper 26e angeordnete Pinselborsten 100e auf. Die Pinselborsten 100e sind auf einer Oberseite an den Grundkörper 26e angespritzt.

Die Griffeinheit 14e ist zu einem Greifen durch den Bediener vorgesehen. Die Griffeinheit 14e weist den materiellen Griffkörper 16e auf. Der materielle Griffkörper 16e der Griffeinheit 14e weist einen Kopfbereich 28e auf. Ferner weist der materielle Griffkörper 16e der Griffeinheit 14e einen Griffbereich 72e auf. Der Kopfbereich 28e ist zu einer Anbindung der Anwendungseinheit 12e vorgesehen. Die Griffeinheit 14e weist zumindest eine Grifffläche 56e auf, welche eine Oberfläche des materiellen Griffkörpers 16e ausbildet. Die Grifffläche 56e verläuft auf einer Vorderseite 58e von dem Kopfbereich 28e des Griffkörpers 16e bis zum unteren Ende des Körperpflegeprodukts 10e an der Unterseite. Die Grifffläche 56e erstreckt sich über den Griffbereich 72e des materiellen Griffkörpers 16e. Die Grifffläche 56e bildet entlang der Längsachse 42e auf der Vorderseite 58e eine teilweise geknickte Form aus. Die geknickte Form ist entlang der Längsachse 42e auf der Rückseite 50e umgekehrt ausgebildet. Der Griffkörper 16e weist beispielhaft eine rechteckige Grundform auf. Der Griffkörper 16e weist ferner eine im Wesentlichen konstante Materialstärke auf.

Der materielle Griffkörper 16e besteht zumindest zu einem Großteil aus einem Papierwerkstoff. Der materielle Griffkörper 16e besteht vollständig aus einem Papierwerkstoff. Der materielle Griffkörper 16e der Griffeinheit 14e weist zumindest eine Schicht 30e aus einem Papierwerkstoff auf, welche dreidimensional geformt ausgebildet ist. Der materielle Griffkörper 16e besteht aus genau einer Schicht 30e aus einem Papierwerkstoff.

Ferner weist das Körperpflegeprodukt 10e die Verbindungseinheit 18e auf. Die Verbindungseinheit 18e ist zu einer formschlüssigen Verbindung mit dem materiellen Griffkörper 16e der Griffeinheit 14e vorgesehen.

Die Verbindungseinheit 18e weist ein Formschlusselement 20e auf. Das Formschlusselement 20e ist dazu vorgesehen, in eine Ausnehmung 22e der Anwendungseinheit 12e und/oder der Griffeinheit 14e einzugreifen. Das Formschlusselement 20e ist dazu vorgesehen, in eine Ausnehmung 22e der Griffeinheit 14e einzugreifen. Die Ausnehmung 22e der Griffeinheit 14e ist von einer Durchgangsausnehmung 24e gebildet. Der materielle Griffkörper 16e begrenzt die Durchgangsausnehmung 24e. Die Durchgangsausnehmung 24e ist in dem Kopfbereich 28e des materiellen Griffkörpers 16e eingebracht. Die Durchgangsausnehmung 24e ist dazu vorgesehen, das Formschlusselement 20e der Verbindungseinheit 18e aufzunehmen. Die Durchgangsausnehmung 24e ist jeweils von einer kreiszylindrischen Ausnehmung 22e gebildet. Das Formschlusselement 20e weist ebenfalls eine zylindrische Grundform auf. Das Formschlusselement 20e ist von einem Niet gebildet. Das Formschlusselement 20e ist von einem Kunststoffniet gebildet. Das Formschlusselement 20e weist in einem mit der Griffeinheit 14e verbundenen Zustand einen Schaft sowie einen Formschlusskopf 74e, 74e' auf. Der Formschlusskopf 74e, 74e' des Formschlusselements 20e weist einen gegenüber der Durchgangsausnehmung 24e einen vergrößerten Durchmesser auf und ist auf einer der Anwendungseinheit 12e abgewandten Rückseite 50e der Griffeinheit 14e angeordnet. Der Formschlusskopf 74e, 74e' des Formschlusselements 20e verhindert ein Zurückziehen der Verbindungseinheit 18e. Das Formschlusselement 20e ist an einem dreieckigen Fortsatz der Verbindungseinheit 18e angeordnet, welcher sich in Richtung einer Unterseite erstreckt.

Ferner weist die Verbindungseinheit 18e zumindest eine Aufnahmenut 32e auf. Die Aufnahmenut 32e ist in einem Bereich der Pinselborsten 100e angeordnet. Die Aufnahmenut 32e ist dazu vorgesehen, eine der Anwendungseinheit 12e zugewandte Vorderkante 34e des materiellen Griffkörpers 16e der Griffeinheit 14e aufzunehmen. Die Aufnahmenut 32e ist zu einer klemmenden Aufnahme zumindest einer Kante des materiellen Griffkörpers 16e vorgesehen. Die Aufnahmenut 32e erstreckt sich im Wesentlichen senkrecht zu einer Längsachse 40e der Griffeinheit 14e. Es ist denkbar, dass in der Aufnahmenut 32e beispielsweise Widerhaken vorgesehen sind, welche eine Klemmung verbessern. Die Aufnahmenut 32e ist zu einer Bereitstellung einer Klemmverbindung mit dem materiellen Griffkörper 16e vorgesehen. Die Aufnahmenut 32e ist dabei aus Kunststoff hergestellt, sodass außen ein Kunststoff der Verbindungseinheit 18e angeordnet ist, welcher innen einen Papierwerkstoff der Griffeinheit 14e aufnimmt. Die Aufnahmenut 32e ist dabei als eine U-förmige Nut ausgebildet, welche zu der Griffeinheit 14e hin geöffnet ist.

Bei einer Herstellung wird die Griffeinheit 14e sowie die Verbindungseinheit 18e bereitgestellt. Anschließend werden die Griffeinheit 14e und die Verbindungseinheit 18e zusammengeführt, wobei die Vorderkante 34e des materiellen Griffkörpers 16e leicht angewinkelt in die Aufnahmenut 32e eingeschoben wird. Anschließend wird der materielle Griffkörper 16e in eine Endposition verschwenkt, wobei sich das Formschlusselement 20e durch die Durchgangsausnehmung 24e erstreckt. Darauffolgend wird der hindurchstehende Schaft des Formschlusselements 20e verformt. Dabei sind verschiedene, einem Fachmann als sinnvoll erscheinende Verformungsmöglichkeiten denkbar, wie beispielsweise durch Druck, wobei das Formschlusselement 20e verdrückt bzw. angestaucht wird, oder durch Druck und Wärme, wobei das Formschlusselement 20e mit einem geheizten Stempel verformt bzw. angestaucht wird. Der Druck ist dabei insbesondere so gewählt, dass ein kontrolliertes Stauchen erfolgt. Bei einem Aufbringen des Drucks wird insbesondere eine Ausbreitung des gestauchten Formschlusselements 20e gesteuert. Vorzugsweise ist ein Stempel dafür insbesondere nicht flach, sondern konkav gewölbt. Eine runde Form der Berührungsfläche steuert insbesondere das Fließen des Materials des Formschlusselements 20e.

Die Anwendungseinheit 12e weist den Grundkörper 26e auf. Der Grundkörper 26e ist fest mit der Verbindungseinheit 18e verbunden. Der Grundkörper 26e ist einstückig mit der Verbindungseinheit 18e verbunden. Der Grundkörper 26e ist einteilig mit der Verbindungseinheit 18e ausgebildet. Die Verbindungseinheit 18e ist in den Grundkörper 26e integriert. Das Formschlusselement 20e und die Aufnahmenut 32e sind einstückig mit dem Grundkörper 26e der Anwendungseinheit 12e ausgebildet. Das Formschlusselement 20e und die Aufnahmenut 32e sind einteilig mit dem Grundkörper 26e der Anwendungseinheit 12e ausgebildet.

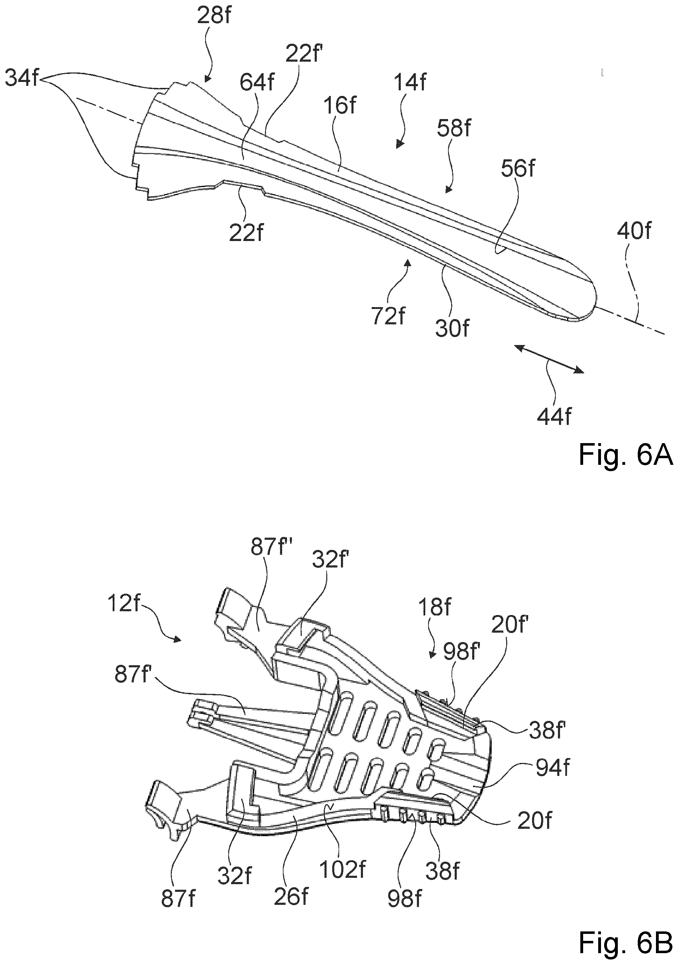

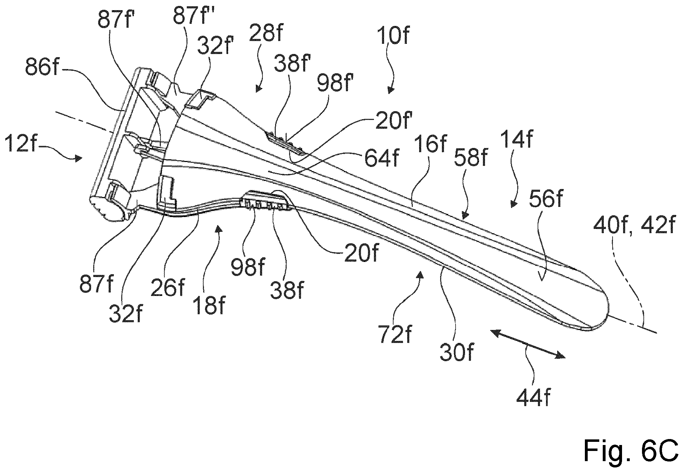

Im Folgenden wird auf die Figuren 6A bis 6C Bezug genommen, welche unterschiedliche Ansichten und Fertigungsstufen des Körperpflegeprodukts 10f zeigen. Aufgrund der unterschiedlichen Ansichten sind einige Elemente nicht in sämtlichen Figuren dargestellt und entsprechend nicht in sämtlichen Figuren mit Bezugszeichen versehen.

Die Figur 6A zeigt eine Griffeinheit 14f eines Körperpflegeprodukts 10f in einer schematischen perspektivischen Darstellung. Das Körperpflegeprodukt 10f ist im vorliegenden Fall als ein Rasierer ausgebildet.

Das Körperpflegeprodukt 10f weist eine Anwendungseinheit 12f auf. Ferner weist das Körperpflegeprodukt 10f eine Griffeinheit 14f auf, welche einen materiellen Griffkörper 16f aufweist. Des Weiteren weist das Körperpflegeprodukt 10f eine Verbindungseinheit 18f auf, welche die Anwendungseinheit 12f mit der Griffeinheit 14f verbindet.

Die Anwendungseinheit 12f bildet einen Rasierkopf des Körperpflegeprodukts 10f. Die Anwendungseinheit 12f weist insbesondere einen als Klingenträger ausgebildeten Grundkörper 26f auf. Der Grundkörper 26f der Anwendungseinheit 12f ist vollständig aus einer Hartkomponente ausgebildet. Es wäre jedoch auch denkbar, dass der Grundkörper 26f der Anwendungseinheit 12f aus einer Hartkomponente und einer Weichkomponente gebildet ist. Der Grundkörper 26f der Anwendungseinheit 12f bildet einen statischen Klingenträger aus, welcher zu einer beweglichen, insbesondere schwenkbaren Aufnahme eines Klingenkopfes 86f vorgesehen ist. Die Anwendungseinheit 12f weist den an dem Grundkörper 26f angeordneten Klingenkopf 86f auf. Der Grundkörper 26f weist drei Gelenkarme 87f, 87f', 87f" auf, welche zu einer schwenkbaren Aufnahme des Klingenkopfes 86f vorgesehen sind. Der Klingenkopf 86f kann insbesondere auswechselbar mit dem Grundkörper 26f verbunden sein.

Die Griffeinheit 14f ist zu einem Greifen durch den Bediener vorgesehen. Die Griffeinheit 14f weist den materiellen Griffkörper 16f auf. Der materielle Griffkörper 16f der Griffeinheit 14f weist einen Kopfbereich 28f auf. Ferner weist der materielle Griffkörper 16f der Griffeinheit 14f einen Griffbereich 72f auf. Der Kopfbereich 28f ist zu einer Anbindung der Anwendungseinheit 12f vorgesehen. Die Griffeinheit 14f weist zumindest eine Grifffläche 56f auf, welche eine Oberfläche des materiellen Griffkörpers 16f ausbildet. Die Grifffläche 56f verläuft auf einer Vorderseite 58f von dem Kopfbereich 28f des Griffkörpers 16f bis zum unteren Ende des Körperpflegeprodukts 10f an der Unterseite. Die Grifffläche 56f erstreckt sich über den Griffbereich 72f des materiellen Griffkörpers 16f. Die Grifffläche 56f bildet entlang einer Längsachse 40f auf der Vorderseite 58f eine teilweise bombierte bzw. teilweise konvexe Form aus. Die bombierte Form ist entlang der Längsachse 40f auf einer Rückseite umgekehrt ausgebildet. Der Griffkörper 16f ist auf der Rückseite 50f in einem Bereich der Grifffläche 56f teilweise konkav geformt. Der Griffkörper 16f weist in der Draufsicht in einem Bereich der Grifffläche 56f eine I-förmige Grundform auf. Der gesamte Griffkörper 16f ist in der Draufsicht insbesondere teilweise Y-förmig oder T-förmig ausgebildet. Der Griffkörper 16f ist dreidimensional verformt, um die bombierte, konvexe Form auszubilden.

Der Griffkörper 16f weist ferner eine im Wesentlichen konstante Materialstärke auf. Ferner weist der Griffkörper 16f eine Nut 64f auf, welche sich auf der Vorderseite 58f von der Anwendungseinheit 12f über die Grifffläche 56f bis zu einer Unterseite der Griffeinheit 14f erstreckt. Die Nut 64f verläuft mittig parallel zu der Längsachse 40f der Griffeinheit 14f. Die Nut 64f weist entlang einer Erstreckung parallel zu der Längsachse 40f einen zumindest annähernd konstanten Querschnitt auf, wobei sich die Nut 64f zu der Anwendungseinheit 12f hin leicht verbreitert. Die Nut 64f weist einen trapezförmigen Querschnitt auf, welcher sich zu der Vorderseite 58f hin aufweitet. Auf der Rückseite 50f des materiellen Griffkörpers 16f ist eine zu der Nut 64f korrespondierende stegförmige Erhebung ausgebildet. Die stegförmige Erhebung entsteht dabei insbesondere durch die konstante Materialstärke bei einer Herstellung der Nut 64f.

Der materielle Griffkörper 16f besteht zumindest zu einem Großteil aus einem Papierwerkstoff. Der materielle Griffkörper 16f besteht vollständig aus einem Papierwerkstoff. Der materielle Griffkörper 16f der Griffeinheit 14f weist zumindest eine Schicht 30f aus einem Papierwerkstoff auf, welche dreidimensional geformt ausgebildet ist. Der materielle Griffkörper 16f besteht aus genau einer Schicht 30f aus einem Papierwerkstoff, es wäre jedoch auch ein mehrschichtiger Aufbau denkbar.

Ferner weist das Körperpflegeprodukt 10f die Verbindungseinheit 18f auf. Die Verbindungseinheit 18f ist zu einer formschlüssigen Verbindung mit dem materiellen Griffkörper 16f der Griffeinheit 14f vorgesehen.

Die Verbindungseinheit 18f weist an jeder Begrenzungswand 38f, 38f' je ein Formschlusselement 20f, 20f' auf. Die Formschlusselemente 20f, 20f' sind dazu vorgesehen, den Griffkörper 16f gegen die Auflagefläche 102f des Grundkörpers 26f zu klemmen. Die Auflagefläche 102f ist insbesondere von einer zu der Unterseite des Kopfbereichs 28f des Griffkörpers 16f korrespondierenden Fläche gebildet, an welcher der Griffkörper 16f großflächig anliegen kann. Die Auflagefläche 102f ist einem Randbereich des Grundkörpers 26f ausgebildet. Die Formschlusselemente 20f, 20f' sind dazu vorgesehen, den Griffkörper 16f zu umgreifen. Die Formschlusselemente 20f, 20f' sind als vorstehende Kanten an die Begrenzungswände 38f, 38f' angeformt und sind so etwas federnd gelagert. Die Kanten sind im Wesentlichen in Richtung der Längsachse 42f des Körperpflegeprodukts ausgerichtet. Die Formschlusselemente 20f, 20f' sind dazu vorgesehen, teilweise in Ausnehmungen 22f, 22f' des Griffkörpers 16f einzugreifen. Die Ausnehmungen 22f, 22f' des Griffkörpers 16f sind von seitlichen Einbuchtungen gebildet.

Der materielle Griffkörper 16f der Griffeinheit 14f weist in dem Kopfbereich 28f auf der Rückseite 50f die Erhebung 66f auf, welche zu einer Positionierung der Verbindungseinheit 18f relativ zu der Griffeinheit 14f vorgesehen ist. Die Erhebung 66f ist dazu vorgesehen, in eine Vertiefung 94f der Verbindungseinheit 18f einzugreifen.

Ferner weist die Verbindungseinheit 18f zumindest eine Aufnahmenut 32f, 32f' auf. Die Verbindungseinheit 18f weist zwei Aufnahmenuten 32f, 32f' auf. Die Aufnahmenuten 32f, 32f' sind an den äußeren Gelenkarmen 87f, 87f" des Grundkörpers 26f der Anwendungseinheit 12f angeordnet. Die Aufnahmenuten 32f, 32f' sind jeweils dazu vorgesehen, eine der Anwendungseinheit 12f zugewandte Vorderkante 34f des materiellen Griffkörpers 16f der Griffeinheit 14f aufzunehmen. Der Griffkörper 16f weist insbesondere eine Y-förmige Grundform auf, wobei insbesondere die beiden äußeren Vorderkanten 34f von den Aufnahmenuten 32f, 32f' aufgenommen werden.

Die Aufnahmenuten 32f, 32f' sind zu einer klemmenden Aufnahme zumindest einer Kante des materiellen Griffkörpers 16f vorgesehen. Die Aufnahmenuten 32f, 32f' erstrecken sich im Wesentlichen senkrecht zu einer Längsachse 40f der Griffeinheit 14f. Es ist denkbar, dass in den Aufnahmenuten 32f, 32f' beispielsweise Widerhaken vorgesehen sind, welche eine Klemmung verbessern. Die Aufnahmenuten 32f, 32f' sind zu einer Bereitstellung einer Klemmverbindung mit dem materiellen Griffkörper 16f vorgesehen. Die Aufnahmenuten 32f, 32f' sind dabei aus Kunststoff hergestellt, sodass außen ein Kunststoff der Verbindungseinheit 18f angeordnet ist, welche innen einen Papierwerkstoff der Griffeinheit 14f aufnimmt. Die Aufnahmenuten 32f, 32f' bilden jeweils eine U-förmige Nut aus, welche zu der Griffeinheit 14f hin geöffnet sind.

Bei einer Herstellung wird die Griffeinheit 14f sowie die Verbindungseinheit 18f bereitgestellt. Anschließend werden die Griffeinheit 14f und die Verbindungseinheit 18f zusammengeführt, wobei die Vorderkante des materiellen Griffkörpers 16f leicht angewinkelt in die Aufnahmenuten 32f, 32f' eingeschoben wird. Anschließend wird der materielle Griffkörper 16f in eine Endposition verschwenkt, wobei die Formschlusselemente 20f, 20f' im Anschluss den Griffkörper 16f klemmen und so verrasten.

Ferner weist die Verbindungseinheit 18f zumindest eine Begrenzungswand 38f, 38f' auf. Die Verbindungseinheit 18f weist zwei Begrenzungswände 38f, 38f' auf. Die Begrenzungswände 38f, 38f' sind zu einer Haupterstreckungsebene der Verbindungseinheit 18f abgewinkelt und erstrecken sich im Wesentlichen parallel zu der Längsachse 40f der Griffeinheit 14f. Die Begrenzungswände 38f, 38f' erstecken sich von den Aufnahmenuten 32f, 32f' in Richtung der Griffeinheit 14f. Die Begrenzungswände 38f, 38f' sind an einer im Wesentlichen parallel zu der Längsachse 42f verlaufenden Außenkante der Verbindungseinheit 18f, insbesondere des Grundkörpers 26f der Anwendungseinheit 12f, angeordnet. Die Begrenzungswände 38f, 38f' sind senkrecht zu der Längsachse der Verbindungseinheit 18f in einem Winkel von zumindest 60°, vorzugsweise zumindest 80°, gegenüber einer Haupterstreckungsebene der Verbindungseinheit 18f abgewinkelt. Die Begrenzungswände 38f, 38f' bilden jeweils eine freie Außenkante des materiellen Griffkörpers 16f aus. Ferner kragen die Begrenzungswände 38f, 38f' jeweils in Richtung der Unterseite aus. An den Begrenzungswänden 38f, 38f' der Verbindungseinheit 18f bilden sich Halteflächen 98f, 98f' aus.

Die Anwendungseinheit 12f weist den Grundkörper 26f auf. Der Grundkörper 26f ist fest mit der Verbindungseinheit 18f verbunden. Der Grundkörper 26f ist einstückig mit der Verbindungseinheit 18f verbunden. Der Grundkörper 26f ist einteilig mit der Verbindungseinheit 18f ausgebildet. Die Verbindungseinheit 18f ist in den Grundkörper 26f integriert. Die Formschlusselemente 20f, 20f', die Aufnahmenuten 32f, 32f' und die Begrenzungswände 38f, 38f' sind einstückig mit dem Grundkörper 26f der Anwendungseinheit 12f ausgebildet. Die Formschlusselemente 20f, 20f', die Aufnahmenuten 32f, 32f' und die Begrenzungswände 38f, 38f' sind einteilig mit dem Grundkörper 26f der Anwendungseinheit 12f ausgebildet. Die Vertiefung 94f ist ebenfalls einteilig mit dem Grundkörper 26f der Anwendungseinheit 12f ausgebildet.

Alternativ wäre auch denkbar, dass die Anwendungseinheit 12f zumindest einen Grundkörper 26f aufweist, welcher wechselbar mit der Verbindungseinheit 18f verbunden ist. Dabei wäre insbesondere denkbar, dass die gesamte Anwendungseinheit 12f beispielsweise als Wechselklinge ausgebildet ist.

### Bezugszeichen

- 10: Körperpflegeprodukt
- 12: Anwendungseinheit
- 14: Griffeinheit
- 16: Griffkörper
- 18: Verbindungseinheit
- 20: Formschlusselement
- 22: Ausnehmung
- 24: Durchgangsausnehmung
- 26: Grundkörper der Anwendungseinheit
- 28: Kopfbereich der Griffeinheit
- 30: Schicht
- 32: Aufnahmenut
- 34: Vorderkante
- 36: Stabilisierungswand
- 38: Begrenzungswand
- 40: Längsachse der Griffeinheit
- 42: Längsachse des Körperpflegeprodukts
- 44: Haupterstreckungsrichtung
- 46: Höhenachse
- 48: Breitenachse
- 50: Rückseite
- 54: Borstenbündel
- 56: Grifffläche
- 58: Vorderseite
- 60: Halsbereich
- 62: Plateau
- 64: Nut
- 66: Erhebung
- 68: Nut
- 70: Erhebung
- 72: Griffbereich
- 74: Formschlusskopf
- 76: Steg
- 77: Faltkante
- 78: Borste
- 80: Funktionselement
- 82: Erhebung
- 84: Vertiefung
- 86: Klingenkopf
- 87: Gelenkarm
- 88: Stielabschnitt
- 90: Kopfabschnitt
- 92: Vertiefung
- 94: Vertiefung
- 96: Haltefläche
- 98: Haltefläche
- 100: Pinselborste
- 102: Auflagefläche

## Patentansprüche

1. Körperpflegeprodukt, insbesondere Zahnbürste, Rasierer oder Applikationspinsel, mit zumindest einer Anwendungseinheit (12a; 12b; 12c; 12d; 12e; 12f), mit einer Griffeinheit (14a; 14b; 14c; 14d; 14e; 14f), welche zumindest einen materiellen Griffkörper (16a; 16b; 16c; 16d; 16e; 16f) aufweist, der zumindest zu einem Großteil aus einem Papierwerkstoff besteht, und mit zumindest einer Verbindungseinheit (18a; 18b; 18c; 18d; 18e; 18f), welche die Anwendungseinheit (12a; 12b; 12c; 12d; 12e; 12f) mit der Griffeinheit (14a; 14b; 14c; 14d; 14e; 14f) verbindet,
**dadurch gekennzeichnet, dass**
die Verbindungseinheit (18a; 18b; 18c; 18d; 18e; 18f) zumindest teilweise zu einer formschlüssigen Verbindung mit dem materiellen Griffkörper (16a; 16b; 16c; 16d; 16e; 16f) der Griffeinheit (14a; 14b; 14c; 14d; 14e; 14f) vorgesehen ist.

2. Körperpflegeprodukt nach Anspruch 1,
**dadurch gekennzeichnet, dass**
die Verbindungseinheit (18a; 18b; 18c; 18d; 18e; 18f) zumindest ein Formschlusselement (20a, 20a'; 20b, 20b'; 20d; 20e; 20f, 20f') aufweist, welches dazu vorgesehen ist, in eine Ausnehmung (22a, 22a'; 22b, 22b'; 22d; 22e; 22f, 22f') der Anwendungseinheit (12a; 12b; 12c; 12d; 12e; 12f) und/oder der Griffeinheit (14a; 14b; 14c; 14d; 14e; 14f) einzugreifen.

3. Körperpflegeprodukt nach Anspruch 2,
**dadurch gekennzeichnet, dass**
der materielle Griffkörper (16a; 16b; 16c; 16d; 16e) zumindest eine Durchgangsausnehmung (24a, 24a'; 24b, 24b'; 24d; 24e) begrenzt, welche dazu vorgesehen ist, das zumindest eine Formschlusselement (20a, 20a'; 20b, 20b'; 20d; 20e) der Verbindungseinheit (18a; 18b; 18c; 18d; 18e) aufzunehmen.

4. Körperpflegeprodukt zumindest nach Anspruch 2,
**dadurch gekennzeichnet, dass**
das Formschlusselement (20d; 20f, 20f') von einem Rastelement gebildet ist.

5. Körperpflegeprodukt zumindest nach Anspruch 2,
**dadurch gekennzeichnet, dass**
das Formschlusselement (20a, 20a'; 20b, 20b'; 20e) von einem Niet, insbesondere einem Kunststoffniet, gebildet ist.

6. Körperpflegeprodukt nach einem der vorhergehenden Ansprüche,
**dadurch gekennzeichnet, dass**
die Anwendungseinheit (12a; 12b; 12c; 12d; 12e; 12f) zumindest einen Grundkörper (26a; 26b; 26c; 26d; 26e; 26f) aufweist, welcher fest mit der Verbindungseinheit (18a; 18b; 18c; 18d; 18e; 18f) verbunden ist.

7. Körperpflegeprodukt nach einem der vorhergehenden Ansprüche,
**dadurch gekennzeichnet, dass**
die Anwendungseinheit (12d) zumindest einen Grundkörper (26d) aufweist, welcher wechselbar mit der Verbindungseinheit (18d) verbunden ist.

8. Körperpflegeprodukt zumindest nach Anspruch 2,
**dadurch gekennzeichnet, dass**
die Anwendungseinheit (12a; 12b; 12c; 12d; 12e; 12f) zumindest einen Grundkörper (26a; 26b; 26c; 26d; 26e; 26f) aufweist, wobei das Formschlusselement (20a, 20a'; 20b, 20b'; 20d; 20e; 20f, 20f') einstückig mit dem Grundkörper (26a; 26b; 26c; 26d; 26e; 26f) der Anwendungseinheit (12a; 12b; 12c; 12d; 12e; 12f) ausgebildet ist.

9. Körperpflegeprodukt nach einem der vorhergehenden Ansprüche,
**dadurch gekennzeichnet, dass**
der materielle Griffkörper (16a; 16b; 16c) der Griffeinheit (14a; 14b; 14c) zumindest einen Kopfbereich (28a; 28b; 28c) aufweist, welcher zu einer Anbindung an der Anwendungseinheit (12a; 12b; 12c) vorgesehen ist, wobei der materielle Griffkörper (16a; 16b; 16c) der Griffeinheit (14a; 14b; 14c) in dem Kopfbereich (28a; 28b; 28c) zumindest eine Vertiefung aufweist, welche zu einer Positionierung der Verbindungseinheit (18a; 18b; 18c) relativ zu der Griffeinheit (14a; 14b; 14c) vorgesehen ist.

10. Körperpflegeprodukt nach einem der vorhergehenden Ansprüche,
**dadurch gekennzeichnet, dass**
der materielle Griffkörper (16a; 16b; 16c; 16d; 16e; 16f) der Griffeinheit (14a; 14b; 14c; 14d; 14e; 14f) aus genau einer Schicht (30a; 30b; 30c; 30d; 30e; 30f) aus einem Papierwerkstoff besteht.

11. Körperpflegeprodukt nach einem der vorhergehenden Ansprüche,
**dadurch gekennzeichnet, dass**
die Verbindungseinheit (18d; 18e; 18f) zumindest eine Aufnahmenut (32d, 32d'; 32e; 32f, 32f') aufweist, welche dazu vorgesehen ist, eine der Anwendungseinheit (12d; 12e; 12f) zugewandte Vorderkante (34d; 34e; 34f) des materiellen Griffkörpers (16d; 16e; 16f) der Griffeinheit (14d; 14e; 14f) aufzunehmen.

12. Körperpflegeprodukt nach einem der vorhergehenden Ansprüche,
**dadurch gekennzeichnet, dass**
der materielle Griffkörper (16a; 16b; 16c; 16d) der Griffeinheit (14a; 14b; 14c; 14d) zumindest eine Stabilisierungswand (36a, 36a'; 36b, 36b'; 36c, 36c'; 36d, 36d') ausbildet, welche zu einer Haupterstreckungsebene der Griffeinheit (14a; 14b; 14c; 14d) abgewinkelt ist und sich zumindest im Wesentlichen parallel zu einer Längsachse (40a; 40b; 40c; 40d) der Griffeinheit (14a; 14b; 14c; 14d) erstreckt.

13. Körperpflegeprodukt nach Anspruch 12,
**dadurch gekennzeichnet, dass**
die Verbindungseinheit (18d) zumindest eine Begrenzungswand (38d, 38d') aufweist, welche zu einer zumindest teilweisen Begrenzung und Stabilisierung der Stabilisierungswand (36d, 36d') des materiellen Griffkörpers (16d) vorgesehen ist.

14. Körperpflegeprodukt nach einem der vorhergehenden Ansprüche,
**dadurch gekennzeichnet, dass**
der materielle Griffkörper (16a; 16c) der Griffeinheit (14a; 14c) entlang einer Längsrichtung der Griffeinheit (14a; 14c) im Wesentlichen S-förmig geschwungen ausgebildet ist.

15. Verfahren zur Herstellung eines Körperpflegeprodukts (10a; 10b; 10c; 10d; 10e; 10f) nach einem der vorhergehenden Ansprüche.
